# EUROPEAN PATENT APPLICATION

(11) **EP 4 603 125 A1**
(43) Date of publication of application: **20.08.2025**
(21) Application number: 23876952.5
(22) Date of filing: 21.06.2023
(51) Int. Cl.: A61M 16/06

(54) **BREATHING AID TOOL, BREATHING AID DEVICE, AND MOISTURE-REMOVING MEMBER**

(30) Priority: 14.10.2022 JP 2022165329; 14.10.2022 JP 2022165330; 26.12.2022 JP 2022208884; 19.06.2023 JP 2023100102
(71) Applicant: Magos Co., Ltd., Kawaguchi-shi, Saitama 332-0015 (JP)
(72) Inventor: NITTA Kazufuku, Kawaguchi-shi, Saitama 332-0015 (JP)
(74) Representative: TBK
(86) International application number: PCT/JP2023/022913
(87) International publication number: WO 2024/079943

(57) **Abstract**

A breathing aid tool of the present invention is a breathing aid tool to guide a gas supplied by a gas-conducting supply pipe to nostrils of a user, and is configured to comprise a first tubular portion which conducts a gas supplied by the supply pipe, which is disposed in a posture of extending in a width direction of a face of the user in an under-nose region between a nose of the user and a mouth thereof, and which has a stretchable structure of being able to extend and retract in its own axial direction (hereinafter referred to as a first tubular side axial direction), and one pair of second tubular portions which is configured to be, while branching from the first tubular portion, able to be inserted into the nostril of the user and is configured to be able to eject, from its own outlet opening, a gas passing through the first tubular portion, wherein spacing of one pair of the second tubular portions can be varied by extending or retracting the first tubular portion in the first tubular side axial direction. This provides a breathing aid tool capable of adjusting a relative position of a part to be inserted into nostrils of a user with respect to the nostrils of a user.

## Description

### [Technical Field]

The present invention relates to a breathing aid tool, a breathing aid device, and a moisture-removing member.

### [Background Art]

As for a breathing aid device, one that supplies gas such as oxygen under positive pressure into a nasal cavity of a patient pervades widely. As for this type of a breathing aid device, one that has a gas source, a delivery conduit to deliver respiratory gas supplied by the gas source, and a nasal cannula to guide, into a nasal cavity of a patient, respiratory gas delivered by the delivery conduit is present, for example (e.g., see JP2013-138874A). Then, a nasal cannula has, for example, a manifold portion whose shape is a substantially tubular form, and a face mounting portion linked to the manifold portion and having one pair of nasal prongs. Respiratory gas supplied through a delivery conduit is transferred to a face mounting portion through a manifold portion, and is supplied to nostrils of a patient by one pair of nasal prongs. Additionally, this delivery conduit has a heating element in its midway section in order to prevent humidified gas generated in such a delivery conduit from having dews formed.

### [Summary of Invention]

### [Problem to be Solved by the Invention]

However, the above-mentioned breathing aid device has a first problem and a secondary problem. The first problem is as follows. The above-mentioned nasal cannula is configured in a manner that spacing of one pair of nasal prongs and a relative angle thereof with respect to a face mounting portion cannot be modified. On the other hand, patients differ from individual to individual as for a distance and an inclination angle for their paired nostrils. Therefore, there is a concern that if the above-mentioned one pair of nasal prongs does not fit a nostrils of a patient in relative position on an occasion of having inserted the above-mentioned one pair of nasal prongs into the nostrils of a patient, the nasal prongs get into contact with a nasal cavity to press the nasal cavity. Additionally, if a state of the above-mentioned one pair of nasal prongs being in contact with a nasal cavity goes on, a patient feels uncomfortable.

In addition, the second problem is as follows. Since the above-mentioned midway section and a last section of a delivery conduit, nearer to a patient between the nasal cannula cannot not be provided with a heating element, respiratory gas is cooled in the last section to have dews formed there and water drops are generated in an internal portion of the delivery conduit in the last section of a delivery conduit. These water drops are likely to be transferred together with respiratory gas through a nasal cannula to nostrils of a patient.

The present invention is, in view of such actual circumstances, intended to provide at least either of a breathing aid tool and a breathing aid device for solving the first problem, and a breathing aid device and a moisture-removing member for solving the second problem. The breathing aid tool and breathing aid device for solving the first problem are configured in a manner that a relative position of a part to be inserted into nostrils of a user with respect to the nostrils of a user can be adjusted. The breathing aid device and moisture-removing member for solving the second problem are configured to prevent water drops generated in an internal portion of the breathing aid device from being delivered to a user.

### [Solution to Problem]

A breathing aid tool handling a first problem of the present invention is a breathing aid tool to guide a gas supplied by a supply pipe to nostrils of a user, and is configured to comprise a first tubular portion which conducts a gas supplied by the supply pipe, which is disposed in a posture of extending in a width direction of a face of the user in an under-nose region between a nose of the user and a mouth thereof, and which has a stretchable structure of being able to extend and retract in its own axial direction (hereinafter referred to as a first tubular side axial direction), and one pair of second tubular portions which is configured to be, while branching from the first tubular portion, able to be inserted into the nostril of the user and is configured to be able to eject, from its own outlet opening, a gas passing through the first tubular portion, wherein spacing of one pair of the second tubular portions can be varied by extending or retracting the first tubular portion in the first tubular side axial direction.

A breathing aid tool handling a first problem of the present invention further comprises a spacing modification mechanism which is, so as to be able to guide a gas supplied by the supply pipe to the first tubular portion, connected to the first tubular portion, and which allows or restricts, by allowing or restricting extension and retraction of the first tubular portion in accordance with a magnitude of external force applied, a modification of spacing of one pair of the second tubular portions in the first tubular side axial direction.

**In** a breathing aid tool handling a first problem of the present invention, the spacing modification mechanism includes one pair of communicating tubular portions which is, so as to be able to guide a gas supplied by the supply pipe to the first tubular portion, connected to each of both ends of the first tubular portion, and one pair of connection position modification mechanisms which is configured to be between the first tubular portion and one pair of the communicating tubular portions, and which allows or restricts a modification of a relative connection position (hereinafter, referred to as a relative connection position) between the first tubular portion and the communicating tubular portion in accordance with a magnitude of external force applied, and when the relative connection position between the first tubular portion and the communicating tubular portion is modified by one pair of the connection position modification mechanisms, the stretchable structure extends and retracts in accordance with a modification of this relative connection position while spacing of one pair of the second tubular portions is modified.

**In** a breathing aid tool handling a first problem of the present invention, the first tubular portion and the communicating tubular portion are, when the relative connection position between the first tubular portion and the communicating tubular portion is modified by the connection position modification mechanism, connected with each other so as to modify a length (hereinafter, referred to as an axial length) in an axial direction (hereinafter, referred to as a communicating side axial direction) of the communicating tubular portion of an overlapping region where the first tubular portion is overlapped with the communicating tubular portion.

**In** a breathing aid tool handling a first problem of the present invention, the connection position modification mechanism includes one pair of first axial engagement regions which is provided, by making a basis out of a middle of the first tubular portion in the first tubular side axial direction, on both sides of the first tubular portion, and a second axial engagement region which is provided on each of one pair of the communicating tubular portions and is, when each of one pair of the communicating tubular portions is connected to the first tubular portion, overlapped with the first axial engagement regions to be engaged with the first axial engagement regions in an axial direction (hereinafter, referred to as a communicating side axial direction) of the communicating tubular portion, wherein the first axial engagement regions and the second axial engagement region are configured in a manner that with the relative connection position being modified, an engagement overlapping region where the first axial engagement regions and the second axial engagement region are overlapped with each other to be engaged varies in a given range in the communicating side axial direction, by applying external force, a modification of a range of the engagement overlapping region is allowed by engagement between the first axial engagement regions and the second axial engagement region being removed so that a relative movement between the first axial engagement regions and the second axial engagement region is allowed, and on removing external force, a relative movement between the first axial engagement regions and the second axial engagement region is restricted by engagement between the first axial engagement regions and the second axial engagement region so that a modification of a range of the engagement overlapping region is restricted.

**In** a breathing aid tool handling a first problem of the present invention, at least either of the first axial engagement regions and the second axial engagement region is constituted by an elastic deformation material, which can be elastically deformed, and by applying external force, at least either of the first axial engagement regions and the second axial engagement region which are constituted by an elastic deformation material is elastically deformed so that engagement between the first axial engagement regions and the second axial engagement region is removed.

**In** a breathing aid tool handling a first problem of the present invention, either of the first axial engagement regions and the second axial engagement region has at least one protrusion portion which protrudes, in a state of being mutually engaged, toward a rest other of the first axial engagement regions and the second axial engagement region, whereas the rest other of the first axial engagement regions and the second axial engagement region has a plurality of engagement portions which is provided in plurality with intervals in the first tubular side axial direction or in the communicating side axial direction and is configured to be, while respectively facing the protrusion portion in a state of being engaged with each other, able to be engaged with the protrusion portion, and the protrusion portion and the engagement portion are configured to be engaged in the communicating side axial direction and restrict relative movement between the first axial engagement regions and the second axial engagement region.

**In** a breathing aid tool handling a first problem of the present invention, the protrusion portion reduces diameter in a radial direction of the first tubular portion in an inner circumferential surface of the first tubular portion, and is constituted by a diameter-reduction portion configured to be able to press the engagement portion in a state of being mutually engaged with the engagement portion, whereas the engagement portion protrudes in a radial direction of the communicating tubular portion in an outer circumferential surface of the communicating tubular portion, while being provided in plurality with intervals in the communicating side axial direction, wherein by dint of a friction force due to a pressing force by the diameter-reduction portion on the engagement portion, the protruding portion and the diameter-reduction portion are engaged with each other.

**In** a breathing aid tool handling a first problem of the present invention, the protrusion portion is provided in plurality with intervals in the first tubular side axial direction or in the communicating side axial direction, whereas the engagement portion is constituted by a plurality of groove portions configured to be able to be fitted together into the protrusion portion, wherein by the protrusion portion and the groove portions being fitted together into each other, the protrusion portion and the groove portions are engaged with each other.

A breathing aid tool handling a first problem of the present invention further comprises a swing mechanism which allows or restricts swing of the second tubular portion around a central axis of the first tubular portion in accordance with a magnitude of external force applied.

**In** a breathing aid tool handling a first problem of the present invention, the spacing modification mechanism has a communicating tubular portion which is, so as to be able to guide a gas supplied by the supply pipe to the first tubular portion, connected to an end portion of the first tubular portion, and the swing mechanism is configured to allow or restrict, in accordance with a magnitude of external force applied, a relative swing of the second tubular portion with respect to at least part of the communicating tubular portion in a circumferential direction of the communicating tubular portion together with a relative rotation of the first tubular portion with at least part of the communicating tubular portion being an axis with respect to at least part of the communicating tubular portion.

In a breathing aid tool handling a first problem of the present invention, the swing mechanism has a first swing engagement region which is provided on the first tubular portion and a second swing engagement region which is provided on the communicating tubular portion and is, when the communicating tubular portion is connected to the first tubular portion, overlapped with the first swing engagement region to be engaged with the first swing engagement region in a circumferential direction of the communicating tubular portion, wherein by applying external force, engagement between the first swing engagement region and the second swing engagement region is removed so that the relative swing of the second tubular portion together with the relative rotation of the first tubular portion is allowed, and on removing external force, the relative swing of the second tubular portion together with the relative rotation of the first tubular portion is restricted by engagement between the first swing engagement region and the second swing engagement region.

In a breathing aid tool handling a first problem of the present invention, the spacing modification mechanism has a connection position modification mechanism which is formed between the first tubular portion and one pair of the communicating tubular portions, and which allows or restricts a modification of a relative connection position (hereinafter, referred to as a relative connection position) between the first tubular portion and the communicating tubular portion in accordance with a magnitude of external force applied, when the relative connection position between the first tubular portion and the communicating tubular portion is modified by the connection position modification mechanism, the stretchable structure extends and retracts in accordance with a modification of this relative connection position while spacing of one pair of the second tubular portions is modified, whereas the connection position modification mechanism includes one pair of first axial engagement regions which is provided, by making a basis out of a middle of the first tubular portion in the first tubular side axial direction, on both sides of the first tubular portion, and a second axial engagement region which is provided on each of one pair of the communicating tubular portions and is, when each of one pair of the communicating tubular portions is connected to the first tubular portion, overlapped with the first axial engagement regions to be engaged with the first axial engagement regions in an axial direction (hereinafter, referred to as a communicating side axial direction) of the communicating tubular portion, wherein the first axial engagement regions and the second axial engagement region are configured in a manner that with the relative connection position being modified, an engagement overlapping region where the first axial engagement regions and the second axial engagement region are overlapped with each other to be engaged varies in a given range in the communicating side axial direction, and the first axial engagement regions and the first swing engagement region are at least partially in common with each other while the second axial engagement region and the second swing engagement region are at least partially in common with each other.

In a breathing aid tool handling a first problem of the present invention, the spacing modification mechanism has one pair of communicating tubular portions which is, so as to be able to guide a gas supplied by the supply pipe to the first tubular portion, connected to each of both ends of the first tubular portion, on an occasion of dividing the first tubular portion into two regions by making a basis out of a middle in the first tubular side axial direction to define as a first region, a region being one side of the first tubular portion and being connected to the communicating tubular portion (hereinafter, referred to as a first communicating tubular portion) on one side while to define as a second region, a region being another side of the first tubular portion and being connected to the communicating tubular portion (hereinafter, referred to as a second communicating tubular portion) on another side, the swing mechanism includes a first swing mechanism piece which allows or restricts, in accordance with a magnitude of external force applied, a relative swing of the second tubular portion of one side in a circumferential direction of the first communicating tubular portion together with a relative rotation of the first region with at least part of the first communicating tubular portion being an axis with respect to at least part of the first communicating tubular portion, and a second swing mechanism piece which allows or restricts, in accordance with a magnitude of external force applied, a relative swing of the second tubular portion of another side in a circumferential direction of the second communicating tubular portion together with a relative rotation of the second region with at least part of the second communicating tubular portion being an axis with respect to at least part of the second communicating tubular portion.

**In** a breathing aid tool handling a first problem of the present invention, the first swing mechanism piece and the second swing mechanism piece operate independently of each other.

In a breathing aid tool handling a first problem of the present invention, the first swing mechanism piece and the second swing mechanism piece operate independently of each other when a relative angle of the second tubular portion of another side by making a basis out of the second tubular portion of one side is less than a threshold value, and when this relative angle is a threshold value or greater, a relative swing is restricted by dint of a resilience due to a twist of the first tubular portion.

A breathing aid tool handling a first problem of the present invention is configured to comprise a communicating tubular portion which is, so as to be able to guide a gas supplied by the supply pipe to the first tubular portion, connected to an end portion of the first tubular portion, and a swing mechanism which allows or restricts, in accordance with a magnitude of external force applied, a swing of the second tubular portion in a circumferential direction of the communicating tubular portion, wherein the communicating tubular portion includes a first connection tubular portion which is connected to the first tubular portion, and a second connection tubular portion whose own one end side is connected to the first connection tubular portion and whose own one end side is connected to the supply pipe, wherein the swing mechanism includes a relative rotation restriction mechanism which restricts a relative rotation between the first tubular portion and the first connection tubular portion, and a relative rotation mechanism which allows or restricts, in accordance with a magnitude of external force applied, a relative rotation between the first connection tubular portion and the second connection tubular portion, wherein with respect to the second connection tubular portion, the first tubular portion together with the first connection tubular portion makes a relative rotation, whereby the second tubular portion swings in a circumferential direction of the communicating tubular portion.

**In** a breathing aid tool handling a first problem of the present invention, the relative rotation restriction mechanism is configured to be in a connection region between the first tubular portion and the first connection tubular portion, and in the connection region, the first tubular portion and the first connection tubular portion are in contact while overlapped with each other, while in a cross section of the first connection tubular portion and the first tubular portion being cut in a direction perpendicular to an axial direction (hereinafter, referred to as a communicating side axial direction) of the communicating tubular portion, a region where the first tubular portion and the first connection tubular portion are in contact with each other is shaped in a non-genuinely circular form.

In a breathing aid tool handling a first problem of the present invention, the relative rotation mechanism includes a first connection tubular side engagement region which is provided on an inner circumferential surface or outer circumferential surface of the first connection tubular portion, and a second connection tubular side engagement region which is provided in the second connection tubular portion and is, on an occasion of the first connection tubular portion and the second connection tubular portion being connected, engaged with the first connection tubular side engagement region while facing the first connection tubular side engagement region, wherein by applying external force, engagement between the first connection tubular side engagement region and the second connection tubular side engagement region is removed so that a relative rotation between the first connection tubular portion and the second connection tubular portion is allowed, and on removing external force, a relative rotation between the first connection tubular portion and the second connection tubular portion is restricted by engagement between the first connection tubular side engagement region and the second connection tubular side engagement region.

In a breathing aid tool handling a first problem of the present invention, the second connection tubular portion includes a middle connection tubular portion whose own one end side is connected to the first connection tubular portion, and a supply pipe side connection tubular portion whose own one end side is connected to another end side of the middle connection tubular portion and whose own another end side is connected relatively rotatably to the supply pipe.

A breathing aid tool handling a first problem of the present invention further comprises a second relative rotation restriction mechanism which restricts a range of a relative rotation between the first connection tubular portion and the second connection tubular portion to a given relative rotation angle, wherein the second relative rotation restriction mechanism includes a partial circumference groove portion which is provided on either of the first connection tubular portion and the second connection tubular portion, and which extends, while recessed in a radial direction of the communicating tubular portion, only by a partial circumference corresponding to the given relative rotation angle in a circumferential direction of the communicating tubular portion, and a restriction protrusion portion which is provided on a rest other of the first connection tubular portion and the second connection tubular portion, which protrudes in a radial direction of the communicating tubular portion, and which is provided so as to be, when the first connection tubular portion and the second connection tubular portion make a relative rotation, able to revolve in a circumferential direction of the communicating tubular portion along the partial circumference groove portion within the partial circumference groove portions, wherein a revolution angle at which the restriction protrusion portion can revolve within the partial circumference groove portion is identical with the given relative rotation angle at which the first connection tubular portion and the second connection tubular portion can make a relative rotation.

A breathing aid tool handling a first problem of the present invention further comprises a connection position modification mechanism which is configured to be between the first tubular portion and the first connection tubular portion, and allows or restricts, in accordance with a magnitude of external force applied, a modification of a relative connection position (hereinafter, referred to as a relative connection position) between the first tubular portion and the first connection tubular portion, wherein when the relative connection position between the first tubular portion and the first connection tubular portion is modified by one pair of the connection position modification mechanism, the stretchable structure extends and retracts in accordance with a modification of this relative connection position while spacing of one pair of the second tubular portions is modified, the connection position modification mechanism includes a first axial engagement region which is provided on the first tubular portion, and a second axial engagement region which is provided on the first connection tubular portion and is, when the first connection tubular portion is connected with the first tubular portion, overlapped with the first axial engagement region to be engaged with the first axial engagement region in an axial direction (hereinafter, referred to as a communicating side axial direction) of the communicating tubular portion, wherein the second axial engagement region is provided on a position distal from the second connection tubular portion in the communicating side axial direction as compared with the first connection tubular side engagement region.

A breathing aid tool handling a first problem of the present invention further comprises a communicating tubular side hold portion which holds the communicating tubular portion in a manner that the first tubular portion is disposed in a posture of extending in a width direction of the user in the under-nose region of the user, and a third relative rotation restriction mechanism which restricts relative rotation of the middle connection tubular portion with respect to the hold portion.

A breathing aid tool handling a first problem of the present invention further comprises a mounting portion to mount one pair of the communicating tubular portions on a face of the user in a manner that on the under-nose region, the first tubular portion is in a posture of extending in the width direction of a face of the user.

In a breathing aid tool handling a first problem of the present invention, the mounting portion is extended from the communicating tubular portion toward the under-nose region, and the communicating tubular portion is, by abutting on a face of the user in the under-nose region, determined in position to a face of the user in a manner that the first tubular portion is in a posture of extending in the width direction of a face of the user.

A breathing aid tool handling a first problem of the present invention further comprises a moisture-removing portion which is constituted by a water-absorbing material having water absorbing property and which absorbs moisture in an internal portion of the supply pipe and the breathing aid tool to discharge it into an external portion of the supply pipe and the breathing aid tool, wherein the moisture-removing portion includes an internal portion side absorption portion which is disposed in an internal portion of at least either of the supply pipe and the breathing aid tool to absorb moisture in an internal portion of at least either of the supply pipe and the breathing aid tool, and an exposure portion which exposes itself, while being linked to the internal portion side absorption portion, to an external portion of the supply pipe and the breathing aid tool to discharge moisture transferred from the internal portion side absorption portion, into an external portion.

A breathing aid tool handling a first problem of the present invention further comprises, while interposed between the mounting portion and the under-nose region, a protection sheet portion to protect the under-nose region by abutting on the under-nose region.

Onto the protection sheet portion of a breathing aid tool handling a first problem of the present invention, tension is applied in the width direction.

A breathing aid tool handling a first problem of the present invention is a breathing aid tool to guide a gas supplied by a gas-conducting supply pipe to nostrils of a user, and is configured to comprise a first tubular portion which conducts a gas supplied by the supply pipe, and which is disposed in a posture of extending in a width direction of a face of the user in an under-nose region between a nose of the user and a mouth thereof, and one pair of second tubular portions which is configured to be, while branching from the first tubular portion, able to be inserted into the nostril of the user and is configured to be able to eject, from its own outlet opening, a gas passing through the first tubular portion, and a communicating tubular portion which is, so as to be able to guide a gas supplied by the supply pipe, connected to an end portion of the first tubular portion, and a swing mechanism which allows or restricts, in accordance with a magnitude of external force applied, a swing of the second tubular portion in a circumferential direction of the communicating tubular portion, wherein the communicating tubular portion includes a first connection tubular portion which is connected to the first tubular portion, and a second connection tubular portion whose own one end side is connected to the first connection tubular portion and whose own one end side is connected to the supply pipe, wherein the swing mechanism includes a relative rotation restriction mechanism which restricts a relative rotation between the first tubular portion and the first connection tubular portion, and a relative rotation mechanism which allows or restricts, in accordance with a magnitude of external force applied, a relative rotation between the first connection tubular portion and the second connection tubular portion, and wherein with respect to the second connection tubular portion, the first tubular portion together with the first connection tubular portion makes a relative rotation, whereby the second tubular portion swings in a circumferential direction of the communicating tubular portion.

A breathing aid tool handling a first problem of the present invention is a breathing aid tool to guide a gas supplied by a gas-conducting supply pipe to nostrils of a user, and comprises a first tubular portion which conducts a gas supplied by the supply pipe, and is disposed in a posture of extending in a width direction of a face of the user in an under-nose region between a nose of the user and a mouth thereof, and one pair of second tubular portions which is configured to be, while branching from the first tubular portion, able to be inserted into the nostril of the user and is configured to be able to eject, from its own outlet opening, a gas passing through the first tubular portion, and one pair of communicating tubular portions which, so as to be able to guide a gas supplied by the supply pipe to the first tubular portion, is connected, on one end side of one moiety, to one end side of the first tubular portion, and is connected, on another end side of one moiety, to the supply pipe, and simultaneously, is connected, on one end side of another moiety, to another end side of the first tubular portion, and one pair of connection position modification mechanisms which is formed between the first tubular portion and one pair of the communicating tubular portions, and which allows, when having a first external force applied, a relative movement between the first tubular portion and the communicating tubular portion to modify a connection position between the first tubular portion and the communicating tubular portion, and a first relative swing mechanism which, on an occasion of dividing the first tubular portion into two regions by making a basis out of a middle in an axial direction of the first tubular portion to define as a first region, a region being one side of the first tubular portion and being connected to the communicating tubular portion (hereinafter, referred to as a first communicating tubular portion) on one side and to define as a second region, a region being another side of the first tubular portion and being connected to the communicating tubular portion (hereinafter, referred to as a second communicating tubular portion) on another side, makes the second tubular portion of one side perform, when having a second external force applied, a relative swing together with the first region with respect to the first communicating tubular portion in a circumferential direction of the first communicating tubular portion, and a second relative swing mechanism which, thereon, makes the second tubular portion of another side perform, when having a third external force applied, a relative swing together with the second region with respect to the second communicating tubular portion in a circumferential direction of the second communicating tubular portion, wherein by one pair of the connection position modification mechanism, a relative movement between the first tubular portion and the communicating tubular portion is, when the first external force is applied, allowed so that a connection position between the first tubular portion and the communicating tubular portion is modified, whereby the stretchable structure extends and retracts in accordance with modification of this connection position, and simultaneously, spacing of one pair of the second tubular portions is varied.

A breathing aid tool handling a first problem of the present invention is a breathing aid tool to guide a gas supplied by a gas-conducting supply pipe to nostrils of a user, and comprises a first tubular portion which conducts a gas supplied by the supply pipe, and is disposed in a posture of extending in a width direction of a face of the user in an under-nose region between a nose of the user and a mouth thereof, and one pair of second tubular portions which is configured to be, while branching from the first tubular portion, able to be inserted into the nostril of the user and is configured to be able to eject, from its own outlet opening, a gas passing through the first tubular portion, and one pair of communicating tubular portions which, so as to be able to guide a gas supplied by the supply pipe to the first tubular portion, is connected, on one end side of one moiety, to one end side of the first tubular portion, and is connected, on another end side of one moiety, to the supply pipe, and simultaneously, is connected, on one end side of another moiety, to another end side of the first tubular portion, and one pair of connection position modification mechanisms which is formed between the first tubular portion and one pair of the communicating tubular portions, and which allows, when having a first external force applied, a relative movement between the first tubular portion and the communicating tubular portion to modify a connection position between the first tubular portion and the communicating tubular portion, wherein the connection position modification mechanism has a diameter-reduction portion which in the first tubular portion, has its diameter reduced in a radial direction of the first tubular portion, and an engagement portion which on an outer circumferential surface of the communicating tubular portion, is provided in plurality with intervals in an axial direction (hereinafter, referred to as a communicating side axial direction) of the communicating tubular portion, and is respectively configured to be able to be engaged with the diameter-reduction portion, and wherein when spacing of one pair of the second tubular portions is varied by the first external force, the stretchable structure extends and retracts in accordance with this variance and simultaneously, relative movement between the diameter-reduction portion and the engagement portion in the communicating side axial direction is allowed, whereby a relative connection position between the first tubular portion and the communicating tubular portion is modified, so that engagement between the diameter-reduction portion and the engagement portion at this connection position modified restricts relative movement between the first tubular portion and the communicating tubular portion.

A breathing aid device handling a first problem of the present invention comprises a supply pipe for supplying a user with gas and a breathing aid tool to guide, to nostrils of the user, a gas supplied through the supply pipe, wherein the breathing aid tool includes a first tubular portion which conducts a gas supplied by the supply pipe, which is disposed in a posture of extending in a width direction of a face of the user in an under-nose region between a nose of the user and a mouth thereof, and which has a stretchable structure of being able to extend and retract in its own axial direction (hereinafter referred to as a first tubular side axial direction), and one pair of second tubular portions which is configured to be, while branching from the first tubular portion, able to be inserted into the nostril of the user and is configured to be able to eject, from its own outlet opening, a gas passing through the first tubular portion, and wherein it is configured in a manner that by extending and retracting the first tubular portion in the first tubular side axial direction, spacing of one pair of the second tubular portions can be varied.

A breathing aid device handling a second problem of the present invention is constituted by a supply pipe for supplying a user with gas, a breathing aid tool to guide a gas supplied through the supply pipe to nostrils of the user or a mouth thereof, a moisture-removing portion which is constituted by a water-absorbing material having water absorbing property and which absorbs moisture in an internal portion of the supply pipe and the breathing aid tool to discharge it into an external portion of the supply pipe and the breathing aid tool, wherein the moisture-removing portion includes an internal portion side absorption portion which is disposed in an internal portion of at least either of the supply pipe and the breathing aid tool to absorb moisture in an internal portion of at least either of the supply pipe and the breathing aid tool, and an exposure portion which exposes itself, while being linked to the internal portion side absorption portion, to an external portion of the supply pipe and the breathing aid tool to discharge moisture transferred from the internal portion side absorption portion, into an external portion.

In a breathing aid device handling a second problem of the present invention, a discharge opening for discharging moisture is formed on at least either of the supply pipe and the breathing aid tool, and the exposure portion exposes itself to an external portion via the discharge opening.

In a breathing aid device handling a second problem of the present invention, the breathing aid tool has a main tubular portion which is disposed in a manner of extending in a width direction of a face of the user and to which the supply pipe is connected on one end portion of this width direction, and an ejection portion which has an ejection orifice able to be opposite the nostrils or mouth of the user while able to eject into an external portion, a gas passing through the main tubular portion, and is provided midway on the main tubular portion, on another end portion side of the main tubular portion, the discharge opening is formed, and wherein the internal portion side absorption portion extends from an internal portion of at least either of the supply pipe and the main tubular portion to the discharge opening.

In a breathing aid device handling a second problem of the present invention, the exposure portion is constituted by a plug portion which blocks the discharge opening, and the plug portion temporarily holds moisture transferred from the internal portion side absorption portion and discharges it into an external portion after vaporizing, by drying, it.

In a breathing aid device handling a second problem of the present invention, the breathing aid tool is provided on another end portion of the main tubular portion and has a gas retaining portion which retains a gas having passed the main tubular portion.

In a breathing aid device handling a second problem of the present invention, the gas retaining portion has a blockage portion which blocks the end portion of the gas retaining portion and has the discharge opening, and the blockage portion can be freely attached and detached.

In a breathing aid device handling a second problem of the present invention, a gap is provided between the discharge opening and the exposure portion, and on an occasion of having defined as a gas passage region, a region through which a gas leaking from the gap passes, the exposure portion has on its surface, an overlapping region which overlaps with the gas passage region.

In a breathing aid device handling a second problem of the present invention, the exposure portion is made so as to protrude into an external portion from the discharge opening and comprises a cover portion which is constituted by a material through which gas can pass, and which covers surroundings of a region protruding from the discharge opening in the exposure region.

In a breathing aid device handling a second problem of the present invention, the internal portion side absorption portion is, by making a basis out of a flow of a gas passing through the main tubular portion, disposed in an upstream side section of the main tubular portion as compared with the ejection portion.

In a breathing aid device handling a second problem of the present invention, the ejection portion branches into two from the main tubular portion, and the internal portion side absorption portion is, by making a basis out of a flow of a gas passing through the main tubular portion, disposed in a manner of spanning an upstream side section of the main tubular portion as compared with the ejection portion and a section between two of the ejection portions.

In a breathing aid device handling a second problem of the present invention, the breathing aid tool has a main tubular portion which is disposed in a manner of extending in a width direction of a face of the user and to which the supply pipe is connected on one end portion of this width direction, and an ejection portion which has an ejection orifice able to be opposite the nostrils or mouth of the user while able to eject into an external portion, a gas passing through the main tubular portion, and is provided midway on the main tubular portion, wherein the exposure portion constitutes part of a circumferential wall of the main tubular portion.

In a breathing aid device handling a second problem of the present invention, the exposure portion constitutes, by making a basis out of a flow of a gas passing through the main tubular portion, part of a circumferential wall of the main tubular portion in an upstream side section as compared with the ejection portion.

In a breathing aid device handling a second problem of the present invention, the ejection portion branches into two from the main tubular portion, and the exposure portion constitutes, by making a basis out of a flow of a gas passing through the main tubular portion, part of a circumferential wall of the main tubular portion in a manner of spanning an upstream side section as compared with the ejection portion and a section between two of the ejection portions.

In a breathing aid device handling a second problem of the present invention, the exposure portion is, on an occasion where the ejection orifices are disposed in a manner of being opposite the nostrils of the user, provided in a contrary side region of the main tubular portion, which is located on a side contrary to an opposite region of the main tubular portion, which is opposite a skin surface under a nose of the user.

In a breathing aid device handling a second problem of the present invention, the exposure portion is, on an occasion of having defined as an exhalation passage region, a region through which an exhalation emitted from the nostrils of the user passes, provided in an overlapping region which overlaps with the exhalation passage region, out of the contrary side region.

A moisture-removing member handling a second problem of the present invention is a moisture-removing member which, in a breathing aid device comprising a supply pipe for supplying a user with gas and a breathing aid tool to guide, to the user, a gas supplied through this supply pipe, absorbs moisture in an internal portion of the supply pipe and the breathing aid tool to discharge it into an external portion of the supply pipe and the breathing aid tool, and comprises an internal portion side absorption portion which is constituted by a water-absorbing material having water absorbing property and which is disposed in an internal portion of at least either of the supply pipe and the breathing aid tool to absorb moisture in an internal portion of at least either of the supply pipe and the breathing aid tool, and an exposure portion which is constituted by the water-absorbing material and which exposes itself, while being linked to the internal portion side absorption portion, to an external portion of the supply pipe and the breathing aid tool to discharge moisture transferred from the internal portion side absorption portion, into an external portion.

### [Advantageous Effects of Invention]

A breathing aid tool and breathing aid device which solves a first problem of the present invention can exert an excellent effect of being able to adjust a relative position of a portion to be inserted into nostrils of a user with respect to the nostrils of a user. Additionally, a breathing aid device and moisture-removing member which solve a second problem of the present invention can exert an excellent effect of preventing water drops generated in an internal portion of the breathing aid device from being delivered into a user.

### [Brief Description of Drawings]

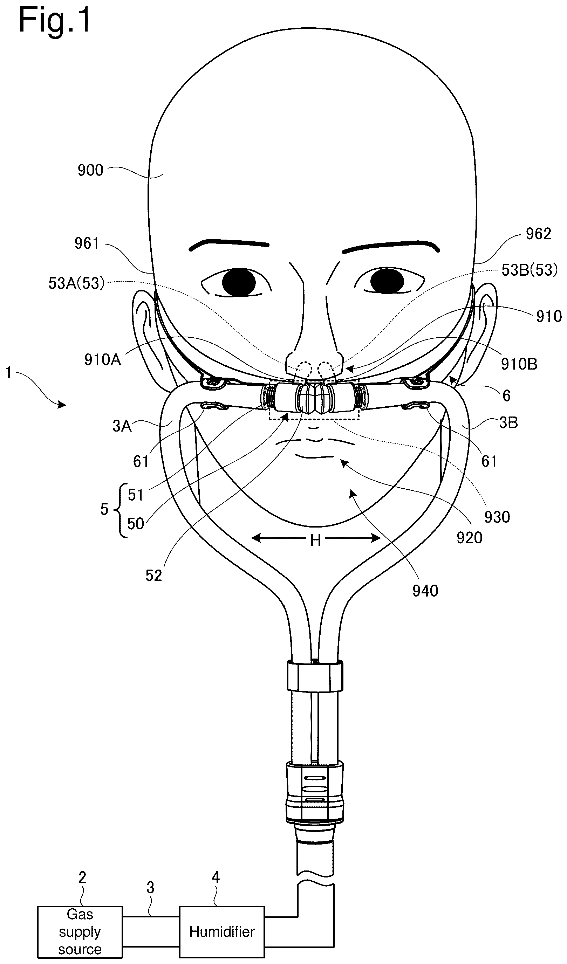

[Fig.1] A front view of a user on whom a breathing aid device in a first embodiment of the present invention is mounted.

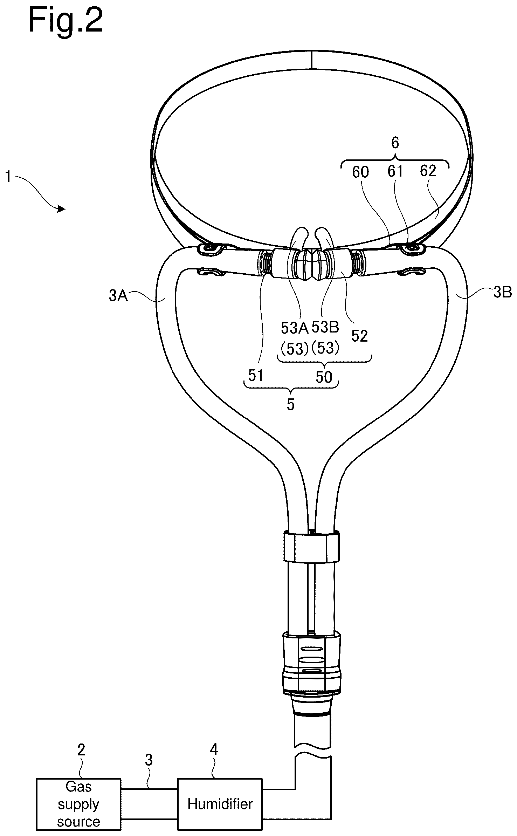

[Fig.2] A front view of the breathing aid device in the first embodiment of the present invention.

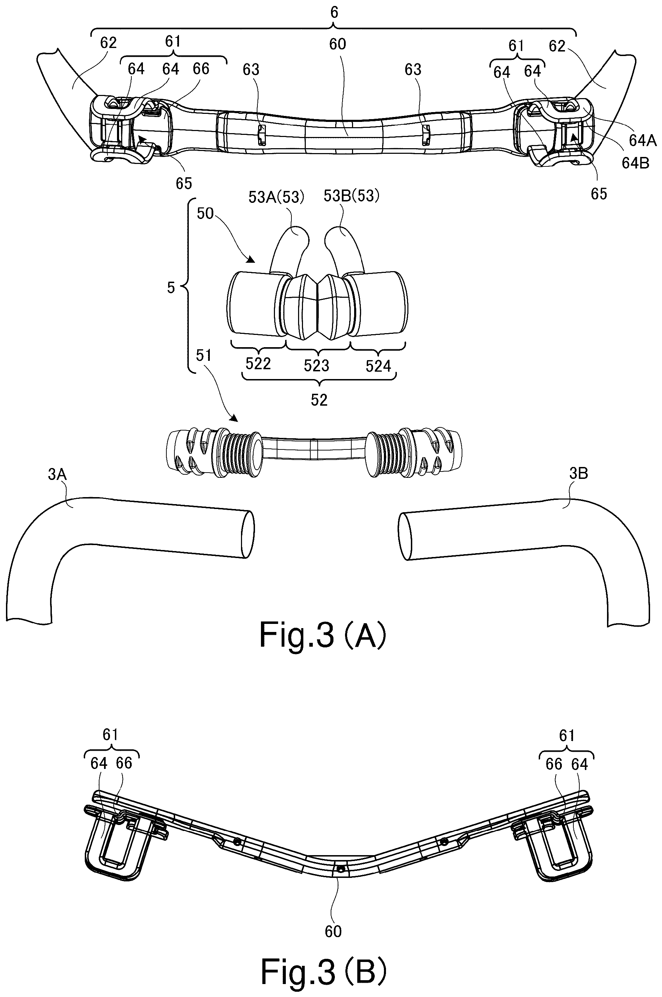

[Fig.3] (A) An exploded view of disassembling a part of the breathing aid device in the first embodiment of the present invention. (B) A top view of a breathing aid tool side hold portion and one pair of supply pipe hold portions in the first embodiment of the present invention.

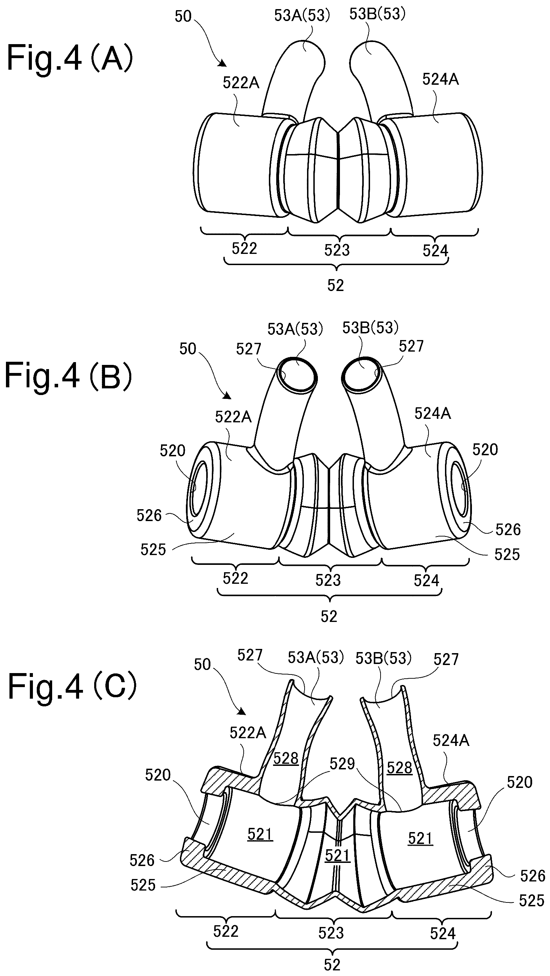

[Fig.4] (A) A front view of a gas guiding portion in the first embodiment of the present invention.
   (B) A rear view of the gas guiding portion in the first embodiment of the present invention.
   (C) A schematic cross-sectional view of the gas guiding portion in the first embodiment of the present invention.

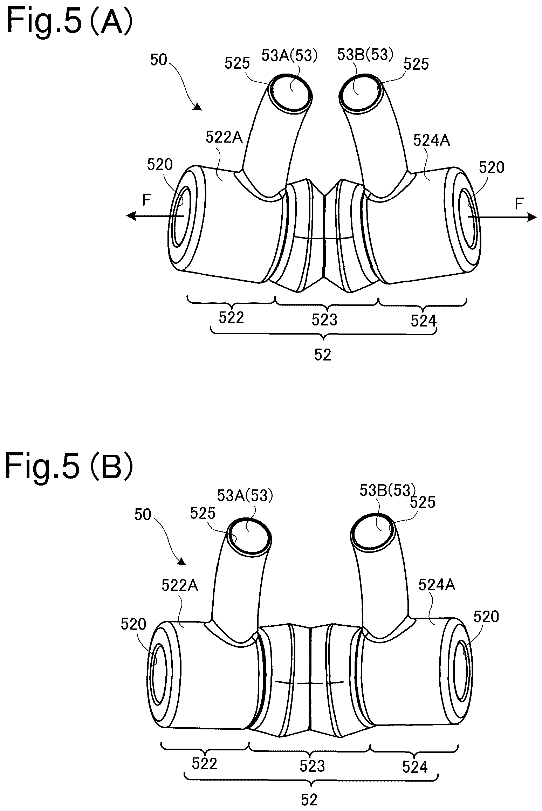

[Fig.5] (A) A rear view of the gas guiding portion before stretching the gas guiding portion in the first embodiment of the present invention in an axial direction (a first tubular side axial direction) of the gas guiding portion (a first tubular portion). (B) A rear view of the gas guiding portion after stretching the gas guiding portion in the first embodiment of the present invention in an axial direction (the first tubular side axial direction) of the gas guiding portion (the first tubular portion).

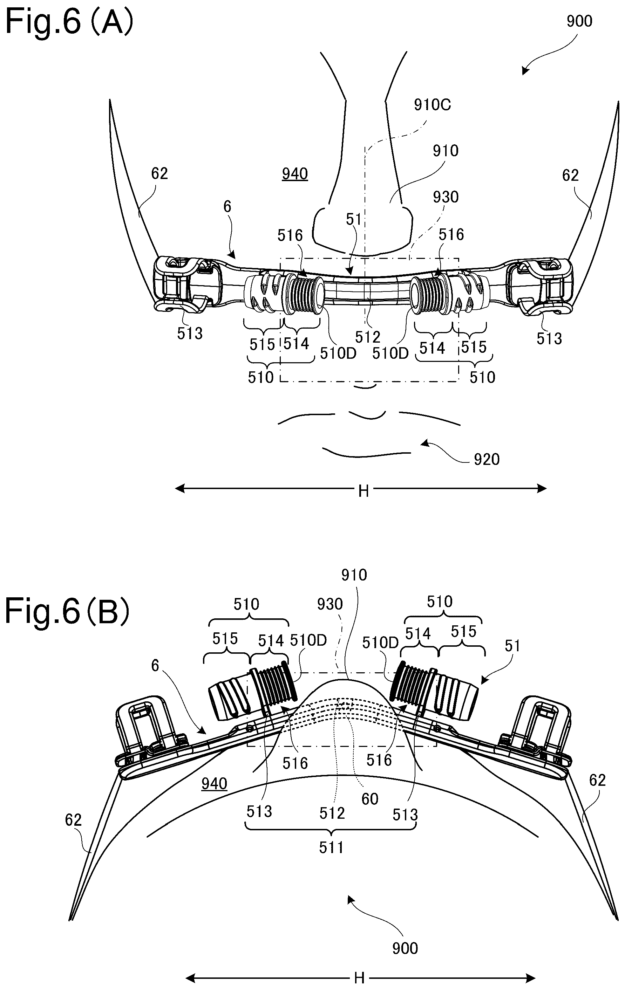

[Fig.6] (A) A front view of a user on an occasion of having mounted a communicating portion on the user, by a mounting portion in the first embodiment of the present invention. (B) A top view of a user on an occasion of having mounted the communicating portion on the user, by the mounting portion in the first embodiment of the present invention.

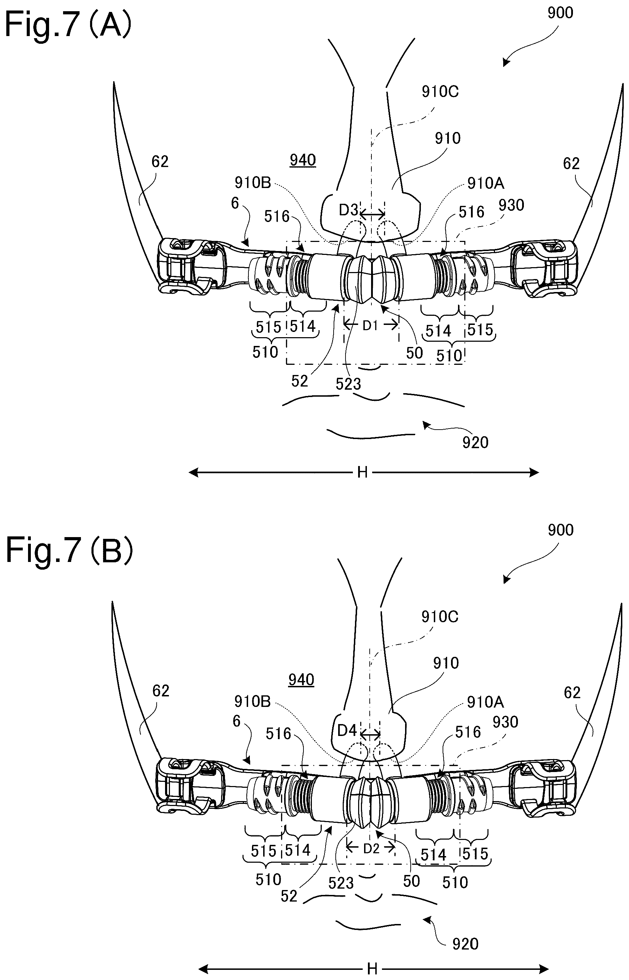

[Fig.7] (A) A front view of a user on an occasion of having mounted the communicating portion and the gas guiding portion on the user, by the mounting portion in the first embodiment of the present invention. (B) A front view of a user on an occasion of making axial spacing between second tubular portions less than in a case of (A).

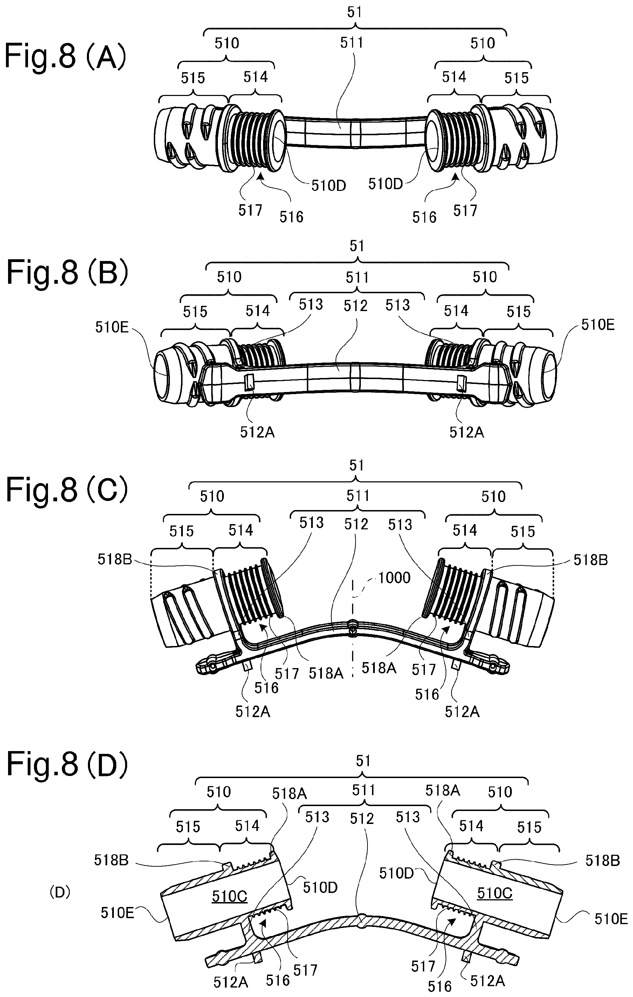

[Fig.8] (A) A front view of the communicating portion in the first embodiment of the present invention. (B) A rear view of the communicating portion in the first embodiment of the present invention. (C) A top view (bottom view) of the communicating portion in the first embodiment of the present invention. (D) A schematic cross-sectional view of the communicating portion in the first embodiment of the present invention.

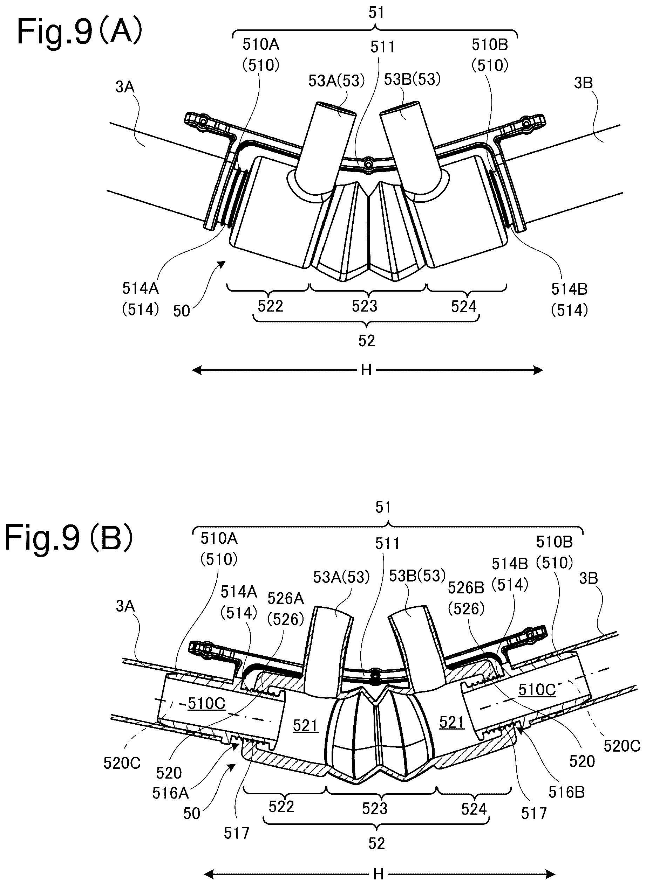

[Fig.9] (A) A top view of the gas guiding portion on an occasion of having mounted the gas guiding portion on the communicating portion in the first embodiment of the present invention. (B) A schematic cross-sectional view of the gas guiding portion on an occasion of having mounted the gas guiding portion on the communicating portion in the first embodiment of the present invention.

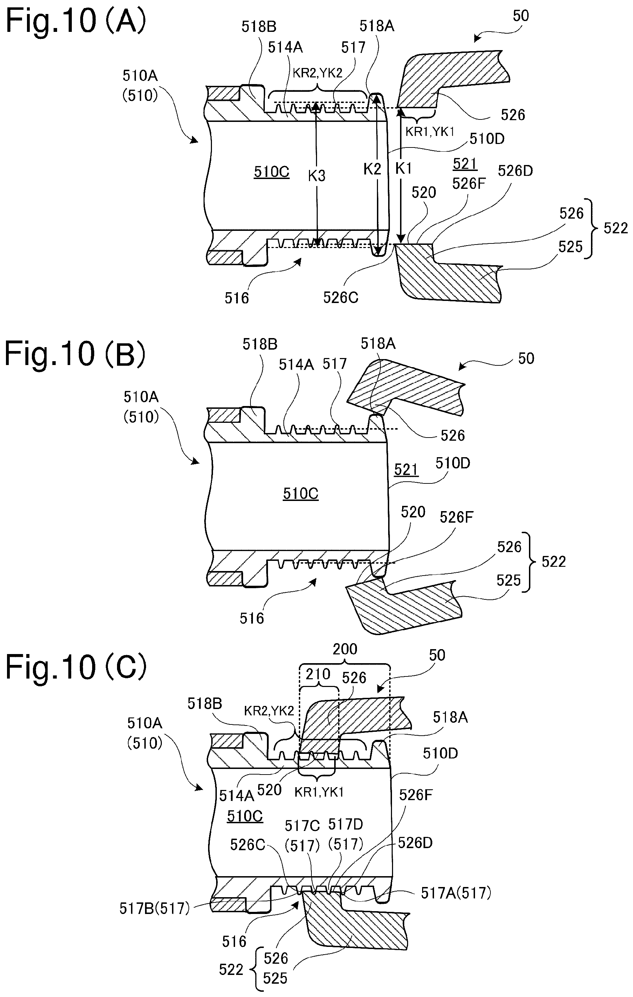

[Fig.10] (A)~(C) A schematic cross-sectional view of the gas guiding portion and the communicating portion, wherein motions on an occasion of mounting the gas guiding portion on such the communicating portion in the first embodiment of the present invention are placed in a time sequence.

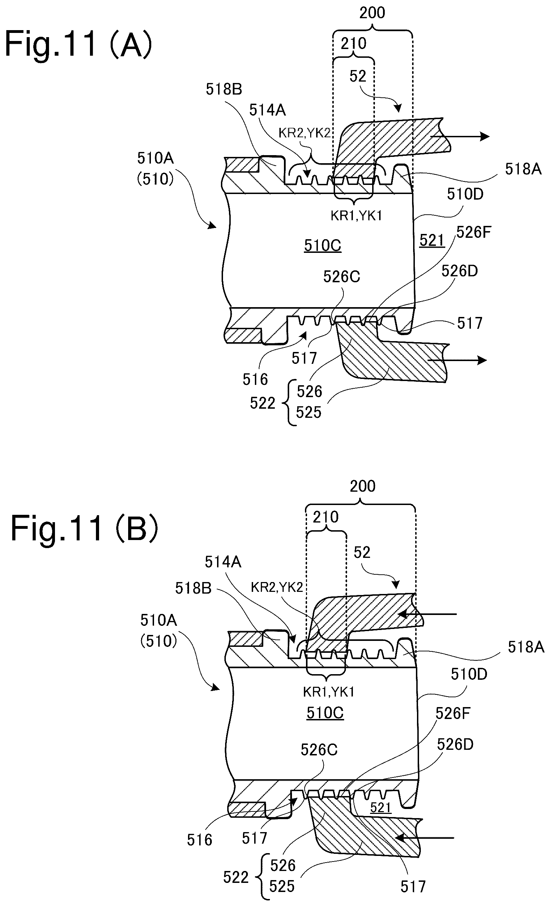

[Fig.11] (A) A schematic cross-sectional view of the gas guiding portion and the communicating portion, which shows, as shown in Fig.10(C), those after moving the gas guiding portion which is determined in position to the communicating portion, toward a tip end of an axial direction of a communicating tubular portion with respect to the communicating tubular portion. (B) A schematic cross-sectional view of the gas guiding portion and the communicating portion, which shows, as shown in Fig.10(C), those after moving the gas guiding portion which is determined in position to the communicating portion, toward a basal end of an axial direction of the communicating tubular portion with respect to the communicating tubular portion.

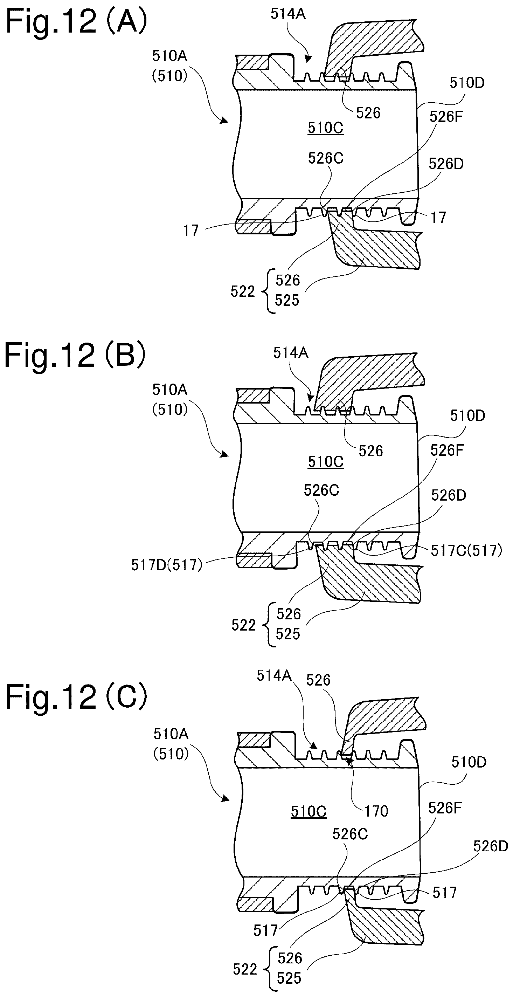

[Fig.12] (A)~(C) A schematic cross-sectional view of the gas guiding portion and the communicating portion, which shows, on an occasion of having varied thickness of a diameter-reduction portion in the first embodiment of the present invention, an aspect of engagement between an engagement portion of the communicating portion and the diameter-reduction portion thereof.

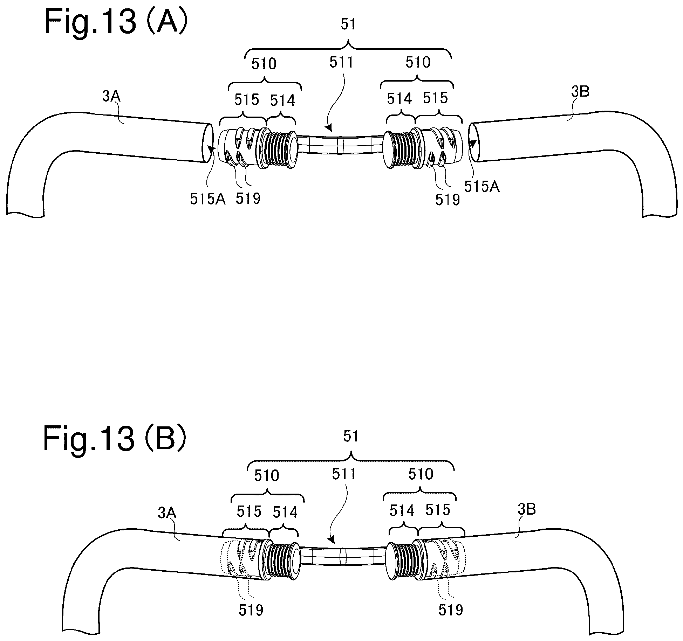

[Fig.13] (A), (B) A diagram where aspects of connecting the communicating portion in the first embodiment of the present invention to a supply pipe are placed in a time sequence.

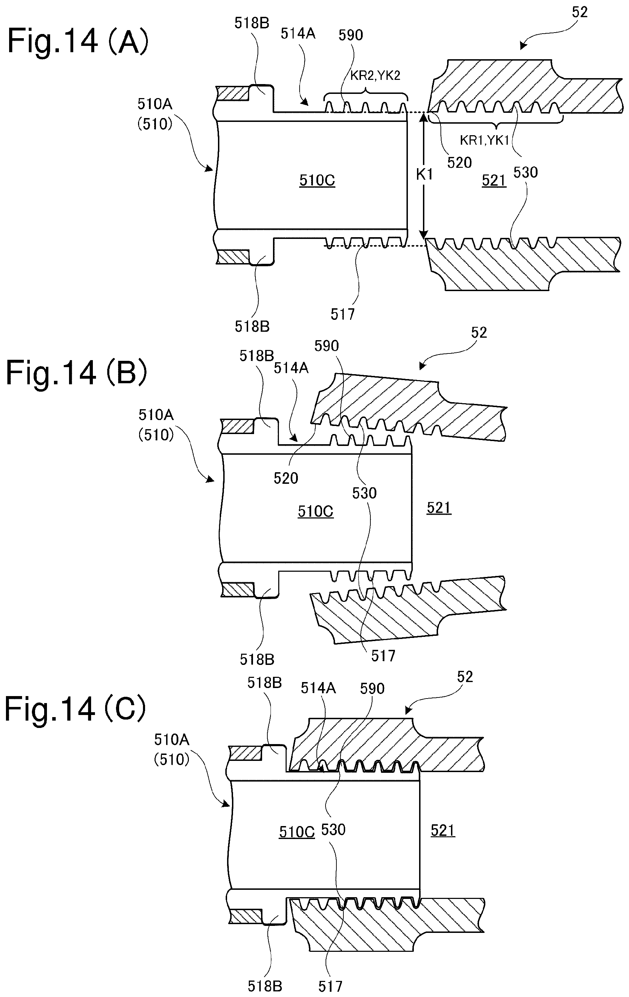

[Fig.14] (A)~(C) A schematic cross-sectional view of the gas guiding portion and the communicating portion, wherein motions on an occasion of mounting the gas guiding portion on such the communicating portion in a modification example of the first embodiment of the present invention are placed in a time sequence.

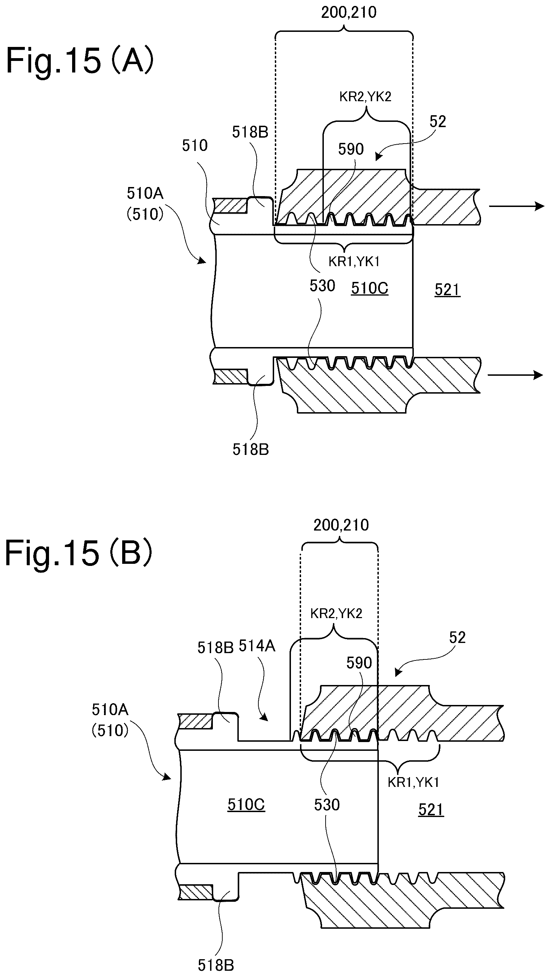

[Fig.15] (A) A schematic cross-sectional view of the gas guiding portion and the communicating portion, which shows, as shown in Fig.14(C), those after moving the gas guiding portion which is determined in position to the communicating portion, toward a tip end of an axial direction of the communicating tubular portion with respect to the communicating tubular portion. (B) A schematic cross-sectional view of the gas guiding portion and the communicating portion, which shows, as shown in Fig.14(C), those after moving the gas guiding portion which is determined in position to the communicating portion, toward a basal end of an axial direction of the communicating tubular portion with respect to the communicating portion.

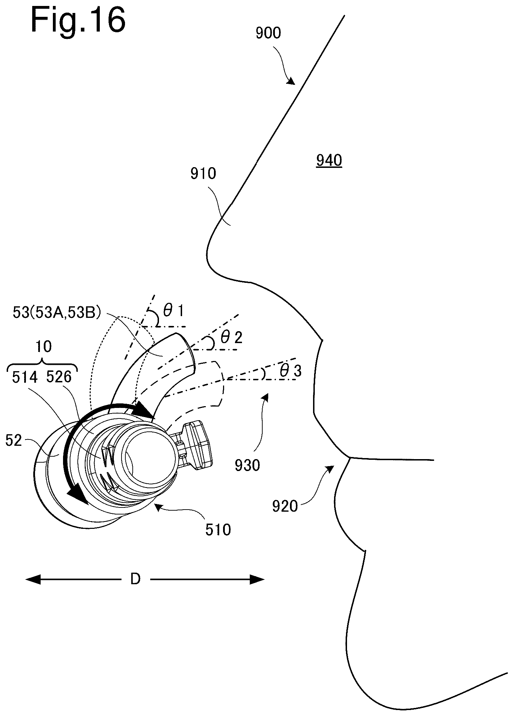

[Fig.16] A side view which shows an aspect of swinging the gas guiding portion, with respect to the communicating portion in the first embodiment of the present invention.

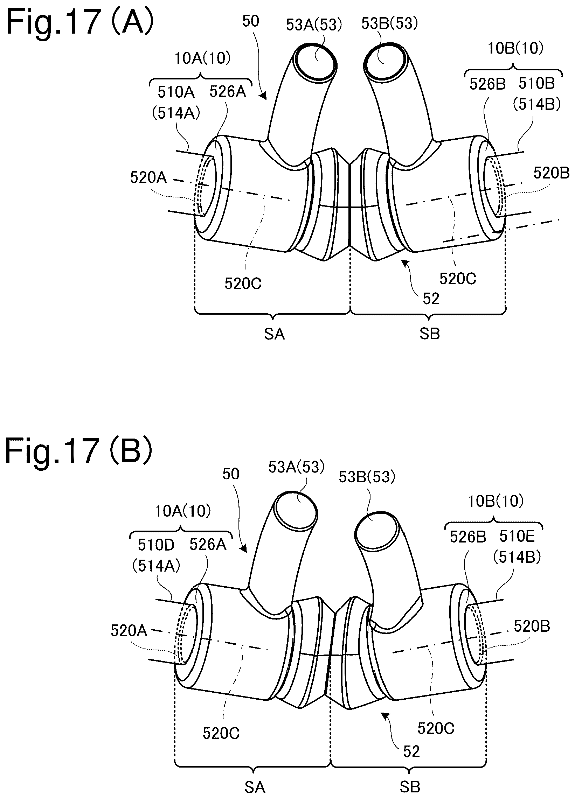

[Fig.17] (A) A rear view of the gas guiding portion on an occasion of a swing angle being identical between a first region and a second region for the first tubular portion of the gas guiding portion in the first embodiment of the present invention. (B) A rear view of the gas guiding portion, which represents an aspect of the first tubular portion being in torsion as a result of a swing angle being made different between a first region and a second region for the first tubular portion of the gas guiding portion in the first embodiment of the present invention.

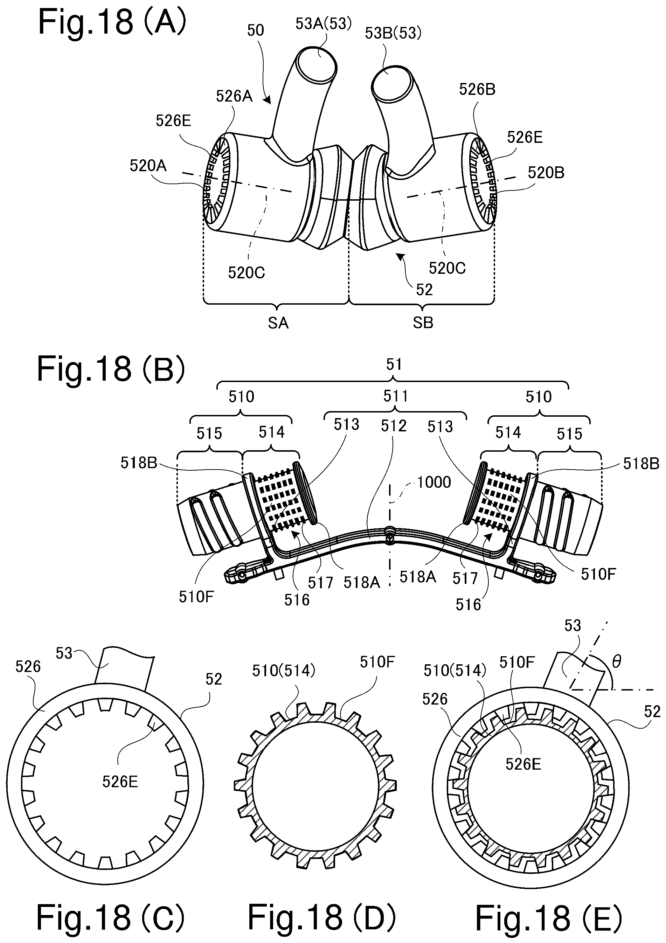

[Fig.18] (A) A rear view of the gas guiding portion in a modification example of the first embodiment of the present invention. (B) A top view (bottom view) of the communicating portion in a modification example of the first embodiment of the present invention. (C) A schematic side view of the gas guiding portion in a modification example of the first embodiment of the present invention. (D) A cross-sectional view on an occasion of having cut open the communicating tubular portion in a modification example of the first embodiment of the present invention in a direction of being perpendicular to an axial direction of the communicating tubular portion. (E) A schematic connection view which shows an aspect on an occasion of having connected the gas guiding portion to the communicating tubular portion.

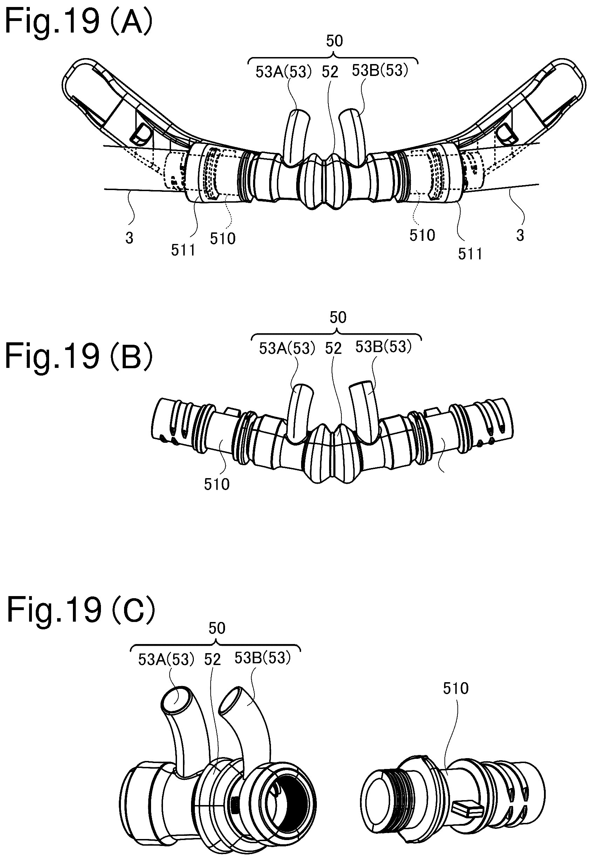

[Fig.19] (A) A front view of the breathing aid device in a second embodiment of the present invention. (B) A front view of a state of the gas guiding portion and the communicating tubular portion being connected with each other in the second embodiment of the present invention. (C) An oblique view of a state of the gas guiding portion and the communicating tubular portion being separated from each other in the second embodiment of the present invention.

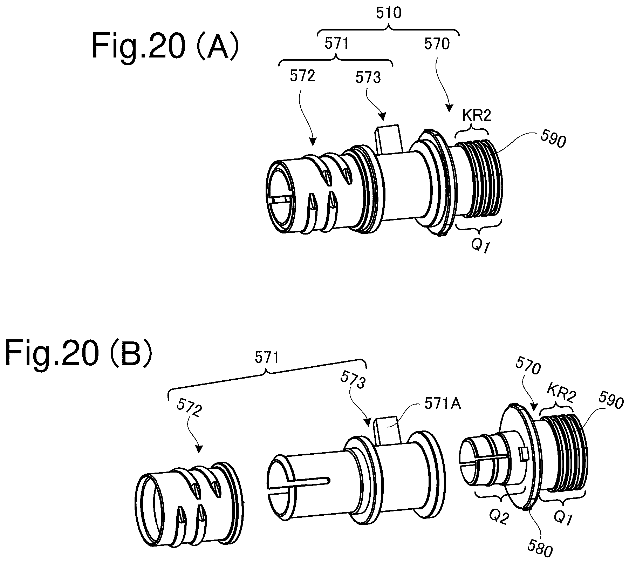

[Fig.20] (A) An oblique view of the communicating tubular portion in the second embodiment of the present invention. (B) A oblique view of a state of the communicating tubular portion in the second embodiment of the present invention being disassembled.

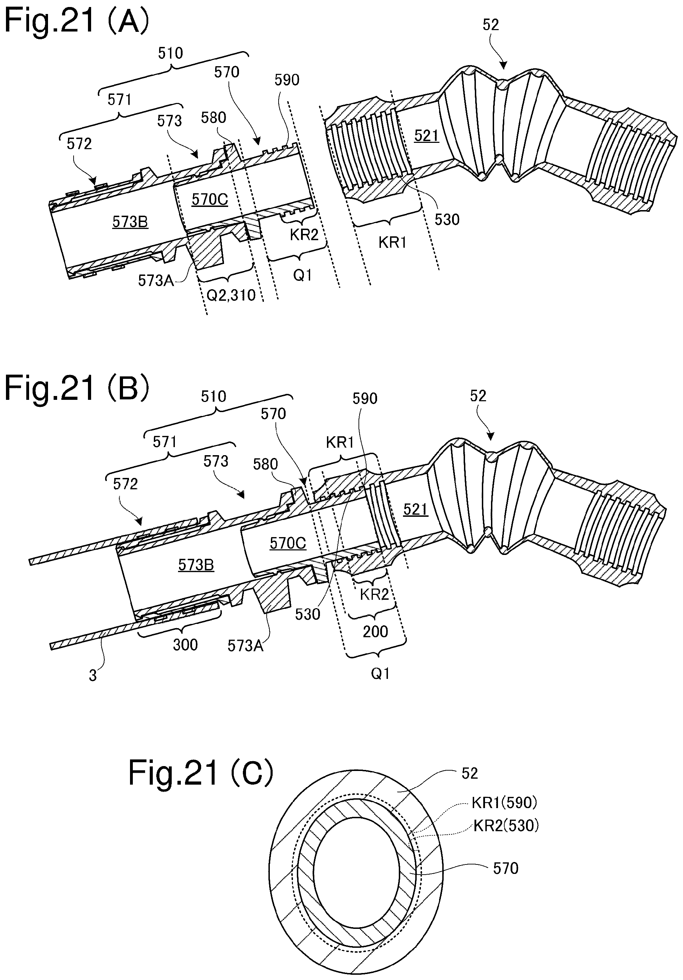

[Fig.21] (A) A cross-sectional view of having cut open the gas guiding portion and the communicating tubular portion in an axial direction of the communicating tubular portion in the second embodiment of the present invention, wherein the gas guiding portion and the communicating tubular portion are in a state of being separated from each other. (B) A cross-sectional view of having cut open the gas guiding portion and the communicating tubular portion in an axial direction of the communicating tubular portion in the second embodiment of the present invention, wherein the gas guiding portion and the communicating tubular portion are in a state of being connected with each other. (C) A cross-sectional view of having cut open an overlapping region between the gas guiding portion and the communicating tubular portion in a direction perpendicular to a central axis of the communicating tubular portion.

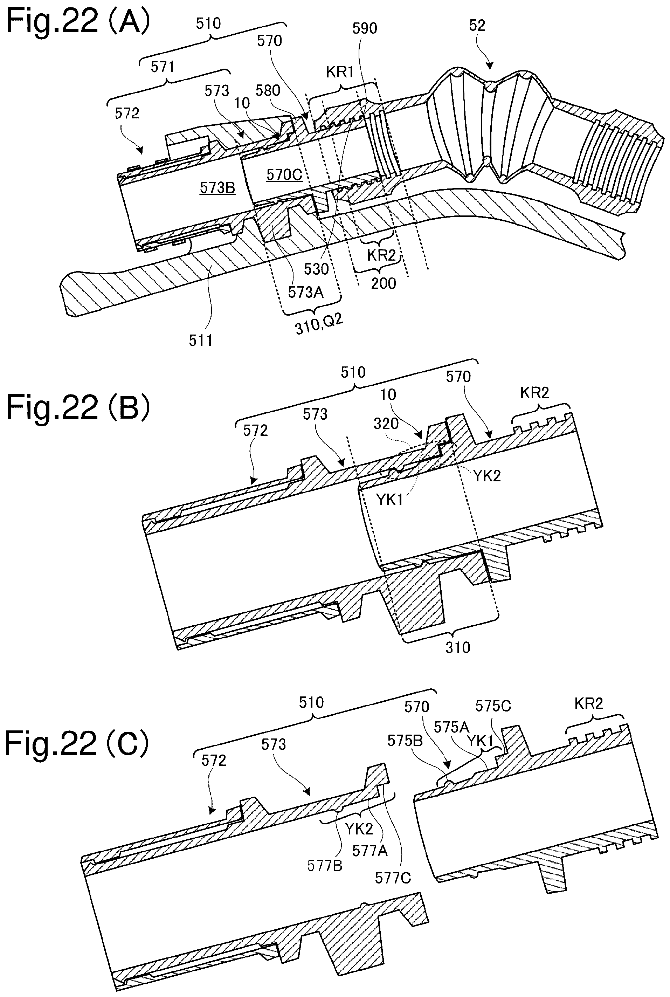

[Fig.22] (A) A cross-sectional view of having cut open the breathing aid device in the second embodiment of the present invention in an axial direction of the communicating tubular portion. (B) A cross-sectional view of having cut open the communicating tubular portion in the second embodiment of the present invention in an axial direction of the communicating tubular portion, wherein the communicating tubular portion is in a state of being not disassembled. (C) A cross-sectional view of having cut open the communicating tubular portion in the second embodiment of the present invention in an axial direction of the communicating tubular portion, wherein part of the communicating tubular portion is in a state of being disassembled.

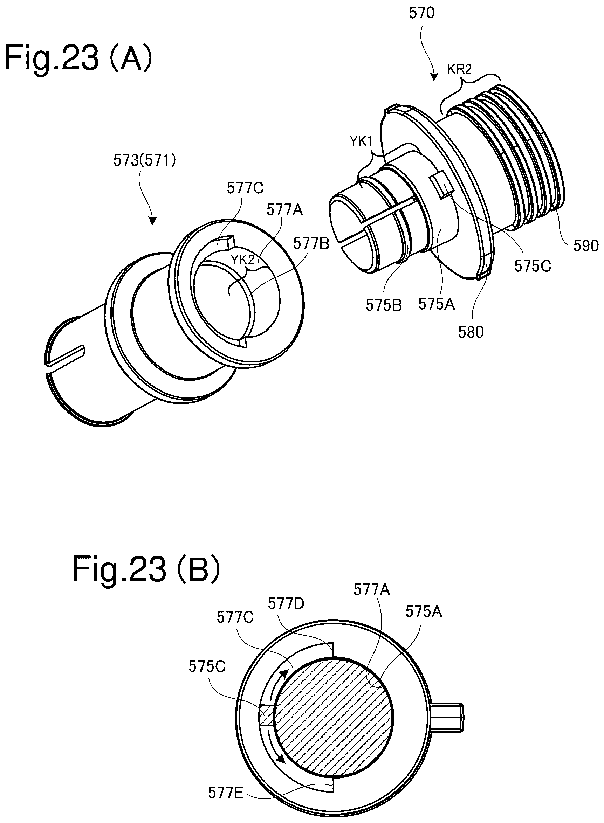

[Fig.23] (A) An oblique view of a state of a first connection tubular portion and a second connection tubular portion being disassembled in the second embodiment of the present invention. (B) A cross-sectional view of a part connected on an occasion of having connected the first connection tubular portion and the second connection tubular portion in the second embodiment of the present invention being cut open in a direction perpendicular to a central axis of the communicating tubular portion.

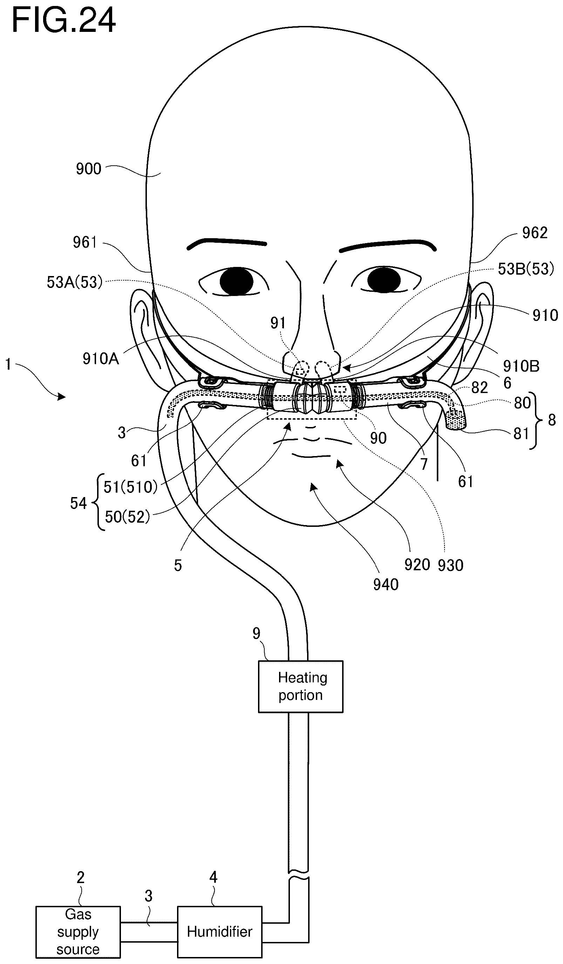

[Fig.24] A front view of a user on whom the breathing aid device in a third embodiment of the present invention is mounted.

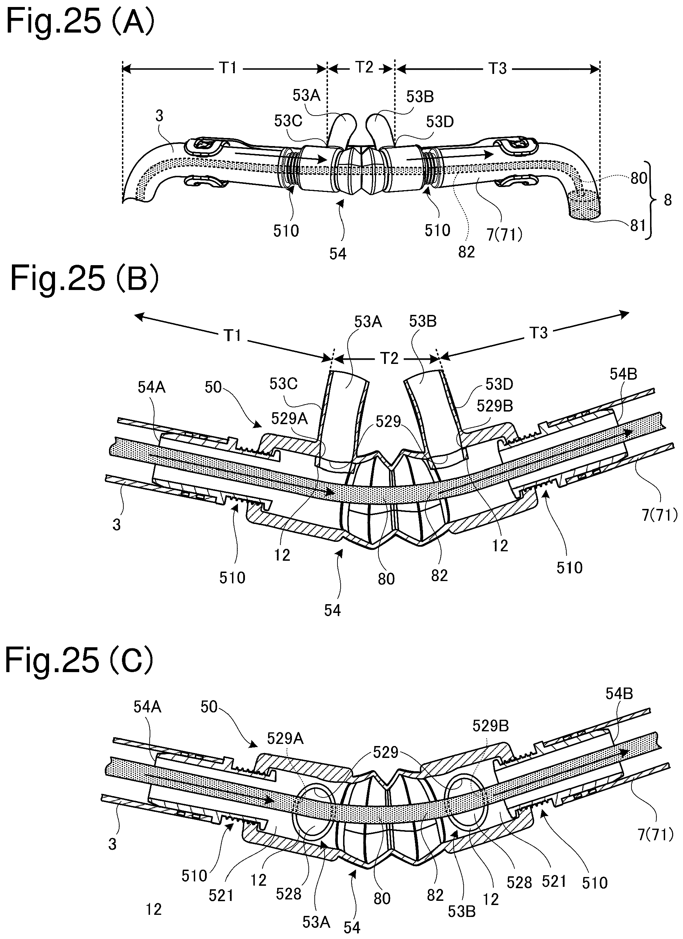

[Fig.25] (A) A front view of the breathing aid device in the third embodiment of the present invention. (B) A schematic cross-sectional view of the breathing aid device in the third embodiment of the present invention. (C) A schematic cross-sectional view of the breathing aid device in the third embodiment of the present invention which is cut open at a position different from one in (B).

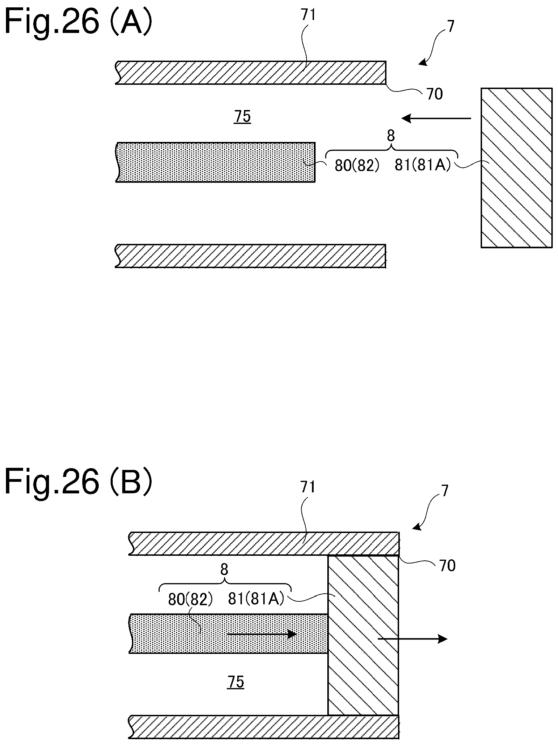

[Fig.26] (A) An enlarged schematic cross-sectional view before mounting an exposure portion of a moisture-removing portion on a gas retaining pipe in the third embodiment of the present invention. (B) An enlarged schematic cross-sectional view after mounting the exposure portion of a moisture- removing portion on the gas retaining pipe in the third embodiment of the present invention.

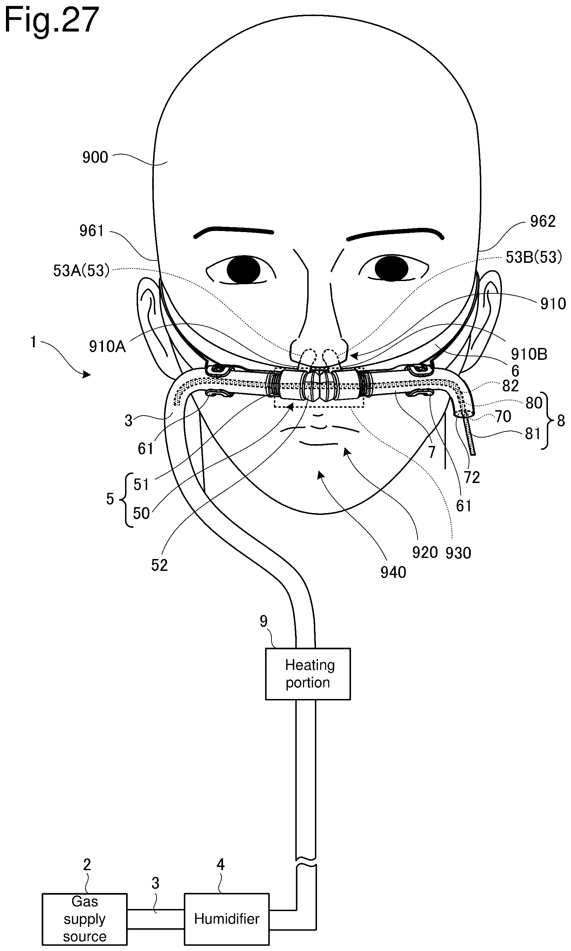

[Fig.27] A front view of a user on whom a first modification example of the breathing aid device in the third embodiment of the present invention is mounted.

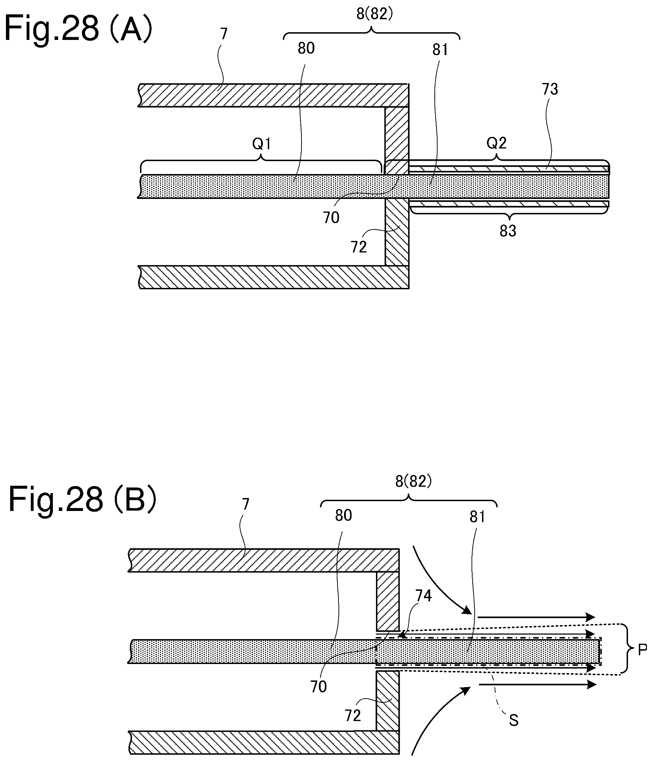

[Fig.28] (A) An enlarged schematic cross-sectional view of a vicinity of the moisture-removing portion of a first modification example of the breathing aid device in the third embodiment of the present invention. (B) An enlarged schematic cross-sectional view of a vicinity of the moisture-removing portion of a second modification example of the breathing aid device in the third embodiment of the present invention.

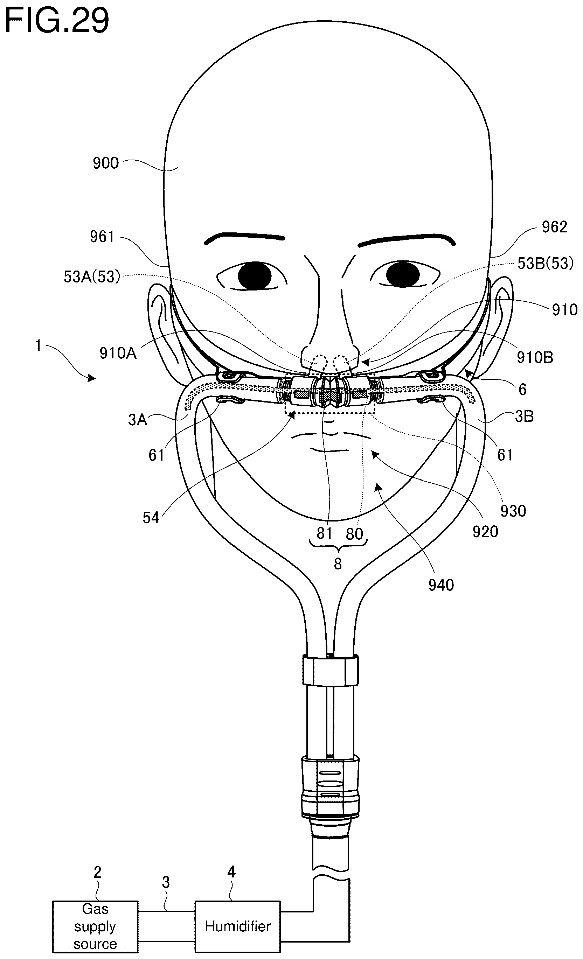

[Fig.29] A front view of a user on whom the breathing aid device in a fourth embodiment of the present invention is mounted.

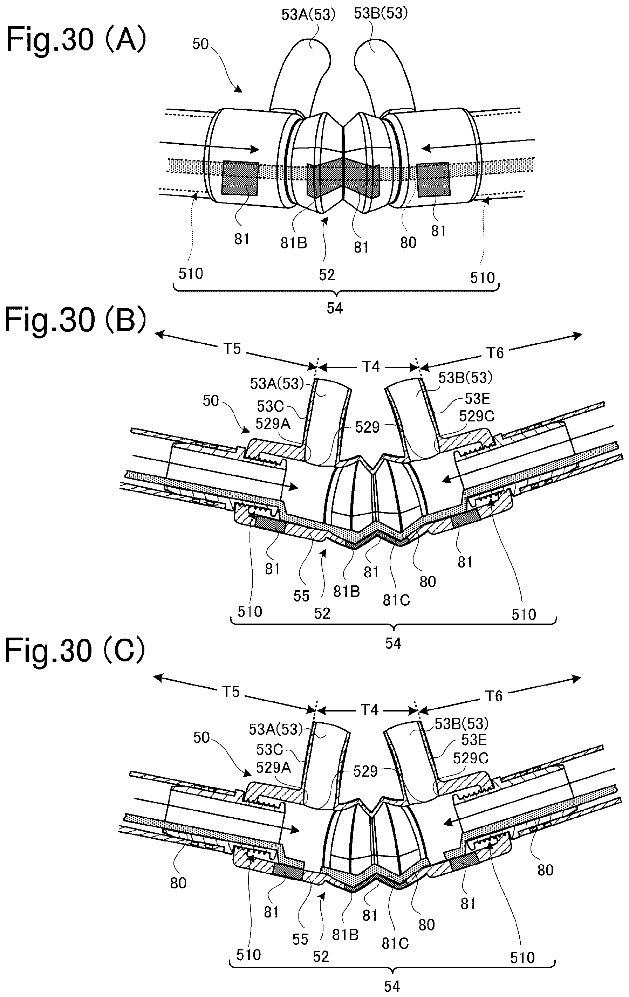

[Fig.30] (A) A front view of the breathing aid tool in the fourth embodiment of the present invention. (B) A schematic cross-sectional view of the breathing aid tool in the fourth embodiment of the present invention. (C) A schematic cross-sectional view of a modification example of the breathing aid tool in the fourth embodiment of the present invention.

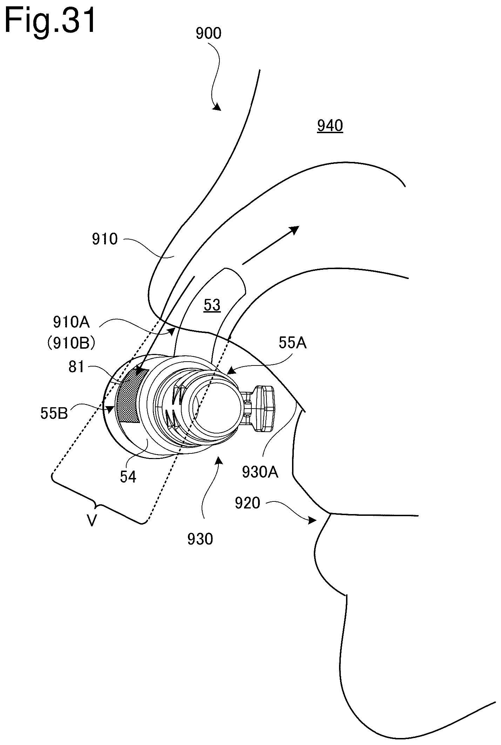

[Fig.31] A side view of a user on an occasion of mounting on the user, the breathing aid tool in the fourth embodiment of the present invention.

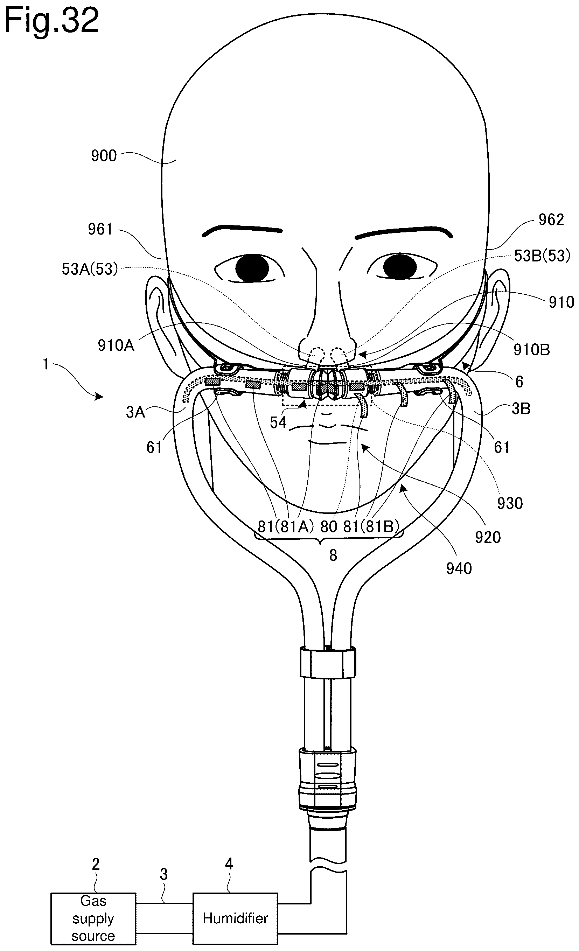

[Fig.32] A front view of a user on whom a modification example of the breathing aid tool in the fourth embodiment of the present invention is mounted.

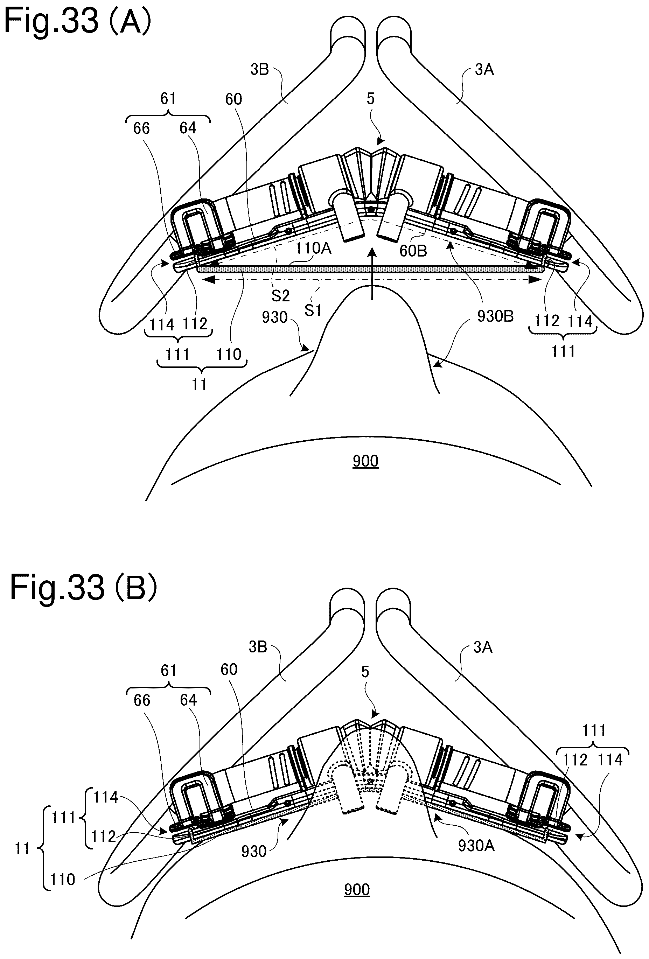

[Fig.33] (A) A top view of the breathing aid device in a fifth embodiment of the present invention, before being mounted by the user. (B) A top view of the breathing aid device in a fifth embodiment of the present invention, after being mounted by the user.

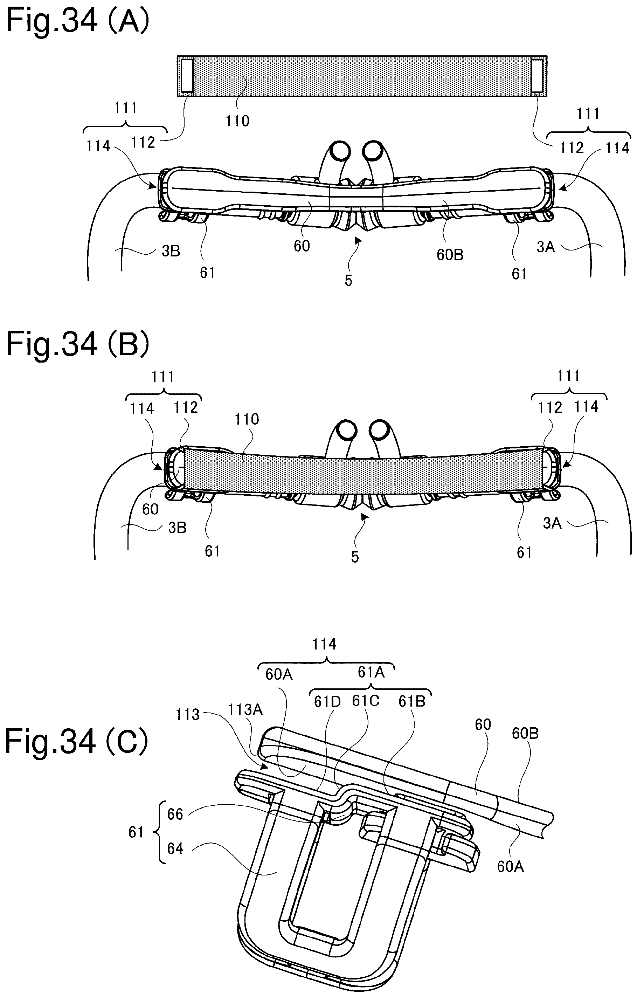

[Fig.34] (A) A rear view of the breathing aid device in a fifth embodiment of the present invention, before having an under-nose region protecting portion mounted. (B) A rear view of the breathing aid device in the fifth embodiment of the present invention, after having the under-nose region protecting portion mounted. (C) A schematic view of an engagement portion in the fifth embodiment of the present invention.

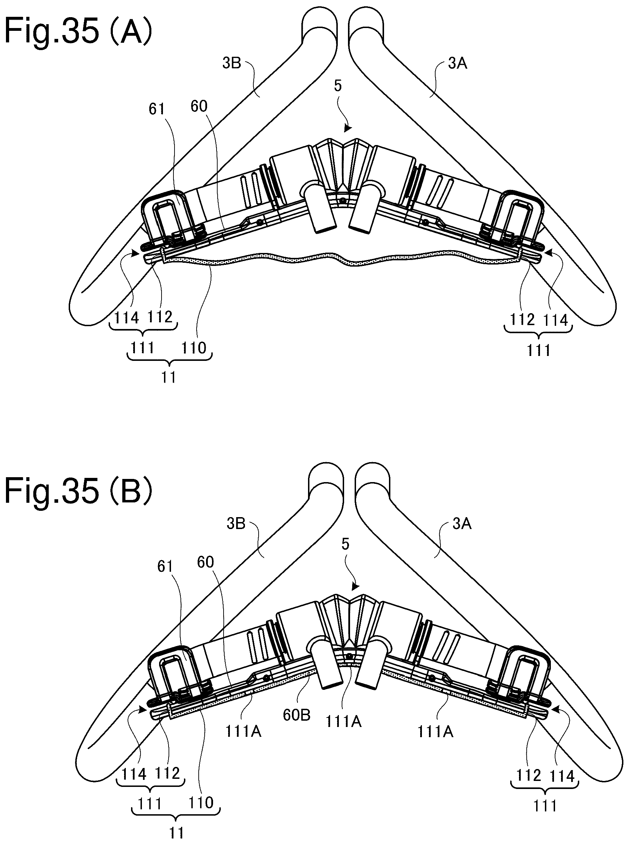

[Fig.35] (A) A top view of a first modification example of the breathing aid device in the fifth embodiment of the present invention, after having the under-nose region protecting portion mounted. (B) A top view of a second modification example of the breathing aid device in the fifth embodiment of the present invention, after having the under-nose region protecting portion mounted.

### [Embodiments of Invention]

Embodiments of the present invention are described below in reference of attached drawings. Each of Fig.1 to Fig. 35 is one example of embodiments of the invention, wherein a part given an identical numerical sign in the drawings represents an identical matter.

### <First embodiment>

A breathing aid device 1 in a first embodiment of the present invention is described in reference of Fig.1 to Fig.18. A breathing aid device 1 of the present embodiment is intended to aid in breathing, a user 900 of the breathing aid device 1 and delivers an inhalation gas (e.g., compressed air, oxygen) into an airway of the user 900. In the present embodiment, the breathing aid device 1 is provided with a gas supply source 2, a supply pipe 3, a humidifier 4, a breathing aid tool 5, and a mounting portion 6, and so on, as shown in Fig.1 and Fig.2.

A gas supply source 2 supplies gas. For that gas supply source 2, an oxygen cylinder, an air-blowing machine, and so on, being well-known, are used.

The supply pipe 3 conducts a gas supplied by the gas supply source 2. One end of the supply pipe 3 is connected to the gas supply source 2. Then, another end of the supply pipe 3 is connected to the breathing aid tool 5. The supply pipe 3 delivers gas through the breathing aid tool 5 to an airway of the user 900.

In the present embodiment, the supply pipe 3 has one pair of branch pipe pieces 3A and 3B, branching into two on a way from the gas supply source 2 to the breathing aid tool 5. The breathing aid tool 5 has one pair of connection orifices for connecting, as described later, each of the branch pipe pieces 3A and 3B. Then, a branch pipe piece 3A is connected to a connection orifice (left end shown in Fig.1) of one side of the breathing aid tool 5 while a branch pipe piece 3B is connected to a connecting orifice (right end shown in Fig.1) of another side of the breathing aid tool 5.

The humidifier 4 humidifies gas inside the supply pipe 3. Although in the present embodiment, the humidifier 4 humidifies gas inside the supply pipe 3 at any position of the supply pipe 3 before branching, it is not limited thereto and it may humidify gas inside the branch pipe pieces 3A and 3B at any positions of the branch pipe pieces 3A and 3B after branching. The humidifier 4 may be configured in any manner as long as it can humidify gas inside the supply pipe 3.

A breathing aid tool 5 guides a gas delivered through the supply pipe 3 (the branch pipe pieces 3A and 3B) to nostrils 910A and 910B of the user 900. The mounting portion 6 mounts the breathing aid tool 5 on the user 900. The breathing aid tool 5 and the mounting portion 6 are described below in detail.

### <Breathing aid tool>

A breathing aid tool 5 is described below, in reference of Fig.1 to Fig.5. The breathing aid tool 5 works, for example, as a nasal cannula to guide gas to the nostrils 910A and 910B, as shown in Fig.1. Fig.3 is intended to depict a state where the breathing aid tool 5 and the mounting portion 6, which are disposed between a nose 910 of the user 900 and a mouth 920 thereof in Fig.1 and Fig.2, are disassembled. A breathing aid tool 5 in the present embodiment has a gas guiding portion 50 and a hold mechanism, as shown in Fig.3.

### <Gas guiding portion>

The gas guiding portion 50 is described below, in reference of Fig.1, Fig.4, and Fig.5. The gas guiding portion 50 guides a gas delivered through the supply pipe 3 (the branch pipe pieces 3A and 3B) to the nostrils 910A and 910B of the user 900. The gas guiding portion 50 has a first tubular portion 52 and one pair of second tubular portions 53, as shown in Fig.4 (A).

### <First tubular portion>

The first tubular portion 52 is described, in reference of Fig.1, Fig.4, and Fig.5. The first tubular portion 52 is, as shown in Figs.4 (B), (C), shaped in a tubular form and have an opening 520 on each of both its ends. Then, the first tubular portion 52 has an internal passageway 521 leading to openings 520 of both its ends, as shown in Fig.4 (C). The internal passageway 521 extends along an axial direction (hereinafter, referred to as a first tubular side axial direction) of the first tubular portion 52 and is opened through the openings 520 of both its ends to an external portion of the first tubular portion 52.

Then, the first tubular portion 52 has a first tubular piece 522, a stretchable structure piece 523, and a second tubular piece 524, as shown in Figs.4 (A) to (C). The first tubular piece 522, the stretchable structure piece 523, and the second tubular piece 524 are aligned in order along the first tubular side axial direction and are continuous to each other. The first tubular piece 522 is configured to be in a tubular form and constitutes an axial direction section (hereinafter, referred to as a one end side section) of the first tubular portion 52 which extends in the first tubular side axial direction from one end of the first tubular portion 52 in the first tubular side axial direction. The second tubular piece 524 is configured to be in a tubular form and constitutes an axial direction section (hereinafter, referred to as an another end side section) of the first tubular portion 52 which extends in the first tubular side axial direction from another end of the first tubular portion 52 in the first tubular side axial direction. The stretchable structure piece 523 is configured to be in a tubular form and constitutes an axial direction section between the first tubular piece 522 and the second tubular piece 524. The first tubular piece 522 (one end side section) and the second tubular piece 524 (another end side section) is preferably identical in axial direction length. Additionally, although the stretchable structure piece 523 is preferably, in its initial state, identical to or less than the first tubular piece 522 and the second tubular piece 524 in axial length, it may be greater in axial direction length.

The first tubular piece 522 and the second tubular piece 524 has a pipe main body portion 525 and a diameter-reduction portion 526, as shown in Figs.4 (B), (C). The pipe main body portion 525 is shaped in a tubular form. The pipe main body portion 525 is preferably constant in inner diameter. The diameter-reduction portion 526 is a part which reduces a diameter (protrudes) inward in a radial direction of the pipe main body portion 525 by making a starting point out of a whole circumference of an inner circumference surface of both ends of the pipe main body portion 525. The diameter-reduction portion 526 can be regarded as a protrusion portion in a meaning of protruding inward in a radial direction of the pipe main body portion 525. An innermost part of the diameter-reduction portion 526 in a radial direction of the first tubular piece 522 constitutes the opening 520. An inner diameter of the opening 520 is smaller than at least, a maximum outer diameter of an engagement portion 517, which makes a basis out of a central axis of a gas guiding portion side tubular piece 514 described later. In the present embodiment, the diameter-reduction portion 526 is, as shown in Fig.4 (B), shaped in an annular form and has the opening 520 in its central region.

The stretchable structure piece 523 has a stretchable structure of being able to extend and retract in the first tubular side axial direction. When to the first tubular portion 52 in a state shown in Fig.5 (A), drawing force F is applied toward both outer sides of the first tubular portion 52 in the first tubular side axial direction, the stretchable structure piece 523 extends in the first tubular side axial direction to have its own overall length increased in its axial direction, as shown in Fig.5 (B). As a result, in the first tubular side axial direction, an overall length of the first tubular portion 52 also increases. Additionally, when to the first tubular portion 52 in a state shown in Fig.5 (A), pressing force is applied toward both inner sides thereof in the first tubular side axial direction, the stretchable structure piece 523 retracts in the first tubular side axial direction to have its own overall length decreased in its axial direction, as dispensing with being illustrated. As a result, in the first tubular side axial direction, an overall length of the first tubular portion 52 also decreases.

Although, this stretchable structure is preferably a structure which keeps, when the stretchable structure piece 523 is made to extend, its state of having extended and keeps, when the stretchable structure piece 523 is made to retract, its state of having retracted, it is not limited thereto. The stretchable structure may be, for example, a structure where such a return force as even if the stretchable structure piece 523 is made to extend or retract in the first tubular side axial direction by an external force in the first tubular side axial direction being applied to the stretchable structure piece 523, it returns to an initial state where the external force in the first tubular side axial direction is not applied to the stretchable structure piece 523 works. It is mentioned as one example that the return force is, for example, due to elastic force.

In the present embodiment, the stretchable structure piece 523 is configured to be a bellows-formed (bellows structure) pipe. It suffices that the bellows structure is, as shown in Fig.5 (A), one made of annular or spiral-form rings connected in plurality. As for the rings, a cross section which cuts the first tubular portion 52 along the first tubular side axial direction is in a convex shape which protrudes outward in a radial direction of the first tubular portion 52 (see Fig.9 (B)). The convex shape protrudes, not only on its outer circumferential side but also on its inner circumferential side, outward in a radial direction of the first tubular portion 52. That allows the first tubular portion 52 to extend and retract in the first tubular side axial direction. The stretchable structure piece 523 is not limited to one shaped in a bellows form. For example, the stretchable structure piece 523 may be in a structure of being able to extend and retract in the first tubular side axial direction by dint of elastic deformation, may be in a structure of being configured in a slack state and able to extend and retract in the first tubular side axial direction, and may be in an extendable and retractable structure where a first tubular piece of one side is slidably inserted in an axial direction into an internal portion of a second tubular piece of another side and where a region in which a first tubular piece and a second tubular piece overlap with each other is configured to be able to be increased and decreased in axial directions of the first tubular piece and the second tubular piece.

Additionally, the first tubular portion 52 is, as shown in Fig.1, held by a hold mechanism in an under-nose region 930 between the nose 910 (the nostrils 910A and 910B) of the user 900 and the mouth 920 thereof, in such a posture as the first tubular portion 52 extends in a width direction H of a face of the user 900. The hold mechanism is described later.

Although the first tubular portion 52 is preferably constituted by an elastic material, which can be elastically deformed, it is not limited thereto and may be constituted by other materials. As an elastic material, for example, an elastomer (e.g., thermosetting elastomer) which contains silicone rubber, fluororubber, urethane rubber, and so on is mentioned as one example.

### <Second tubular portion>

A second tubular portion 53 is described, in reference of Fig.1 and Fig.4. The second tubular portion 53 is a pipe which branches from the first tubular portion 52 as shown in Figs.4 (A) to (C) and two pieces thereof are provided. A second tubular portion 53 in the present embodiment works as nasal prongs of a nasal cannula, for example. Below, as appropriate, a numerical sign of a second tubular portion 53 of one side is made 53A and a numerical sign of the second tubular portion 53 of another side is made 53B, for convenience of explanation. The second tubular portion 53 may be alternatively read as a nose insertion tubular portion 53, as appropriate, since it is inserted into the nostrils 910A and 910B of the user 900.

As shown in Figs.4 (A) to (C), the second tubular portion 53A branches from the first tubular piece 522 in such a manner as protruding, by making a starting point out of an outer circumferential surface 522A around an axis of the first tubular piece 522 in the first tubular piece 522, to a side (outward in a radial direction of the first tubular piece 522) of leaving the outer circumferential surface 522A. A second tubular portion 53B branches from the second tubular piece 524 in such a manner as protruding, by making a starting point out of an outer circumferential surface 524A around an axis of the second tubular piece 524 in the second tubular piece 524, to a side (outward in a radial direction of the second tubular piece 524) of leaving the outer circumferential surface 524A. The second tubular portions 53A and 53B are disposed alongside each other, and both of them protrude from the first tubular piece 522 and the second tubular piece 524, respectively, to a same side.

A second tubular portion 53A has, as shown in Fig.1, such a shape and a size that it can be inserted into a nostril 910A of one side of the user 900. A second tubular portion 53B has, as shown in Fig.1, such a shape and a size that it can be inserted into a nostril 910B of another side of the user 900.

Each of the second tubular portions 53A and 53B has, as shown in Fig.4 (B), an outlet opening 527 on its tip end and an internal passageway 528 leading to the outlet opening 527. Additionally, as shown in Fig.4 (C), the internal passageway 528 of each of the second tubular portions 53A and 53B is continuous to the internal passageway 521 of the first tubular portion 52 through a communicating orifice 529 of each of them, and is opened to an external portion through the outlet opening 527. The communicating orifices 529 refer, respectively, to an internal opening of the gas guiding portion 50, provided on a boundary between the internal passageway 521 of the first tubular piece 522 and the internal passageway 528 of the second tubular portion 53A, and an internal opening of the gas guiding portion 50, provided on a boundary between the internal passageway 521 of the second tubular piece 524 and the internal passageway 528 of the second tubular portion 53B.

The first tubular portion 52 and the second tubular portions 53A and 53B may be formed in unity, and may be configured to be, as discrete members, able to be connected with each other. Although the second tubular portions 53A and 53B are preferably constituted by an elastic material, which can be elastically deformed, they are not limited thereto and may be constituted by other materials. As an elastic material, for example, an elastomer (e.g., thermosetting elastomer) which contains silicone rubber, fluororubber, urethane rubber, and so on is mentioned as one example. The second tubular portions 53A and 53B and the first tubular portion 52 may be constituted by an identical sort of material, and may be constituted by different kinds of materials.

Accordingly, a gas passing through the internal passageway 521 of the first tubular portion 52 is guided through the communicating orifice 529 to the internal passageway 528 of each of the second tubular portions 53A and 53B and is ejected through the outlet opening 527 into an external portion.

Then, spacing between the second tubular portions 53A and 53B can be varied by extending and retracting the stretchable structure piece 523. In other words, the stretchable structure piece 523 extends or retracts in accordance as the spacing between the second tubular portions 53A and 53B varies. As a result, the present embodiment allows spacing (hereinafter, referred to as axial direction spacing) between the second tubular portions 53A and 53B in the first tubular side axial direction to be modified in accordance with spacing between the nostrils 910A and 910B of the user 900. Hence, the above axial direction spacing between the second tubular portions 53A and 53B can be easily modified, in a manner that the second tubular portions 53A and 53B do not get in contact with nostrils of the nose 910 of the user 900.

### <Hold mechanism>

A hold mechanism is described, in reference of Fig.4, Fig.6 to Fig.12. The hold mechanism holds the gas guiding portion 50 (the first tubular portion 52) in a manner that a gas supplied through the supply pipe 3 can be supplied to the gas guiding portion 50 (the first tubular portion 52). Then, the hold mechanism has a spacing modification mechanism which allows or restricts, in accordance with an external force applied, a modification of axial spacing between second tubular portions 53A and 53B in the first tubular side axial direction. The spacing modification mechanism is, so as to be able to guide a gas supplied by the supply pipe 3 to the first tubular portion 52, connected to the first tubular portion 52, and allows or restricts, by allowing or restricting extension and retraction of the first tubular portion 52 (the stretchable structure piece 523) in accordance with a magnitude of external force applied to the first tubular portion 52 and the spacing modification mechanism and so on, a modification of axial spacing between the second tubular portions 53A and 53B in the first tubular side axial direction. Although the spacing modification mechanism has, for example, a communicating portion 51 in the present embodiment, it is not limited thereto and may be configured in other ways.

### <Communicating portion>

The communicating portion 51 is, in a manner that a gas supplied by the supply pipe 3 can be supplied to the gas guiding portion 50 (the first tubular portion 52), connected, between the supply pipe 3 and the first tubular portion 52, to both of them. That is, the communicating portion 51 is intended, in a way of receiving a gas supplied by the supply pipe 3 and passing it on to the gas guiding portion 50 (the first tubular portion 52), to communicate between the supply pipe 3 and the gas guiding portion 50 (the first tubular portion 52). Then, the communicating portion 51 is disposed in an under-nose region between the nose 910 of the user 900 and a mouth thereof as shown in Figs.6 (A), (B).

Additionally, the communicating portion 51 has a connection structure 516 which is configured in a manner that a relative connection position with respect to the gas guiding portion 50 (the first tubular portion 52) in the first tubular side axial direction (hereinafter, referred to as a relative connection position) can be modified. Then, the connection structure 516 is, as shown in Figs.7 (A), (B), configured in a manner that relative positions of the second tubular portions 53A and 53B with respect to the nostrils 910A and 910B of the user 900 can be adjusted in collaboration with the stretchable structure piece 523 of the first tubular portion 52.

Here, it is supposed that as shown in Fig.7 (A),the axial direction spacing between the second tubular portions 53A and 53B by making a basis out of tip end centers of the second tubular portions 53A and 53B is made to be D3 and that from this state, as shown in Fig.7 (B), the axial direction spacing between the second tubular portions 53A and 53B is made to be D4 by reducing the axial direction spacing in an axial direction of the second tubular portions 53A and 53B through applying external force. Note that D3>D4. When axial direction spacing in an axial direction of the second tubular portions 53A and 53B is reduced through applying external force, the stretchable structure piece 523 whose axial direction length is D1 has, as shown in Figs.7 (A), (B), its axial direction length reduced to D2 in accordance as the spacing varies. Note that D1>D2. That is, on modifying the relative connection position between the communicating portion 51 and the gas guiding portion 50 (the first tubular portion 52) while modifying the axial direction spacing between second tubular portions 53A and 53B through applying external force, the stretchable structure piece 523 varies in axial direction length in accordance as the axial direction spacing between the second tubular portions 53A and 53B varies. As a result, a relative position between the second tubular portions 53A and 53B by making a basis of the nostrils 910A and 910B of the user 900 is modified. The communicating portion 51 as above has, as shown in Figs.8 (A) to (D), one pair of communicating tubular portions 510 and a communicating tubular side hold portion 511.

### <One pair of communicating tubular portions>

Each of one pair of communicating tubular portions 510 is shaped in a tubular form and has openings 510D, 510E on both its ends, as shown in Figs.8 (A), (B). Then, each of one pair of communicating tubular portions 510 has an internal passageway 510C leading to openings 510D, 510E of both its end, as shown in Fig.8 (D).

Then, each of one pair of communicating tubular portions 510 has the gas guiding portion side tubular piece 514 and a supply pipe side tubular piece 515, as shown in Fig.8 (A) to (D). The gas guiding portion side tubular piece 514 and the supply pipe side tubular piece 515 are aligned in order along its own axial direction and are continuous to each other. On this occasion, the gas guiding portion side tubular piece 514 and the supply pipe side tubular piece 515 is preferably coaxial for their central axes, respectively.

Although each of one pair of communicating tubular portions 510 is preferably constituted by a hard material which is not elastically deformed and is higher in rigidity than the gas guiding portion 50, it is not limited thereto and may be constituted by other materials. As a hard material, for example, a resin such as polypropylene, polyethylene, or metal is mentioned as one example.

### <Gas guiding portion side tubular piece>

The gas guiding portion side tubular piece 514 is described in reference of Fig.8 to Fig.12. The gas guiding portion side tubular piece 514 is, as shown in Figs.8 (A) to (D), shaped in a tubular form and constitutes one section of a communicating tubular portion 510. Then, the gas guiding portion side tubular piece 514 has the connection structure 516 which can be connected to the first tubular portion 52 (the first tubular piece 522, the second tubular piece 524). The connection structure 516 has a plurality of positions which can be connected to the first tubular portion 52 in an axial direction (hereinafter, referred to as a communicating side axial direction) of the gas guiding portion side tubular piece 514 (the communicating tubular portion 510).

The connection structure 516 is, as shown in Figs.8 (A) to (D), provided in plurality with intervals in the communicating side axial direction on an outer circumferential surface of the gas guiding portion side tubular piece 514 (the communicating tubular portion 510), and has the engagement portion 517 configured to be able to be engaged with the diameter-reduction portion 526 each. While each of the engagement portions 517 protrudes outward in a radial direction of the gas guiding portion side tubular piece 514 in an outer circumferential surface of the gas guiding portion side tubular piece 514, it circles in a circumferential direction of the gas guiding portion side tubular piece 514. In such a sense, each of the engagement portions 517 is allowed to be referred to as a protrusion portion. In the present embodiment, the engagement portion 517 is configured to be an annular protrusion ring which fully circles an outer circumferential surface of the gas guiding portion side tubular piece 514 in a circumferential direction of the gas guiding portion side tubular piece 514. The engagement portion 517 is not limited thereto and may be configured to be a protrusion ring which does not fully circle in a circumferential direction of the gas guiding portion side tubular piece 514 but goes partially around (partially circles) an outer circumferential surface of the gas guiding portion side tubular piece 514.

Although a plurality of engagement portions 517 are preferably aligned at an equal interval, they may be at an unequal interval. Additionally, the engagement portions 517 are preferably equal to each other in height in a radial direction. That is, the engagement portions 517 are preferably equal to each other in a maximum outer diameter by making a basis out of a central axis of the gas guiding portion side tubular piece 514. Additionally, stoppers 518A and 518B are greater in height in a radial direction than the engagement portions 517. That is, the stoppers 518A and 518B are greater in a maximum outer diameter by making a basis out of the central axis of the gas guiding portion side tubular piece 514 than the engagement portions 517.

Additionally, the connection structure 516 has, on a tip end of an axial direction of the gas guiding portion side tubular piece 514 and a basal end (a boundary between the gas guiding portion side tubular piece 514 and the supply pipe side tubular piece 515) thereof, the stoppers 518A and 518B which circles, while protruding outward in a radial direction of the gas guiding portion side tubular piece 514 in an outer circumferential surface of the gas guiding portion side tubular piece 514, in a circumferential direction of the gas guiding portion side tubular piece 514. In the present embodiment, the stoppers 518A and 518B is configured to be an annular protrusion ring which fully circles an outer circumferential surface of the gas guiding portion side tubular piece 514 in a circumferential direction of the gas guiding portion side tubular piece 514. The stoppers 518A and 518B is not limited thereto and may be configured to be a protrusion ring which does not fully circle in a circumferential direction of the gas guiding portion side tubular piece 514 but goes partially around (hereinafter referred to as partially circles) an outer circumferential surface of the gas guiding portion side tubular piece 514.

When in a case where each of one pair of communicating tubular portions 510 (a gas guiding portion side tubular pieces 514) is defined as a first communicating tubular portion 510A and a second communicating tubular portion 510B (a first gas guiding portion side tubular piece 514A and a second gas guiding portion side tubular piece 514B), the first tubular portion 52 is connected to the first communicating tubular portion 510A and the second communicating tubular portion 510B (the first gas guiding portion side tubular piece 514A and the second gas guiding portion side tubular piece 514B) as shown in Figs.9 (A), (B), the first gas guiding portion side tubular piece 514A and the second gas guiding portion side tubular piece 514B are, in the present embodiment, inserted through openings 520 of both ends of the first tubular portion 52 (the first tubular piece 522 and second tubular piece 524) to an internal portion of the first tubular portion 52. Then, an inner diameter K1 of the openings 520 of the first tubular portion 52 (the diameter-reduction portion 526) in an initial state where no external force is applied is, as shown in Fig.10 (A), smaller than a maximum outer diameter K2 of the stopper 518A by making a basis out of a central axis of the first gas guiding portion side tubular piece 514A. Hence, it is necessary to deform the first tubular portion 52 elastically by applying external force to the first tubular portion 52 in a manner of widening the openings 520 temporarily as shown in Fig.10 (B). Since the first tubular portion 52 is constituted by an elastic material, which can be elastically deformed, the openings 520 can be widened temporarily.

On inserting the first gas guiding portion side tubular piece 514A into an internal portion of the first tubular portion 52 through the opening 520 of the first tubular portion 52 and clearing external force which is applied, the first tubular portion 52 is, as shown in Fig.10 (C), elastically deformed in a manner that the opening 520 which is widened returns to an inner diameter K of its initial state. As a result, an innermost surface 526F of the diameter-reduction portion 526 which corresponds to the opening 520 gets in contact with the engagement portion 517 from an apical portion side of the engagement portion 517.

Additionally, the inner diameter K1 of the openings 520 of the first tubular portion 52 is, as shown in Fig.10 (A), smaller than a maximum outer diameter K3 of an engagement portions 517, which is based on the central axis of the gas guiding portion side tubular piece 514. Hence when the innermost surface 526F of the diameter-reduction portion 526 gets in contact with the engagement portion 517 from an apical portion side of the engagement portion 517, the diameter-reduction portion 526 presses the engagement portion 517 from the apical portion side of the engagement portion 517. On this occasion, the innermost surface 526F is, as shown in Fig.10 (C), elastically deformed by being pressed by engagement portions 517C and 517D to be deformed in a recessed form. Then, friction force due to the engagement portions 517C and 517D being pressed by the diameter-reduction portion 526 is generated between the engagement portions 517C and 517D and the diameter-reduction portion 526. As a result, the engagement portions 517C and 517D engage with the diameter-reduction portion 526 in the communicating side axial direction, a relative movement (hereinafter, referred to as an axial direction relative movement) therebetween in the communicating side axial direction is restricted. Below, an engagement in the communicating side axial direction is referred to as an axial direction engagement in some case, and an engagement force which works on both sides of engagement counterparts due to the axial direction engagement is referred to as an axial direction engagement force in some case, as appropriate.

Additionally, an internal portion side vicinity region 526D of the diameter-reduction portion 526, being a vicinity of the innermost surface 526F (opening 520) of diameter-reduction portion 526 abuts on an engagement portion 517A from a circumferential surface side of the engagement portion 517A, as shown in Fig.10 (C). As a result, since the internal portion side vicinity region 526D and the engagement portion 517A are engaged (axially engaged: hereinafter, same) with each other in the communicating side axial direction, the axial direction relative movement to a side vicinal to the stopper 518A in the communicating side axial direction is restricted. In the present embodiment, the internal portion side vicinity region 526D of the diameter-reduction portion 526 corresponds to an internal portion side surface of the diameter-reduction portion 526, being continuous to the innermost surface 526F of the diameter-reduction portion 526, or an internal portion side angular portion thereof.

Additionally, an external portion side vicinity region 526C of the diameter-reduction portion 526, being a vicinity of the innermost surface 526F of the diameter-reduction portion 526 abuts on an engagement portion 517B from a circumferential surface side of the engagement portion 517B, which is located in a side closer to the supply pipe side tubular piece 515 than the engagement portion 517A. As a result, since the external portion side vicinity region 526C and the engagement portion 517B are engaged with each other in the communicating side axial direction, the axial direction relative movement toward the side approaching the stopper 518B in the communicating side axial direction is restricted. In the present embodiment, the external portion side vicinity region 526C of the diameter-reduction portion 526 corresponds to an external portion side surface of the diameter-reduction portion 526, being continuous to the innermost surface 526F of the diameter-reduction portion 526, or an external portion side angular portion thereof. As above, the diameter-reduction portion 526 and the engagement portion 517 are engaged with each other, and as a result, the axial direction relative movement between the diameter-reduction portion 526 and the engagement portion 517 is restricted.

The above description can be similarly applied to a relation between the first tubular portion 52 and the second communicating tubular portion 510B (the second gas guiding portion side tubular piece 514B). Hence, the diameter-reduction portion 526 of the first tubular portion 52 is, by its engagement in the communicating side axial direction with engagement portions 517A to 517D of each of the first communicating tubular portion 510A and the second communicating tubular portion 510B, restricted from moving relatively in an axial direction with respect to the first communicating tubular portion 510A and the second communicating tubular portion 510B (the first gas guiding portion side tubular piece 514A and the second gas guiding portion side tubular piece 514B). As a result, the relative connection position of the diameter-reduction portion 526 in the connection structure 516 is finalized. When the first communicating tubular portion 510A and the second communicating tubular portion 510B (the first gas guiding portion side tubular piece 514A and the second gas guiding portion side tubular piece 514B) are inserted into the first tubular portion 52, the communicating side axial direction and the first tubular side axial direction are substantially in parallel with each other.

In a case of modifying the relative connection position of the diameter-reduction portion 526 in the connection structure 516 shown in Fig.10 (C), an external force (hereinafter, referred to as an axial direction external force) in the communicating side axial direction is, in order to make, as shown in Figs.11 (A), (B), the diameter-reduction portion 526 relatively move in an axial direction with respect to the connection structure 516 (the first gas guiding portion side tubular piece 514A), given to the first tubular portion 52 or the communicating tubular portion 510, for example. Since the diameter-reduction portion 526 is, on this occasion, elastically deformed by the axial direction external force, engagement between the diameter-reduction portion 526 and the engagement portion 517 is removed, and therefore, the diameter-reduction portion 526 is allowed to move relatively in its axial direction with respect to the first communicating tubular portion 510A (the first gas guiding portion side tubular piece 514A). Specifically, on applying, for example, for the diameter-reduction portion 526 to move relatively in an axial direction on a side approaching the stopper 518A of the first communicating tubular portion 510A (the first gas guiding portion side tubular piece 514A) or on a side approaching the stopper 518B thereof, the axial direction external force to the first tubular portion 52 or the communicating tubular portion 510, the innermost surface 526F (opening 520) of the diameter-reduction portion 526 and a vicinity region thereof is, while surmounting friction force, elastically deformed due to being pressed by the engagement portion 517, and together with the pipe main body portion 525, moves relatively in an axial direction with respect to the first communicating tubular portion 510A (the first gas guiding portion side tubular piece 514A) on a side approaching the stopper 518A of the communicating side axial direction or on a side approaching the stopper 518B thereof. Then, when the axial direction external force is removed, the diameter-reduction portion 526 and the engagement portion 517 are engaged with each other in the communicating side axial direction, and the axial direction relative movement of the diameter-reduction portion 526 with respect to the first communicating tubular portion 510A is restricted. A relative connection position between the connection structure 516 and the diameter-reduction portion 526 is modified thereby.

After the above relative connection position is modified, the internal portion side vicinity region 526D of the diameter-reduction portion 526 and the external portion side vicinity region 526C thereof get in contact with a circumferential surface of another engagement portion 517. On this occasion, such friction force as described above is generated between the innermost surface 526F (opening 520) of the diameter-reduction portion 526 and that other engagement portion 517, and the diameter-reduction portion 526 is, by that friction force, engaged with that other engagement portion 517.

Axial direction engagement which acts between the diameter-reduction portion 526 and the engagement portion 517 in the present embodiment is mainly generated due to an elastic force of the diameter-reduction portion 526, a friction force between the diameter-reduction portion 526 and the engagement portion 517, and contact in the communicating side axial direction between the diameter-reduction portion 526 and the engagement portion 517. Hence, for removing axial direction engagement between the diameter-reduction portion 526 and the engagement portion 517, it is necessary to apply the axial external force which is sufficient to cause sufficient elastic deformation of the diameter-reduction portion 526 or to overcome the frictional force between the diameter-reduction portion 526 and the engagement portion 517,to at least one of the first tubular portion 52 and the first communicating tubular portion 510A.. When a magnitude of the axial direction external force is equal to or less than that of the axial direction engagement force acting between the diameter-reduction portion 526 and the engagement portion 517, axial direction engagement between the diameter-reduction portion 526 and the engagement portion 517 is not removed, and the axial direction relative movement between the diameter-reduction portion 526 and the engagement portion 517 is restricted. Hence, the axial direction external force is required to have an extent enough to exceed the axial direction engagement force. When the axial direction external force has an extent enough to exceed the axial direction engagement force, axial direction engagement between the diameter-reduction portion 526 and the engagement portion 517 is removed, and the diameter-reduction portion 526 is allowed to move relatively in an axial direction with respect to the engagement portion 517. The above description of a modification of the relative connection position can be similarly applied to a relation between the first tubular portion 52 and the second communicating tubular portion 510B (the second gas guiding portion side tubular piece 514B).

Although the above makes a description by presupposing that the engagement portion 517 is constituted by a hard material which is not elastically deformed and is higher in rigidity than the gas guiding portion 50, the engagement portion 517 may be constituted by an elastic material. In this case, it follows that the engagement portion 517 is also elastically deformed due to pressing force which it receives from the diameter-reduction portion 526. Additionally, it is allowed that the engagement portion 517 is constituted by an elastic material and that the diameter-reduction portion 526 is constituted by a hard material higher in rigidity than the engagement portion 517. In this case, it follows that the engagement portion 517 is elastically deformed due to pressing force which it receives from the diameter-reduction portion 526.

Although in the present embodiment, the diameter-reduction portion 526 is, as shown in Fig.10 and Fig.11, determined in position by being engaged (in contact) with engagement portions 517 which interpose two engagement portions 517 therebetween, limitation thereto should not be made. The diameter-reduction portion 526 may, for example, have such thickness as it is engaged (in contact) with engagement portions 517 which lie at a position where to place, for example, one (see Fig.12 (A)), or three or more engagement portions 517 therebetween.

Additionally, As shown in Figure 12 (B), the diameter-reduction portion 526 may have a thickness such that either the internal portion side vicinity region 526D or the external portion side vicinity region 526C of the diameter-reduction portion 526 is engaged with (contacts) the corresponding engagement portion 517 (the former is engagement portion 517C, and the latter is engagement portion 517D, which is located closer to the supply pipe side tubular piece 515 than engagement portion 517C). **In** this case, the diameter-reduction portion 526 may be disposed at such a position that neither the internal portion side vicinity region 526D nor the external portion side vicinity region 526C is not engaged (in contact) with the engagement portion 517.

Additionally, the diameter-reduction portion 526 is allowed to be, as shown in Fig.12 (C), one that has such a thickness that it can be inserted into a trough region 170 between adjacent engagement portions 517. **In** this case, a position where the first tubular portion 52 and a connection portion 16 are able to be connected with each other can be increased in number more than in other modes above.

In a case where the diameter-reduction portion 526 is constituted by an elastic material, the diameter-reduction portion 526 is easy to deform elastically when the diameter-reduction portion 526 decreases in thickness, whereas the diameter-reduction portion 526 is hard to deform elastically when the diameter-reduction portion 526 increases in thickness. In consideration of a degree of elastic deformation of the diameter-reduction portion 526, thickness of the diameter-reduction portion 526, intervals of the engagement portions 517 adjacent thereto, and so on are decided.

As mentioned above, as for the diameter-reduction portion 526, the relative connection position of the diameter-reduction portion 526 in the connection structure 516 is finalized by a friction force which it generates with the engagement portion 517 due to pressing force against the engagement portion 517, and/or engagement with the engagement portion 517 due to contact between the diameter-reduction portion 526 and the engagement portion 517 in the communicating side axial direction.

As above, spacing in an axial direction between second tubular portions 53A and 53B can be varied by modifying the relative connection position (a relative position) in the communicating side axial direction of the diameter-reduction portion 526 with respect to the gas guiding portion side tubular piece 514 and making the stretchable structure piece 523 of the first tubular portion 52 extend and retract on accordance therewith. As a result, a relative position in the communicating side axial direction between the second tubular portions 53A and 53B with respect to the nostrils 910A and 910B of the user 900 can be adjusted finely. Hence, since adjustment can be carried out in a manner that the second tubular portions 53A and 53B are not in contact with a nasal cavity of the user 900, the user can be prevented from being made to feel unpleasant.

It can be considered that by the diameter-reduction portion 526 and the engagement portion 517, a connection position modification mechanism which is formed between the first tubular portion 52 and one pair of communicating tubular portions 510 and which allows or restricts, in accordance with an external force applied, a modification of the relative connection position between the first tubular portion 52 and the communicating tubular portion 510 in the communicating side axial direction will be constituted. The connection position modification mechanism allows, when having a given external force (a connection position modification side external force) applied, a relative movement between the first tubular portion 52 and the communicating tubular portion 510 to allow a connection position between the first tubular portion 52 and the communicating tubular portion 510 to be modified, and restricts, when having the given external force removed, the relative movement between the first tubular portion 52 and the communicating tubular portion 510. Additionally, it can be considered that by the stretchable structure piece 523 of the first tubular portion 52, and a connection position modification mechanism, the above spacing modification mechanism or a second tubular portion relative position modification mechanism which can modify relative positions of second tubular portions 53A and 53B with respect to the nostrils 910A and 910B of the user 900.

Additionally, the first tubular portion 52 is provided with the diameter-reduction portion 526 on each of both its ends in the first tubular side axial direction. Here, as shown in Fig.9 (B), the diameter-reduction portion 526 on one end side of the first tubular side axial direction is defined as a first diameter-reduction portion 526A, the diameter-reduction portion 526 on another end side of the first tubular side axial direction as a second diameter-reduction portion 526B, the connection structure 516 corresponding to the first diameter-reduction portion 526A as a first connection structure 516A, the connection structure 516 corresponding to the second diameter-reduction portion 526B as a second connection structure 516B, the gas guiding portion side tubular piece 514 provided with the first connection structure 516A as the first gas guiding portion side tubular piece 514A, and the gas guiding portion side tubular piece 514 provided with the second connection structure 516B as the second gas guiding portion side tubular piece 514B. A relative connection position of the first diameter-reduction portion 526A with respect to the first connection structure 516A and the relative connection position of the second diameter-reduction portion 526B with respect to the second connection structure 516B do not depend on each other, and each of them can be set freely.

In Fig.9 (B), the first gas guiding portion side tubular piece 514A and the second gas guiding portion side tubular piece 514B are identical to each other in axial direction length. Then, the first connection structure 516A has six pieces of the engagement portion 517, which are aligned at equal intervals on the first gas guiding portion side tubular piece 514A. The second connection structure 516B is in such a structure as the first connection structure 516A and has six pieces of the engagement portion 517, which are aligned at equal intervals on the second gas guiding portion side tubular piece 514B. In Fig.9 (B), it is engagement portions 517 third and fourth by being counted from inside that are engaged with the first diameter-reduction portion 526A, whereas it is engagement portions 517 fourth and fifth by being counted from inside that are engaged with the second diameter-reduction portion 526B. That is, the relative connection position between the first diameter-reduction portion 526A and the first connection structure 516A, and the relative connection position between the second diameter-reduction portion 526B and the second connection structure 516B may be both in a left-right symmetry and in a left-right asymmetry, and each of them can be set at a free position independently of each other. Hence, even in a case where positions of the nostrils 910A and 910B of the user 900 are in a left-right asymmetry by making a basis out of a center line where one pair of communicating tubular portions 510 pass a center of the width direction H of a face 940 of the user 900 and which extends in a length direction of the face 940, positions of second tubular portions 53A and 53B can be adjusted to positions of the nostrils 910A and 910B, by adjusting, to positions which that user 900 has arranged, the relative connection position between the first diameter-reduction portion 526A and the first connection structure 516A, and the relative connection position between the second diameter-reduction portion 526B and the second connection structure 516B.

It can be considered that by the first diameter-reduction portion 526A and a plurality of engagement portions 517 of the first gas guiding portion side tubular piece 514A, a first connection position modification mechanism which is configured to be between the first tubular portion 52 and one pair of communicating tubular portions 510 and which allows or restricts, in accordance with an external force applied, a modification of the relative connection position between the first tubular portion 52 and the communicating tubular portion 510 (the first communicating tubular portion 510A) of one side in the communicating side axial direction will be constituted. Additionally, it can be considered that by the second diameter-reduction portion 526B and the engagement portion 517 of the second gas guiding portion side tubular piece 514B, a second connection position modification mechanism which is configured to be between the first tubular portion 52 and one pair of communicating tubular portions 510 and which allows or restricts, in accordance with an external force applied, a modification of the relative connection position between the first tubular portion 52 and the communicating tubular portion 510 (the second communicating tubular portion 510B) of another side in the communicating side axial direction will be constituted. Then, a first connection position modification mechanism and a second connection position modification mechanism remove axial direction engagement between the diameter-reduction portion 526 and the engagement portion 517, allow the axial direction relative movement therebetween, and allow a modification of the relative connection position therebetween, when the axial direction external force is applied to at least either the first tubular portion 52 or the communicating tubular portion 510, whereas they restrict, when having the axial direction external force removed, the axial direction relative movement therebetween by axial direction engagement between the diameter-reduction portion 526 and the engagement portion 517, and restrict (refuse) a modification of the relative connection position therebetween.

An axial direction external force exceeds the axial direction engagement force acting between the diameter-reduction portion 526 and the engagement portion 517 in extent. When the axial direction external force exceeds the axial direction engagement force in extent, axial direction engagement therebetween is, when the axial direction external force is applied, removed so that the axial direction relative movement between the diameter-reduction portion 526 and the engagement portion 517 which correspond is allowed and that a modification of the relative connection position therebetween is allowed. On the other hand, when a magnitude of the axial direction external force is equal to or less than that of the axial direction engagement force, engagement between the diameter-reduction portion 526 and the engagement portion 517 which correspond is maintained or not removed so that the axial direction relative movement therebetween is restricted and that a modification of the relative connection position therebetween is refused. Here, The above axial direction engagement forces which are generated in a first connection position modification mechanism and a second connection position modification mechanism are respectively defined as a first axial direction engagement force and a second axial direction engagement force, whereas axial direction external forces which are required to modify the relative connection position in the first connection position modification mechanism and the second connection position modification mechanism are respectively defined as a first axial direction external force and a second axial direction external force. A first axial direction engagement force and a second axial direction engagement force can be set in extent, independently of each other and freely, in accordance with configurations of a first connection position modification mechanism and a second connection position modification mechanism. In accordance with such setting, a first axial direction external force and a second axial direction external force vary in extent, too.

When the relative connection position between both is modified, a relative position (hereinafter, simply referred to as a communicating side axial direction relative position) in the communicating side axial direction by making a basis out of the communicating tubular portion 510 of a specific position (hereinafter, simply referred to as a specific position of the first tubular portion) such as an end portion of the first tubular portion 52 in the first tubular side axial direction is also modified. Hence, the relative connection position between both may be alternatively read as the communicating side axial direction relative position of the specific position of the first tubular portion. In this case, a connection position modification mechanism, a first connection position modification mechanism, and a second connection position modification mechanism may be alternatively read, respectively, as a relative position modification mechanism, a first relative position modification mechanism, and a second relative position modification mechanism, which allow or restrict, in accordance with an external force applied, a modification of the communicating side axial direction relative position of the specific position of the first tubular portion. As a result, a first connection position modification mechanism (a first relative position modification mechanism) and a second connection position modification mechanism (a second relative position modification mechanism) can freely modify as for positions of second tubular portions 53A and 53B. A specific position of a first tubular portion refers to a position of the first tubular portion 52 which can be, in accordance as the first tubular portion 52 extends and retracts, changed in a relative position by making a basis out of the communicating tubular portion 510 in the communicating side axial direction. Although as the specific position of the first tubular portion, an end portion or end portion vicinity of the first tubular portion 52 is mentioned for example, limitation thereto should not be made, and another position suffices.

Additionally, although in the present embodiment, a mode wherein the communicating tubular portion 510 is inserted into the internal passageway 521 of the first tubular portion 52 and wherein the communicating tubular portion 510 is disposed in the internal passageway 521 comes out as shown in Fig.9 (B), limitation thereto should not be made, and on the contrary, a mode wherein the first tubular portion 52 is inserted into the internal passageway 510C of the communicating tubular portion 510 and wherein the first tubular portion 52 is disposed in the internal passageway 510C also suffices. That is, when an tubular portion of a side making insertion is defined as an insertion portion and when a tubular portion of a side accepting the insertion portion is defined as an acceptance portion, it follows that either of the first tubular portion 52 and the communicating tubular portion 510 is an insertion portion whereas a rest other thereof is an acceptance portion. When the first tubular portion 52 and the communicating tubular portion 510 are connected with each other as above, a region 200 (hereinafter, referred to as an overlapping region) wherein a case of being seen in a radial direction of the first tubular portion 52 or the communicating tubular portion 510, the first tubular portion 52 and the first communicating tubular portion 510A are overlapped with each other is formed between the first tubular portion 52 and the communicating tubular portion 510, as shown in Figs.11 (A), (B). A radial direction of the first tubular portion 52 or communicating tubular portion 510 is equivalent to a direction perpendicular to the first tubular side axial direction or communicating side axial direction.

As described above, on modifying the relative connection position (the communicating side axial direction relative position of the specific position of the first tubular portion 52) with a first connection position modification mechanism (a first relative position modification mechanism), a second connection position modification mechanism (a second relative position modification mechanism), and so on, the first tubular portion 52 extend and retracts in accordance therewith, and as a result, a length (hereinafter, referred to as an axial length) of the overlapping region 200 in the communicating side axial direction is also modified. For example, when it is assumed that a relative connection position between both is modified from a state shown in Fig.11 (A) to a state shown in Fig.11 (B), it follows that such a modification that increases an axial length of the overlapping region 200 has been made. In a case of making a basis out of an axial length of the overlapping region 200, it can be considered that an overlapping region axial length modification mechanism which allows or restricts, in accordance with the axial direction external force applied, a modification of an axial length of the overlapping region 200 will be constituted by the diameter-reduction portion 526, and the engagement portion 517 of the gas guiding portion side tubular piece 514.

In the present invention, as shown in Fig.10 (A), a region including the diameter-reduction portion 526 can be extended to the concept of a first engagement region KR1 provided in a region on an inner circumferential side or outer circumferential side of the first tubular portion 52, whereas a region including a plurality of engagement portions 517 can be extended to the concept of a second engagement region KR2 provided in a region on an inner circumferential side or outer circumferential side of the communicating tubular portion 510. As for the first engagement region KR1, at least one piece is provided on both sides by making a basis out of a middle of the first tubular portion 52 in the first tubular side axial direction. Additionally, the second engagement region KR2 is configured to be, on an occasion of having connected the first tubular portion 52 and the communicating tubular portion 510, engaged, while overlapping with the first engagement region KR1 in a radial direction of the communicating tubular portion 510, therewith in the communicating side axial direction. The first engagement region KR1 can be referred to as a first axial engagement region KR1. Additionally, the second engagement region KR2 can be referred to as a second axial engagement region KR2. Engagement between the first engagement region KR1 and the second engagement region KR2 in the communicating side axial direction is mainly due to at least either a friction force between the first engagement region KR1 and the second engagement region KR2 or contact between the first engagement region KR1 and the second engagement region KR2 in the communicating side axial direction.

Then, the first engagement region KR1 and the second engagement region KR2 have a structure by which they can, in accordance with the axial direction external force applied, restrict the axial direction relative movement between the first engagement region KR1 and the second engagement region KR2 by mutual axial engagement therebetween to restrict a modification of a present state of the first tubular portion 52 and the communicating tubular portion 510, or allow, by removing mutual engagement therebetween, the above axial direction relative movement therebetween to allow a modification of a present state of the first tubular portion 52 and the communicating tubular portion 510. Specifically, when the axial direction external force is applied to at least either of the first tubular portion 52 and the communicating tubular portion 510, engagement between the first engagement region KR1 and the second engagement region KR2 is removed, the axial direction relative movement between the first engagement region KR1 and the second engagement region KR2 is allowed, and the above modification of a present state of the first tubular portion 52 and the communicating tubular portion 510 is allowed. In a case where engagement between the first engagement region KR1 and the second engagement region KR2 includes an element due to a mutual friction force therebetween in the communicating side axial direction, that mutual engagement is removed by the axial direction external force becoming greater than the friction force. Additionally, in a case where engagement between the first engagement region KR1 and the second engagement region KR2 includes an element due to mutual contact therebetween in the communicating side axial direction, that mutual engagement is removed, for example, by at least either of the first engagement region KR1 and the second engagement region KR2 being, when having the axial direction external force applied, elastically deformed to remove mutual contact in the communicating side axial direction. However, limitation thereto should not be made, and all other configurations wherein mutual contact is removed in accordance with a magnitude of the axial direction external force are included in a scope of the present invention. Then, when the axial direction external force above is removed, the first engagement region KR1 and the second engagement region KR2 are engaged with each other so that a state where the axial direction relative movement between the first engagement region KR1 and the second engagement region KR2 is restricted comes out. As a result, the above modification of a present state of the first tubular portion 52 and the communicating tubular portion 510 is finalized. A present state of the first tubular portion 52 and the communicating tubular portion 510 includes a present relative connection position (engagement position) therebetween, a present axial length of the overlapping region 200, a present communicating side axial direction relative position of the specific position of the first tubular portion 52, and so on.

When a magnitude of the axial direction external force above is equal to or less than that of the axial direction engagement force acting between the first engagement region KR1 and the second engagement region KR2, axial direction engagement between the diameter-reduction portion 526 and the engagement portion 517 is not removed, and the above modification of a present state of the first tubular portion 52 and the communicating tubular portion 510 is restricted (refused), and therefore, the axial direction external force is required to have an extent enough to exceed the axial direction engagement force. When the axial direction external force has an extent enough to exceed the axial direction engagement force, engagement between the diameter-reduction portion 526 and the engagement portion 517 is removed, and the first engagement region KR1 and the second engagement region KR2 are allowed to move relatively in an axial direction with respect to each other, and the above modification of a present state of the first tubular portion 52 and the communicating tubular portion 510 is allowed. When the axial direction relative movement between the first engagement region KR1 and the second engagement region KR2 is allowed, an axial length of the overlapping region 200 is modified by an overlapping region axial length modification mechanism. As a result of this, a first connection position modification mechanism (a first relative position modification mechanism), a second connection position modification mechanism (a second relative position modification mechanism), and an overlapping region axial length modification mechanism are extended to ones that include the first engagement region KR1 and the second engagement region KR2. When, thereafter, the above axial direction external force is removed or a magnitude of the axial direction external force applied is equal to or less than that of the axial direction engagement force, the axial direction relative movement between the first engagement region KR1 and the second engagement region KR2 is restricted, and the first engagement region KR1 and the second engagement region KR2 are engaged with each other at an engagement position (a relative connection position) different from that before the axial direction relative movement.

In a case where axial direction engagement acting between the first engagement region KR1 and the second engagement region KR2 is due to a plurality of forces such as an elastic force and a friction force, a magnitude of the axial direction engagement force between the first engagement region KR1 and the second engagement region KR2 can vary within a specific range by dint of interaction of such a plurality of forces. Hence, a magnitude of the axial direction external force required to remove axial direction engagement acting between the first engagement region KR1 and the second engagement region KR2 can be invariable, and one which can vary within a specific range in accordance with an engagement force for each engagement state between the first engagement region KR1 and the second engagement region KR2 also suffices. This can vary in accordance with an engagement mode between the first engagement region KR1 and the second engagement region KR2.

Further, modification examples of the first engagement region KR1 and the second engagement region KR2 are described below in reference of Fig.14. The second engagement region KR2 of the present modification example has, as shown in Fig.14 (A), a protrusion portion 590 which is provided in plurality with spacing along the communicating side axial direction and protrudes from an outer circumferential surface of the gas guiding portion side tubular piece 514 outward in a radial direction of the gas guiding portion side tubular piece 514. The protrusion portion 590 may be configured to have a configuration like that of the engagement portion 517 above. Each of a plurality of protrusion portions 590 is configured to be, for example, an annular or spiral-form protrusion ring which fully circles or partially circles an outer circumferential surface of the gas guiding portion side tubular piece 514 in a circumferential direction of the gas guiding portion side tubular piece 514. On the other hand, the first engagement region KR1 of the present modification example is configured to have, as shown in Fig.14 (A), a plurality of groove portions 530 which is provided in plurality with spacing along the first tubular side axial direction and can be fitted into each of the plurality of protrusion portions 590. The plurality of groove portions 530 is provided with spacing like each spacing between the plurality of protrusion portions 590. In a case where each of the plurality of protrusion portions 590 is configured to be a protrusion ring, each of the plurality of groove portions 530 is configured to be an annular or spiral-form annular groove which is, while fully circling or partially circling on an inner circumferential surface of the first tubular portion 52 in a circumferential direction of an inner circumferential side of the first tubular portion 52, recessed in a radial direction of the first tubular portion 52.

If the first tubular portion 52 is constituted by a material which can be elastically deformed, it shall be made so that by applying an external force so as to temporarily widen the opening 520 of the first tubular portion 52, as shown in Fig.14 (B) to insert the communicating tubular portion 510 into the internal passageway 521 of the first tubular portion 52, the communicating tubular portion 510 is disposed in the internal passageway 521 of the first tubular portion 52. When it is, by external force being removed, elastically deformed for the opening 520 of the first tubular portion 52 to return to its initial state with the inner diameter K1, each of the plurality of protrusion portions 590 is, as shown in Fig.14 (C), fitted into each of the plurality of groove portions 530, whereby the first engagement region KR1 and the second engagement region KR2 are engaged with each other in the communicating side axial direction.

When the axial direction external force is applied to at least either of the first tubular portion 52 and the communicating tubular portion 510, engagement by the plurality of groove portions 530 (the first engagement region KR1) and the plurality of protrusion portions 590 (the second engagement region KR2) being fitted into each other is removed, the axial direction relative movement between the plurality of groove portions 530 (the first engagement region KR1) and the plurality of protrusion portions 590 (the second engagement region KR2) is allowed, and the above modification of a present state of the first tubular portion 52 and the communicating tubular portion 510 is allowed. Then, when the axial direction external force above is removed, the axial direction engagement between the plurality of groove portions 530 (the first engagement region KR1) and the plurality of protrusion portions 590 (the second engagement region KR2) due to fitting therebetween makes a state where the axial direction relative movement between the groove portions 530 (the first engagement region KR1) and the plurality of protrusion portions 590 (the second engagement region KR2) is restricted come out. As a result, the above modification of a present state of the first tubular portion 52 and the communicating tubular portion 510 is finalized.

In the present modification example, a magnitude of the axial direction engagement force acting between the first engagement region KR1 and the second engagement region KR2 differs in accordance with a count of the groove portions 530 and the plurality of protrusion portions 590 which are fitted. That is, the larger the count of the groove portions 530 and the plurality of protrusion portions 590 which are fitted, the greater the axial direction engagement force, whereas the smaller the count of the groove portions 530 and the plurality of protrusion portions 590 which are fitted, the less the axial direction engagement force. Additionally, the axial direction engagement force is due to a plurality of forces such as an elastic force and a friction force. Hence, in the present modification example, the axial direction external force above which are required to remove engagement between the first engagement region KR1 and the second engagement region KR2 can also vary within a specific range in accordance with an engagement force for each engagement state between the first engagement region KR1 and the second engagement region KR2. In any case, when a magnitude of the axial direction external force above is equal to or less than that of the axial direction engagement force, the axial direction relative movement between the first engagement region KR1 and the second engagement region KR2 is restricted by axial direction engagement between the first engagement region KR1 and the second engagement region KR2. On the other hand, when the axial direction external force above exceeds the axial direction engagement force in extent, the first engagement region KR1 or the second engagement region KR2 is, as shown in Fig.15 (A), is elastically deformed sufficiently so that axial direction engagement between the first engagement region KR1 and the second engagement region KR2 is removed and that the axial direction relative movement between the first engagement region KR1 and the second engagement region KR2 is allowed. When, thereafter, the above axial direction external force is removed or a magnitude of the axial direction external force applied is equal to or less than that of an engagement force, the axial direction relative movement between the first engagement region KR1 and the second engagement region KR2 is restricted as shown in Fig.15 (B), and the first engagement region KR1 and the second engagement region KR2 are engaged (fitted) with each other at an engagement position (a relative connection position) different from that before the axial direction relative movement.

Here, A region (hereinafter, referred to as an engagement overlapping region) 210 where on an occasion of viewing the first tubular portion 52 or the communicating tubular portion 510 in a radial direction, the first engagement region KR1 and the second engagement region KR2 are overlapped with each other shall be defined. An axial length of the engagement overlapping region 210 varies, as shown in Figs.15 (A), (B), between that before and that after the axial direction relative movement between the first engagement region KR1 and the second engagement region KR2. In an embodiment shown in Figs.11 (A), (B), for example, an axial length of the engagement overlapping region 210 of Fig.11 (A) and an axial length of the engagement overlapping region 210 of Fig.11 (B) are identical to each other, and an axial length of the engagement overlapping region 210 is invariable, whereas a position in the communicating side axial direction of the engagement overlapping region 210 varies. In any case, the first engagement region KR1 and the second engagement region KR2 are configured to have, in accordance as the overlapping region 200 is modified in axial length and relative connection position, their engagement overlapping region 210 vary between that before and that after the axial direction relative movement between the first engagement region KR1 and the second engagement region KR2, within a given range in the communicating side axial direction.

A mode wherein, contrary to the above, the plurality of protrusion portions 590 in the present modification example shown in Fig.15 (A) is provided in the first engagement region KR1 while the plurality of groove portions 530 therein is provided in the second engagement region KR2 also suffices. Likewise, a mode wherein the engagement portion 517 in the present embodiment shown in Fig.11 (A) to (C) is provided in the first engagement region KR1 while the diameter-reduction portion 526 therein is provided in the second engagement region KR2 also suffices.

To put the above together, either of the first engagement region KR1 and the second engagement region KR2 has at least one protrusion portion which protrudes, in a state where they are engaged with each other, toward a rest other of the first engagement region KR1 and the second engagement region KR2. That protrusion portion corresponds to the diameter-reduction portion 526 in the present embodiment and the protrusion portion 590 in the present modification example. The rest other of the first engagement region KR1 and the second engagement region KR2 has a plurality of engagement portions which is provided in plurality with intervals in the first tubular side axial direction or in the communicating side axial direction and is configured to be, while respectively facing protrusion portions in a state of being engaged with each other, able to be engaged with the protrusion portions. That engagement portion corresponds to the engagement portion 517 in the present embodiment and a groove portion 530 in the present modification example. Then, that protrusion portion and that engagement portion are engaged with each other in the communicating side axial direction, and are configured to restrict a relative movement of the specific position of the first tubular portion 52 in the communicating side axial direction with respect to the communicating tubular portion 510.

Additionally, the first engagement region KR1 and the second engagement region KR2 are not what are limited to configurations as above, and include all configurations wherein mutual engagement can be maintained or removed in accordance with a magnitude of the axial direction external force applied, wherein simultaneously, on an occasion where axial direction engagement has been removed, an axial length of the overlapping region 200 and the relative connection position above varying, and wherein meanwhile, the engagement overlapping region 210 varies within a given range in the communicating side axial direction. Then, when either of the first engagement region KR1 and the second engagement region KR2 is constituted by an elastic deformation material, which can be elastically deformed, one that is constituted by an elastic deformation material is elastically deformed in a case where a magnitude of the axial direction external force exceeds that of the axial direction engagement force between the first engagement region KR1 and the second engagement region KR2, and axial direction engagement between the first engagement region KR1 and the second engagement region KR2 is removed. On the other hand, in a case where a magnitude of the axial direction external force applied is equal to or less than that of the axial direction engagement force, the one that is constituted by an elastic deformation material is not elastically deformed sufficiently, and axial direction engagement between the first engagement region KR1 and the second engagement region KR2 is maintained.

### <Supply pipe side tubular piece>

The supply pipe side tubular piece 515 is described in reference of Fig.13. The supply pipe side tubular piece 515 has a connection orifice 515A to connect the branch pipe pieces 3A and 3B, as shown in Figs.13 (A), (B). By the supply pipe side tubular piece 515 being inserted into the branch pipe pieces 3A and 3B, the supply pipe side tubular piece 515 is connected to the branch pipe pieces 3A and 3B. A gas supplied by the branch pipe pieces 3A and 3B through the connection orifice 515A is accepted by the supply pipe side tubular piece 515.

The supply pipe side tubular piece 515 has, as shown in Fig.13 (A), a plurality of protruding portions 519 which, while protruding outward in a radial direction of the supply pipe side tubular piece 515 in an outer circumferential surface of the supply pipe side tubular piece 515, circles in a circumferential direction of the supply pipe side tubular piece 515. The protruding portion 519 are, in the present embodiment, formed by partially circling an outer circumferential surface of the supply pipe side tubular piece 515 in a circumferential direction of the supply pipe side tubular piece 515. The protruding portion 519 are not limited thereto and may fully circle an outer circumferential surface of the supply pipe side tubular piece 515 in a circumferential direction thereof to be shaped in an annular form.

On the other hand, a recessed portion (dispensing with being illustrated) able to be fitted into the protruding portion 519 is provided in an inner circumferential portion of an end portion vicinity region of branch pipe pieces 3A,3B (supply pipe 3). The recessed portion is configured to be able to be fitted into the protruding portion 519. On inserting the supply pipe side tubular piece 515 into the branch pipe pieces 3A and 3B (supply pipe 3), both sides are connected with each other by the protruding portion 519 being fitted into the recessed portion. The protruding portion 519 and the recessed portion make up a connection mechanism between the supply pipe side tubular piece 515 and the branch pipe pieces 3A and 3B.

A connection mechanism between the supply pipe side tubular piece 515 and the branch pipe pieces 3A and 3B (supply pipe 3) is not limited to the above and may be configured in other ways, all of which are included in the present invention.

Although in the above, the communicating tubular portion 510 and the supply pipe 3 are configured to be discrete members which can be separated from each other, limitation thereto should not be made. For example, the supply pipe 3 can be even configured to have, on its own terminal end section, a structure corresponding to the gas guiding portion side tubular piece 514. In this case, the first tubular portion 52 of the present embodiment is directly connected to the supply pipe 3. In any case, it suffices that a member which is, so as to be able to supply gas, connected to an end portion of the first tubular portion 52 of the present embodiment is provided with a structure which the gas guiding portion side tubular piece 514 has, and such is included in a scope of the present invention.

### <Swing mechanism>

A swing mechanism 10 in the present embodiment is described in reference of Fig.10, and Fig.16 to Fig.18. In the present embodiment, the breathing aid tool 5 has the swing mechanism 10. The swing mechanism 10 allows or restricts, in accordance with a magnitude of external force applied, swings of the second tubular portions 53A and 53B around a central axis 520C of the first tubular portion 52. The swing mechanism 10 allows, when having a given external force (a swing side external force) applied, swings of the second tubular portions 53A and 53B around the central axis 520C of the first tubular portion 52, whereas restricts, when having the given external force removed, the above swings of the second tubular portions 53A and 53B. In the present embodiment, the swing mechanism 10 is configured, as shown in Fig.16 and Fig.17, to allow or restrict a relative swing of the second tubular portion 53 (53A, 53B) in a circumferential direction of the communicating tubular portion 510 (the gas guiding portion side tubular piece 514), together with a relative rotation of the first tubular portion 52 with the communicating tubular portion 510 (the gas guiding portion side tubular piece 514) being an axis with respect to the communicating tubular portion 510 (the gas guiding portion side tubular piece 514). On connecting the first tubular portion 52 to the communicating tubular portion 510 (the gas guiding portion side tubular piece 514), central axes of both of them is coaxial. Hence, the swing mechanism 10 in the present embodiment swings relatively the second tubular portion 53 (53A, 53B) with a central axis of the communicating tubular portion 510 (the gas guiding portion side tubular piece 514) being an axis. This allows a relative angle of the second tubular portion 53 (53A, 53B) by making a basis out of a depth direction of the face 940 of the user 900, to be modified. In Fig.14, θ1, θ2, and θ3 are depicted as relative angles.

A swing mechanism 10 has the diameter-reduction portion 526 (the first engagement region KR1) in the overlapping region 200 and the engagement portion 517 (the second engagement region KR2) therein, as shown in Fig.10 (C). This holds likewise for a first connection position modification mechanism (a first relative position modification mechanism), a second connection position modification mechanism (a second relative position modification mechanism), and an overlapping region axial length modification mechanism. As a result, in the present embodiment, configurations of a first connection position modification mechanism (a first relative position modification mechanism), a second connection position modification mechanism (a second relative position modification mechanism), and an overlapping region axial length modification mechanism also serve as a configuration of the swing mechanism 10, and they are configured to be in common with a configuration of the swing mechanism 10. However, the present invention is not limited to the above configurations, and it suffices that the first connection position modification mechanism (a first relative position modification mechanism), the second connection position modification mechanism (a second relative position modification mechanism), and the overlapping region axial length modification mechanism are configured to partially serve as or to be partially in common with the swing mechanism 10, and it also suffices that both are configured to be discrete mechanisms which do not serve as each other and can act independently of each other (see a second embodiment described later).

As shown in Fig.10 (A), the inner diameter K1 of the opening 520 of the first tubular portion 52 (the diameter-reduction portion 526) is smaller than the maximum outer diameter K3 of the engagement portion 517 by making a basis out of the central axis of the gas guiding portion side tubular piece 514. Additionally, the diameter-reduction portion 526 is constituted by a material which can be elastically deformed. Hence, on an occasion of having inserted, as shown in Fig.10 (C), the communicating tubular portion 510 (the first communicating tubular portion 510A in Fig.10 (C): likewise below) into an internal portion of the first tubular portion 52 through the opening 520 of the diameter-reduction portion 526, the opening 520 of the diameter-reduction portion 526 is, by dint of a resilience of elastic deformation, engaged with the engagement portion 517 of the communicating tubular portion 510 from an apical portion side of the engagement portion 517, and presses the communicating tubular portion 510. Then, between the opening 520 of the diameter-reduction portion 526 and the engagement portion 517 in the communicating tubular portion 510, a friction force due to the above pressing force is generated on a contact surface therebetween. This generates engagement in a circumferential direction of the communicating tubular portion 510 (the gas guiding portion side tubular piece 514) due to the friction force, between the diameter-reduction portion 526 and the engagement portion 517. As a result, the diameter-reduction portion and the engagement portion 517 are engaged with each other, due to the above friction force, in a circumferential direction of the communicating tubular portion 510 (the gas guiding portion side tubular piece 514). Below, engagement in a circumferential direction of the communicating tubular portion 510 (the gas guiding portion side tubular piece 514) is referred to as a circumferential direction engagement in some case, and an engagement force which works on both sides of engagement counterparts due to the circumferential direction engagement is referred to as a circumferential direction engagement force in some case, as appropriate.

When in the present embodiment, an external force (hereinafter, referred to as a circumferential direction external force) is applied in a circumferential direction of the gas guiding portion side tubular piece 514 with respect to the gas guiding portion 50 (the diameter-reduction portion 526), the circumferential direction external force overcomes the circumferential direction engagement force acting between the diameter-reduction portion 526 and the engagement portion 517, and a relative rotation of the first tubular portion 52 (the diameter-reduction portion 526) with respect to the gas guiding portion side tubular piece 514 and a relative swing of the second tubular portion 53 with respect to the gas guiding portion side tubular piece 514 is allowed. Then, when the above circumferential direction external force is removed, circumferential direction engagement between the diameter-reduction portion 526 and the engagement portion 517 brings the above relative rotation of the first tubular portion 52 (the diameter-reduction portion 526) and the above relative swing of the second tubular portion 53 into a state of being restricted, and brings the gas guiding portion 50 into a state of relatively standing still with respect to the gas guiding portion side tubular piece 514. As a result, the above relative angle of the second tubular portion 53 varies. A magnitude of the above circumferential direction external force which can surmount the above circumferential direction engagement force is preferably an extent falling within a range where a human can apply it.

Circumferential direction engagement acting between the diameter-reduction portion 526 and the engagement portion 517 in the present embodiment is mainly generated by a friction force between the diameter-reduction portion 526 and the engagement portion 517. Hence, for removing circumferential direction engagement between the diameter-reduction portion 526 and the engagement portion 517, it is necessary to apply, to at least either of the first tubular portion 52 and the first communicating tubular portion 510A, the circumferential direction external force to a magnitude of allowing the circumferential direction engagement force between the diameter-reduction portion 526 and the engagement portion 517 to be surmounted. When a magnitude of the circumferential direction external force is equal to or less than that of the circumferential direction engagement force acting between the diameter-reduction portion 526 and the engagement portion 517, engagement between the diameter-reduction portion 526 and the engagement portion 517 is not removed, and the above relative rotation of the first tubular portion 52 (the diameter-reduction portion 526) and the above relative swing of the second tubular portion 53 are restricted, and therefore, the circumferential direction external force is required to have an extent enough to exceed the circumferential direction engagement force. When the circumferential direction external force has an extent enough to exceed the circumferential direction engagement force, circumferential direction engagement between the diameter-reduction portion 526 and the engagement portion 517 is removed, and the above relative rotation of the first tubular portion 52 (the diameter-reduction portion 526) and the above relative swing of the second tubular portion 53 is allowed.

Additionally, the engagement portion 517 (the second engagement region KR2) may be constitutes by a material which can be elastically deformed. As an elastic material, for example, an elastomer (e.g., thermosetting elastomer) which contains silicone rubber, fluororubber, urethane rubber, and so on is mentioned. In this case, the diameter-reduction portion 526 (the first engagement region KR1) may be constituted by a hard material which is higher in rigidity than the engagement portion 517 (the second engagement region KR2). Additionally, for increasing friction force, the diameter-reduction portion 526 (the first engagement region KR1) and the engagement portion 517 (the second engagement region KR2) may be constituted by a material high in coefficient of friction and may include a rough surface region which has a recessed/protruding portion on their mutual contact surface.

A configuration of the swing mechanism 10 is not limited to a combination between the above diameter-reduction portion 526 and engagement portion 517, and may be a modification example as below. In that modification example, the diameter-reduction portion 526 may be configured, as shown in Figs.18 (A), (C), to be provided in plurality with intervals in a circumferential direction of the first tubular portion 52 and have, each, a protruding portion 526E (the first engagement region KR1) which reduces its diameter inward in a radial direction thereof. Additionally, the engagement portion 517 may be configured, as shown in Figs.18 (B), (D), to be provided in plurality with intervals in a circumferential direction of the communicating tubular portion 510 (the gas guiding portion side tubular piece 514) and have, each, a recessed portion 510F (the second engagement region KR2) which is recessed inward in a radial direction of the communicating tubular portion 510 (the gas guiding portion side tubular piece 514). Then, at least either of the diameter-reduction portion 526 and the engagement portion 517 is preferably constituted by a material which can be elastically deformed. A configuration is made in a manner that on an occasion of having connected the communicating tubular portion 510 to the first tubular portion 52, the protruding portion 526E faces, as shown in Fig.18 (E), the recessed portion 510F mutually in a radial direction of the first tubular portion 52 to be fitted into the recessed portion 510F. By circumferential direction engagement due to the protruding portion 526E and the recessed portion 510F being fitted into each other, a relative angle θ of the second tubular portion 53 is finalized.

Then, when the circumferential direction external force is applied to at least either of the first tubular portion 52 and the communicating tubular portion 510, the protruding portion 526E is elastically deformed sufficiently to be detached from the recessed portion 510F and be relieved of engagement deriving from being fitted thereinto. As a result, the above relative rotation of the first tubular portion 52 and the above relative swing of the second tubular portion 53 with respect towith respect to the communicating tubular portion 510 are allowed, and a relative angle of the second tubular portion 53 is modified. When, thereafter, the above circumferential direction external force is removed or a magnitude of the circumferential direction external force is equal to or less than that of the circumferential direction engagement force, the above relative rotation of the first tubular portion 52 and the above relative swing of the second tubular portion 53 with respect to the communicating tubular portion 510 are restricted by circumferential direction engagement due to the protruding portion 526E and the recessed portion 510F being fitted into each other. As a result, a relative angle of the second tubular portion 53 is finalized.

In the swing mechanism 10, it is also allowable that the diameter-reduction portion 526 and the protruding portion 526E, as described above can be respectively extended to the concept of a first swing engagement region YK1 and that the engagement portion 517 and the recessed portion 510F, as such can be respectively extended to the concept of a second swing engagement region YK2. In this case, the first swing engagement region YK1 and the second swing engagement region YK2 in the swing mechanism 10 are, while being overlapped in a radial direction of the communicating tubular portion 510, engaged in a circumferential direction thereof, whereby a relative angle of the second tubular portion 53 is maintained. Then, when the circumferential direction external force is applied to either of the first tubular portion 52 and the communicating tubular portion 510, engagement between the first swing engagement region YK1 and the second swing engagement region YK2 is removed, and the above relative rotation of the first tubular portion 52 and the above relative swing of the second tubular portion 53 with respect to the communicating tubular portion 510 are allowed. In a case where engagement between the first swing engagement region YK1 and the second swing engagement region YK2 includes an element due to a mutual friction force therebetween in a circumferential direction of the gas guiding portion side tubular piece 514, that mutual engagement is removed by the circumferential direction external force becoming greater than the friction force. Additionally, in a case where engagement between the first swing engagement region YK1 and the second swing engagement region YK2 includes an element due to mutual contact therebetween in a circumferential direction of the gas guiding portion side tubular piece 514, that mutual engagement is removed, for example, by at least either of the first swing engagement region YK1 and the second swing engagement region YK2 being, when having the circumferential direction external force applied, elastically deformed to remove mutual contact in a circumferential direction of the gas guiding portion side tubular piece 514. However, limitation thereto should not be made, and all other configurations wherein mutual contact is removed in accordance with a magnitude of the circumferential direction external force are included in a scope of the present invention. Then, when the above circumferential direction external force is removed, the above relative rotation of the first tubular portion 52 with respect to the communicating tubular portion 510 and the above relative swing of the second tubular portion 53 and the communicating tubular portion 510 are restricted by circumferential direction engagement between the first swing engagement region YK1 and the second swing engagement region YK2. As a result, a relative angle of the second tubular portion 53 is finalized. The first swing engagement region YK1 and the second swing engagement region YK2 are, when being able to make circumferential direction engagement as above, included in a scope of the present invention, no matter how they are configured. Although the first swing engagement region YK1 and the first engagement region KR1 may be configured to be at least partially in common, they are not limited thereto and may be configured as non-common regions which are not in common with each other. Likewise, although the second swing engagement region YK2 and the second engagement region KR2 may be configured to be at least partially in common, they are not limited thereto and may be configured as non-common regions which are not in common with each other. Additionally, at least either of the first swing engagement region YK1 and the second swing engagement region YK2 may be constituted by a material which can be elastically deformed.

When a magnitude of the above circumferential direction external force is equal to or less than that of the circumferential direction engagement force acting between the first swing engagement region YK1 and the second swing engagement region YK2, circumferential direction engagement between the first swing engagement region YK1 and the second swing engagement region YK2 is not removed, and the above relative rotation of the first tubular portion 52 and the above relative swing of the second tubular portion 53 and the communicating tubular portion 510 are restricted, and a relative angle of the second tubular portion 53 is maintained, and therefore, the circumferential direction external force is required to have an extent enough to exceed the circumferential direction engagement force. When the circumferential direction external force has an extent enough to exceed the circumferential direction engagement force, circumferential direction engagement between the first swing engagement region YK1 and the second swing engagement region YK2 is removed, and the above relative rotation of the first tubular portion 52 and the above relative swing of the second tubular portion 53 and the communicating tubular portion 510 is allowed. As a result, it follows that a relative angle of the second tubular portion 53 can be modified.

In a case where circumferential direction engagement acting between the first swing engagement region YK1 and the second swing engagement region YK2 is due to a plurality of forces such as an elastic force and a friction force, a magnitude of the circumferential direction engagement force between the first swing engagement region YK1 and the second swing engagement region YK2 can vary within a specific range. Hence, a magnitude of the circumferential direction external force required to remove circumferential direction engagement acting between the first swing engagement region YK1 and the second swing engagement region YK2 can be invariable, and one which can vary within a specific range in accordance with the circumferential direction engagement force for each engagement state between the first swing engagement region YK1 and the second swing engagement region YK2 also suffices. This can vary in accordance with the circumferential direction engagement mode between the first swing engagement region YK1 and the second swing engagement region YK2.

Here, on an occasion of dividing the first tubular portion 52 into two regions by making a basis out of a middle in the first tubular side axial direction as shown in Figs.17 (A), (B), a region being one side of the first tubular portion 52 and being connected to the first communicating tubular portion 510A on one side is defined as a first region SA while a region being another side of the first tubular portion 52 and being connected to the second communicating tubular portion 510B on another side is defined as a second region SB. Then, the swing mechanism 10 is, in the present embodiment, divided into two to define one as a first swing mechanism piece 10A, and another as a second swing mechanism piece 10B.

The first swing mechanism piece 10A allows or restricts, in accordance with a magnitude of the circumferential direction external force applied, a relative rotation of the first region SA and a relative swing of the second tubular portion 53A, with respect to the first communicating tubular portion 510A in a circumferential direction of the first communicating tubular portion 510A. The first swing mechanism piece 10A allows, when having a given a magnitude of external force (a first swing side external force) applied, a relative rotation of the first region SA and a relative swing of the second tubular portion 53A with respect to the first communicating tubular portion 510A in a circumferential direction of the first communicating tubular portion 510A, and restricts, when having a given the magnitude of external force removed, the above relative rotation and the above relative swing. The first swing mechanism piece 10A has the first swing engagement region YK1 (e.g. the first diameter-reduction portion 526A) and the second swing engagement region YK2 (e.g. the engagement portion 517 of the first communicating tubular portion 510A). The first communicating tubular portion 510A (the first gas guiding portion side tubular piece 514A) works as a swing axis of the first region SA. In other words, the first region SA rotates relatively to the first communicating tubular portion 510A about a central axis (a central axis of the first tubular portion 52) extending in the communicating side axial direction of the first communicating tubular portion 510A. A second tubular portion 53A relatively swings with the above central axis being an axis with respect to the first communicating tubular portion 510A.

The second swing mechanism piece 10B allows or restricts, in accordance with a magnitude of the circumferential direction external force applied, a relative rotation of the second region SB and a relative swing of the second tubular portion 53B, with respect to the second communicating tubular portion 510B in a circumferential direction of the second communicating tubular portion 510B. The second swing mechanism piece 10B allows, when having a given the magnitude of external force (a second swing side external force) applied, a relative rotation of the second region SB and a relative swing of the second tubular portion 53B with respect to the second communicating tubular portion 510B in a circumferential direction of the second communicating tubular portion 510B, and restricts, when having a given the magnitude of external force removed, the above relative rotation and the above relative swing. The second swing mechanism piece 10B has tthe first swing engagement region YK1 (e.g. the second diameter-reduction portion 526B) and the second swing engagement region YK2 (e.g. the engagement portion 517 of the second communicating tubular portion 510B). The second communicating tubular portion 510B (the second gas guiding portion side tubular piece 514B) works as a swing axis of the second region SB. In other words, the second region SB rotates relatively to the second communicating tubular portion 510B about a central axis (the central axis of the first tubular portion 52) extending in the communicating side axial direction of the second communicating tubular portion 510B. A second tubular portion 53B relatively swings with the above central axis being an axis with respect to the second communicating tubular portion 510B.

Since the first swing mechanism piece 10A and the second swing mechanism piece 10B are respectively configured as above, the second tubular portion 53A and the second tubular portion 53B can be respectively made to relatively swing (action) independently of each other. Hence, the first region SA and the second region SB can, with the first swing mechanism piece 10A and the second swing mechanism piece 10B, be both made same as and made different from each other in swing angle. The first region SA and the second region SB can be both made identical to and made different from each other in swing angle. In a case where the first region SA and the second region SB are identical to each other in swing angle, a state where the first tubular portion 52 is in torsion does not come out (see Fig.17 (A)), and the second tubular portion 53A and the second tubular portion 53B are identical to each other as for relative angles which make a basis out of a depth direction of the face 940 of the user 900. On the other hand, in a case where the first region SA and the second region SB are different from each other in swing angle, a state where the first tubular portion 52 is in torsion comes out (see Fig.17 (B)), and the second tubular portion 53A and the second tubular portion 53B are different from each other as for relative angles which make a basis out of a depth direction of the face 940 of the user 900.

Here, the above circumferential direction engagement forces which respectively generate in the first swing mechanism piece 10A and the second swing mechanism piece 10B are respectively defined as a first circumferential direction engagement force and a second circumferential direction engagement force, whereas circumferential direction external forces which are respectively required to modify relative angles of the second tubular portion 53 in the first swing mechanism piece 10A and the second swing mechanism piece 10B are respectively defined as a first circumferential direction external force and a second circumferential direction external force. A magnitude of the first circumferential direction engagement force and that of the second circumferential direction engagement force can, in accordance with configurations of the first swing mechanism piece 10A and the second swing mechanism piece 10B, be set independently of each other. In accordance with that setting, the magnitude of the first circumferential direction external force and that of the second circumferential direction external force vary.

In a case where the first region SA and the second region SB of the first tubular portion 52 are formed in unity with a material which can be elastically deformed, resilience of elastic deformation due to torsion of the first tubular portion 52, a first regulation force due to engagement between the first swing engagement region YK1 and the second swing engagement region YK2 on a side of the first communicating tubular portion 510A, and a second regulation force due to engagement between the first swing engagement region YK1 and the second swing engagement region YK2 on a side of the second communicating tubular portion 510B have an effect on each other, swing angles of the first region SA and the second region SB are restricted. For example, when the relative angle of the second tubular portion 53B with respect to the second tubular portion 53A is less than a threshold value, a first regulation force and a second regulation force have an extent equal to or greater than that of resilience of elastic deformation due to torsion of the first tubular portion 52, and therefore, the first swing mechanism piece 10A and the second swing mechanism piece 10B can be made to action independently of each other, and swing angles of the first region SA and the second region SB can be freely set. when the relative angle of the second tubular portion 53B with respect to the second tubular portion 53A is equal to or greater than the threshold value, a first regulation force and a second regulation force have an extent less than that of resilience of elastic deformation due to torsion of the first tubular portion 52, and therefore, a swing angle of either of the first region SA and the second region SB is restricted by resilience of elastic deformation due to the torsion. It is also allowable that the first region SA and the second region SB are configured to be, with a central axis of each of them being a center, connected in a manner that they can relatively rotates with each other. Since in this case, the resilience of elastic deformation due to torsion of the first tubular portion 52 is not generated, the first swing mechanism piece 10A and the second swing mechanism piece 10B can be made to action independently of each other all over a circle.

A swing mechanism 10, the first swing mechanism piece 10A, and the second swing mechanism piece 10B can also be applied to an first tubular portion 52 which is not provided with a stretchable structure or a breathing aid tool which does not comprise a spacing modification mechanism.

### <Communicating tubular side hold portion>

The communicating tubular side hold portion 511 is described in reference of Fig.6 and Fig.8. The communicating tubular side hold portion 511 holds one pair of communicating tubular portions 510 in a given posture. The communicating tubular side hold portion 511 has a main body holding portion 512 and a connection portion 513 as shown in Figs. 8 (B) to (D).

The main body holding portion 512 is roughly shaped in a belt-like form and curves. As shown in Fig.6 (B), a degree of curving is approximate to a degree of curving of a middle of the face 940 in a width direction of the face 940 of the user 900 on a skin surface in the under-nose region 930 of the user 900.

The connection portion 513 is intended to connect one pair of communicating tubular portions 510 to the main body holding portion 512 in a state where the one pair of communicating tubular portions 510 is made to get into a given posture at a given position with respect to the main body holding portion 512. A give position is a position which makes line symmetry, as shown in Fig.8 (C), by making a basis out of a middle line 1000 which divides the main body holding portion 512 into two in a length direction middle of the main body holding portion 512. A given posture refers to a posture wherein each of gas guiding portion side tubular pieces 514 of one pair of communicating tubular portions 510 is positioned on sides nearer to a middle of the main body holding portion 512, wherein each of supply pipe side tubular pieces 515 of one pair of communicating tubular portions 510 is positioned on sides nearer to both ends of the main body holding portion 512, and wherein, simultaneously, each axial direction (the communicating side axial direction) of one pair of communicating tubular portions 510 is, in the above given position, in parallel in an extending direction of the main body holding portion 512. A given posture may be a posture wherein axial directions (communicating side axial directions) of one pair of communicating tubular portions 510 are inclined to an extending direction of the main body holding portion 512.

Then, when the communicating portion 51 is mounted on the user 900 by the mounting portion 6 as shown in Fig.6 (A), (B), the main body holding portion 512 gets into a posture of extending in the width direction H along a skin surface of the face 940 of the user 900 in the under-nose region 930 and curving in a middle of the width direction H of the face 940. Additionally, one pair of communicating tubular portions 510 are, by making a basis out of a middle (a center line 910C of the nose 910) in the width direction H of the face 940 of the user 900, disposed with spacing on both sides in that width direction. That is, one pair of communicating tubular portions 510 are, by interposing therebetween a middle (thecenter line 910C of the nose 910) in the width direction H of the face 940 of the user 900, disposed alongside each other in the width direction H of the face 940. On this occasion, as shown in Fig.6 (B), each of gas guiding portion side tubular pieces 514 of one pair of communicating tubular portions 510 is positioned on a side nearer to a middle (the center line 910C of the nose 910) in the width direction H of the face 940 of the user 900, and each of supply pipe side tubular pieces 515 of one pair of communicating tubular portions 510 is positioned on a side farther from a middle in the width direction H of the face 940 of the user 900, and simultaneously, one pair of communicating tubular portions 510 are, in a posture of going, in accordance as their own axial directions (communicating side axial directions) go toward a middle in the width direction H of the face 940 of the user 900, toward a part in front of the face 940 of the user 900, disposed in a front side region being a vicinity of a skin surface of the face 940.

Then, as shown in Figs.6 (A), (B), each of openings 510D of one pair of gas guiding portion side tubular pieces 514 faces forward the face 940 of the user 900 in the under-nose region 930 or a vicinity thereof, and also Each of openings 510D of one pair of gas guiding portion side tubular pieces 514 faces the other paired the gas guiding portion side tubular piece 514. That is, each of openings 510D of one pair of communicating tubular portions 510 is opposite a part obliquely in front of the user 900 toward a middle in a width direction of the face 940 of the user 900. In the present embodiment, openings 510D are disposed so as to be opposite each other in a width direction of the face 940 of the user 900.

Although in the present embodiment, the connection portion 513 ties an end portion vicinity of the main body holding portion 512 and the stopper 518B with each other as shown in Fig.8 (C), it is not limited thereto and may tie another position of the main body holding portion 512 and another position of the communicating tubular portion 510 with each other.

### <Mounting portion>

In reference of Fig.1 to Fig.8, the mounting portion 6 is described. The mounting portion 6 mounts the communicating portion 51 on the face 940 of the user 900 in order to get into a posture wherein in the under-nose region 930, the first tubular portion 52 extends in the width direction H of the face 940 of the user 900, as shown in Fig.1. In the present embodiment, the mounting portion 6 determines one pair of communicating tubular portions 510 in position to the face 940 of the user 900 in a manner that by being extended from the communicating tubular portion 510 toward the under-nose region 930 and abutting on the face 940 of the user 900 in the under-nose region 930, the first tubular portion 52 gets into a posture (see, Figs.7 (A), (B)) of extending in the width direction H of the face 940 of the user 900. Additionally, the mounting portion 6 as well extends, when mounted, in the width direction H of the face 940 of the user 900. Then, in the present embodiment, the mounting portion 6 has a breathing aid tool side hold portion 60, one pair of supply pipe hold portions 61, and a band portion 62, as shown in Fig.2 and Fig.3.

### <Breathing aid tool side hold portion>

A breathing aid tool side hold portion 60 is described in reference of Fig.1, Fig.3, Fig.6 to Fig.8. A breathing aid tool side hold portion 60 holds the breathing aid tool 5 (the communicating portion 51). Additionally, the breathing aid tool side hold portion 60 is, as shown in Fig.3, roughly shaped in a belt-like form and curves in its own middle. Additionally, as shown in Fig.6 (B), a degree of curving of the breathing aid tool side hold portion 60 is like that of the main body holding portion 512 of the communicating portion 51, and is approximate to a degree of curving on a skin surface of the user 900 in the under-nose region 930 in a width direction of the face 940 of the user 900. Then, as shown in Figs.6 (A), (B), the breathing aid tool side hold portion 60 gets, when mounted on the user 900, into a posture of extending in the width direction H of the face 940 of the user 900 while its own middle (curving part) is abutting on the face 940 of the user 900 in the under-nose region 930. On this occasion, both side parts of a middle of the breathing aid tool side hold portion 60 extend toward ears of the user 900. A breathing aid tool side hold portion 60 extends to under cheeks of the user 900, as shown in Fig.1 and Fig.6 (B).

A attachment and detachment mechanism is provided between the breathing aid tool side hold portion 60 and the breathing aid tool 5. In the present embodiment, the attachment and detachment mechanism is, as shown in Fig.3 and Figs.8 (B) to (D), constituted by one pair of recessed portions 63 (see Fig.3) which is recessed toward an internal part of its own by making a starting point out of a front side surface of the breathing aid tool side hold portion 60, and one pair of protruding portions 512A (see Figs.8 (B) to (D)) which protrudes by making a starting point out of a reverse side surface of the main body holding portion 512. One pair of recessed portions 63 is provided with spacing in a length direction of the breathing aid tool side hold portion 60. One pair of protruding portions 512A is provided with spacing same as that of one pair of recessed portions 63, on a reverse side surface of the main body holding portion 512.

A recessed portion 63 and the protruding portion 512A are configured in a manner that when the protruding portion 512A is inserted into the recessed portion 63, the protruding portion 512A is fitted into the recessed portion 63 and that the breathing aid tool side hold portion 60 and the main body holding portion 512 can be linked to each other. Additionally, the recessed portion 63 and the protruding portion 512A are configured in a manner that on pulling the main body holding portion 512 so as to separate it from the breathing aid tool side hold portion 60, the protruding portion 512A is detached from the recessed portion 63. The above attachment and detachment mechanism is one example, and another attachment and detachment mechanism also suffices.

Although in the present embodiment, the breathing aid tool side hold portion 60 and the communicating tubular side hold portion 511 is configured to be discrete members, limitation thereto should not be made and both of them may be formed in unity. In this case, the breathing aid tool side hold portion 60 and the communicating tubular side hold portion 511 are made to be one constituent portion. That constituent portion is connected through the connection portion 513 to one pair of communicating tubular portions 510.

When the breathing aid tool side hold portion 60 is mounted on the user 900 while the communicating portion 51 is held by the breathing aid tool side hold portion 60, one pair of communicating tubular portions 510 is determined in position to the face 940 of the user 900 in a manner that the first tubular portion 52 mounted on the one pair of communicating tubular portions 510 gets into a posture of extending in the width direction H of the face 940 of the user 900, as shown in Figs.7 (A), (B).

### <Supply pipe hold portion>

A supply pipe hold portion 61 is described, in reference of Fig.1 and Fig.3. One pair of supply pipe hold portion 61 hold, as shown in Fig.1, one section being in a midway of the branch pipe pieces 3A and 3B, respectively, at a given position of the breathing aid tool side hold portion 60 and in posture of going along an extending direction of the breathing aid tool side hold portion 60. In the present embodiment, the supply pipe hold portion 61 has, as shown in Fig.3, one pair of constraint pieces 64 provided in the breathing aid tool side hold portion 60 and a foundation portion 66. The foundation portion 66 is provided on a front side surface 60A being a vicinity of both sides of the breathing aid tool side hold portion 60 in a length direction thereof. The front side surface 60A of the breathing aid tool side hold portion 60 is a surface which is toward a side contrary to the user 900. On the front side surface 60A, the breathing aid tool 5 as well is provided. One pair of constraint pieces 64 constrains the supply pipe 3, respectively, to a surface (a surface which is toward a side contrary to the user 900) of the breathing aid tool side hold portion 60.

One pair of constraint pieces 64 is each shaped in a plate form, and curves. As shown in Fig.3, one pair of the constraint pieces 64 is, in a manner that outside surfaces (protruding surfaces) 64A of the constraint pieces 64 are outward in a width direction of the breathing aid tool side hold portion 60 and that, simultaneously, inside surfaces (recessed surfaces) 64B of the constraint pieces 64 are opposite each other in a width direction of the breathing aid tool side hold portion 60, provided upright from the foundation portion 66 in a vicinity of both ends in a width direction of the breathing aid tool side hold portion 60. Then, the supply pipe 3 is made to pass through a region 65 between one pair of the constraint pieces 64 (recessed surfaces 64B) and the supply pipe 3 is made to be surrounded by one pair of the constraint pieces 64 and the breathing aid tool side hold portion 60. As a result, one pair of the constraint pieces 64 and the breathing aid tool side hold portion 60 collaborate with each other to constrain the supply pipe 3 on a front side surface of the breathing aid tool side hold portion 60. When the supply pipe 3 (the branch pipe pieces 3A and 3B) is, as shown in Fig.1, connected to the communicating portion 51 (the supply pipe side tubular piece 515), a state where the supply pipe 3 is, while guided along the breathing aid tool side hold portion 60, constrained by the breathing aid tool side hold portion 60 comes out.

As above, one pair of supply pipe hold portions 61 can guide the supply pipe 3 to such a position as the supply pipe 3 does not bother the user 900.

### <Band portion>

The band portion 62 is, as shown in Fig.2, shaped in a belt-like form and has both its ends to be attached to both ends of the breathing aid tool side hold portion 60 or a vicinity thereof. A breathing aid tool side hold portion 60 is, in a state (see, Figs.6 (A), (B)) of getting into a posture of extending in a width direction of the face 940 of the user 900 in the under-nose region 930 thereof, mounted on the user 900 in a manner that the band portion 62 goes, as shown in Fig.1, around the face 940 (a head portion) of the user 900 via a right-side head portion 961, a back-of-head portion, a left-side head portion 962, of the user 900. This makes the breathing aid tool side hold portion 60 be mounted on the under-nose region 930 in the above posture.

### <Second embodiment>

A breathing aid device 1 in the second embodiment of the present invention is described in reference of Fig.19 to Fig.23. A breathing aid device 1 in the present embodiment is different from that of the first embodiment in configurations of communicating tubular portions 510 and swing mechanisms 10 which make up part of holding mechanisms. As for matters other than that, what has been described in the first embodiment can be all applied to the present embodiment. When in a breathing aid device 1 in the present embodiment, shown in Fig.19 (A), the gas guiding portion 50 and the communicating tubular portion 510 are separated from the communicating tubular side hold portion 511, a mode as shown in Fig.19 (B) comes out. When further, the gas guiding portion 50 and the communicating tubular portion 510 are separated from each other, a mode as shown in Fig.19 (C) comes out.

### <Communicating tubular portion>

The communicating tubular portion 510 in the present embodiment is described in reference of Fig.20 and Fig.21. The communicating tubular portion 510 in the present embodiment has, as shown in Fig.20 (A), (B), a first connection tubular portion 570 and a second connection tubular portion 571. As shown in Fig 21 (B),the first connection tubular portion 570 is connected the first tubular portion 52 so as to be able to guide gas. The second connection tubular portion 571 is, so as to be able to guide gas to the first connection tubular portion 570, connected, on its one end side, to the first connection tubular portion 570, and connected, on another end side, to the supply pipe 3. That is, in a breathing aid device 1 in the present embodiment, the first tubular portion 52, the first connection tubular portion 570, the second connection tubular portion 571, and the supply pipe 3 are substantially coaxially disposed in that order, and connected so as to be able to guide gas to what each of them is adjacent to.

### <First connection tubular portion>

As shown in Fig.20 (B) and Fig.21 (A), the first connection tubular portion 570 has, along the communicating side axial direction, a first section Q1 having the second engagement region KR2 (a region having the plurality of protrusion portions 590, in Fig.21) and a second section Q2 to be inserted into the second connection tubular portion 571. A flange 580 which is, on an occasion of having connected the first connection tubular portion 570 and the second connection tubular portion 571 with each other, in contact with an end portion of the second connection tubular portion 571 is provided on a boundary between the first section Q1 and the second section Q2.

Additionally, the first tubular portion 52 has, as shown in Fig.21 (A), the first engagement region KR1 (a region having a plurality of groove portions 530, in Fig.21) which extend along the first tubular side axial direction. Then, when the first connection tubular portion 570 is, as shown in Fig.21 (B), inserted into the first tubular portion 52 to make the first tubular portion 52 and the first connection tubular portion 570 be connected with each other, the overlapping region 200 is formed between the first engagement region KR1 of the first tubular portion 52 and the first section Q1 of the first connection tubular portion 570. Then, the first engagement region KR1 and the second engagement region KR2 are engaged with each other in the communicating side axial direction. Then, between the first engagement region KR1 of the first tubular portion 52 and the second engagement region KR2 of the first section Q1 of the first connection tubular portion 570, a first connection position modification mechanism (a first relative position modification mechanism), a second connection position modification mechanism (a second relative position modification mechanism), and an overlapping region axial length modification mechanism, which are described in the first embodiment, are formed. As a matter of course, one wherein all matters regarding the first engagement region KR1, the second engagement region KR2, a first connection position modification mechanism (a first relative position modification mechanism), a second connection position modification mechanism (a second relative position modification mechanism), and an overlapping region axial length modification mechanism, which are described in the first embodiment, are applied to the present embodiment is also included in a scope of the present invention. Hence, although in Figs.21 (A), (B), a configuration is made in a manner that the first engagement region KR1 has the plurality of groove portions 530 whereas the second engagement region KR2 has the plurality of protrusion portions 590, it is not limited thereto and another configuration (e.g. a configuration wherein the first engagement region KR1 has the diameter-reduction portion 526 whereas the second engagement region KR2 has a plurality of engagement portions 517) also suffices.

Additionally, although in the present embodiment, a mode wherein the first section Q1 of the first connection tubular portion 570 is inserted into the internal passageway 521 of the first tubular portion 52 and wherein the first connection tubular portion 570 is disposed in the internal passageway 521 comes out as shown in Fig.21 (A), limitation thereto should not be made, and on the contrary, a mode wherein the first tubular portion 52 is inserted into an internal passageway 570C of the first section Q1 of the first connection tubular portion 570 and wherein the first tubular portion 52 is disposed in the internal passageway 570C also suffices. As a result, the overlapping region 200 is formed between the first tubular portion 52 and the first section Q1 of the first connection tubular portion 570. When an tubular portion of a side making insertion is defined as an insertion portion and when a tubular portion of a side accepting the insertion portion is defined as an acceptance portion, it follows that either of the first tubular portion 52 and the first connection tubular portion 570 is an insertion portion whereas a rest other thereof is an acceptance portion.

Additionally, in a connection region between the first tubular portion 52 and the first connection tubular portion 570, a first relative rotation restriction mechanism which restricts a relative rotation between the first tubular portion 52 and the first connection tubular portion 570. That connection region refers to a region (the overlapping region 200) where the first tubular portion 52 and the first connection tubular portion 570 can be connected and overlapped with each other. In Fig.21 (C), a cross-sectional view which cuts the overlapping region 200 in a direction perpendicular to the communicating side axial direction is shown. Since the first engagement region KR1 and the second engagement region KR2 is engaged (in contact) with each other, it is preferable, for making the above relative rotation be restricted, that in a section where the first engagement region KR1 of the first tubular portion 52 is provided and in a section where the second engagement region KR2 of the first connection tubular portion 570 is provided, an inner circumferential side shape of a cross section of the first tubular portion 52 and an outer circumferential side shape of a cross section of the first connection tubular portion 570 are configured to be, as shown in a cross-sectional view of Fig.21 (C), in a non-genuinely circular form which is not a genuine circle. As such a non-genuinely circular form, an elliptical form, an polygonal form, and so on are mentioned as one example. It suffices that a tubular portion (e.g. the first connection tubular portion 570) corresponding to an insertion portion is in a non-genuinely circular form for its outer circumferential side shape whereas it suffices a tubular portion (e.g. the first tubular portion 52) corresponding to an acceptance portion is in a non-genuinely circular form for its inner circumferential side shape. As a result, a circumferential region where a tubular portion corresponding to an insertion portion and a tubular portion corresponding to an acceptance portion are in contact with each other is shaped in a non-genuinely circular form. When the above cross-sectional shapes of the first engagement region KR1 of the first tubular portion 52 and the second engagement region KR2 of the first connection tubular portion 570 are a non-genuinely circular form, a relative rotation of the first tubular portion 52 with respect to the first connection tubular portion 570 can be restricted. The above configuration of the first relative rotation restriction mechanism is one example and may be another configuration.

### <Second connection tubular portion>

In the present embodiment, the second connection tubular portion 571 has, for example, a supply pipe side connection tubular portion 572, and a middle connection tubular portion 573, as shown in Fig.20 (B). The supply pipe side connection tubular portion 572 is connected to the supply pipe 3, as shown in Fig.21 (B). The middle connection tubular portion 573 is, on one end side, connected to the first connection tubular portion 570 and is, on another end side, connected to the supply pipe side connection tubular portion 572. As a result, the middle connection tubular portion 573 is interposed between the first connection tubular portion 570 and the supply pipe side connection tubular portion 572. Then, the supply pipe side connection tubular portion 572 is connected so as to be restricted from rotating relatively with respect to the middle connection tubular portion 573.

When a user changes his/her posture, unreasonable load is likely to be imposed on the supply pipe 3. Then, if unreasonable load is imposed on the supply pipe 3, the supply pipe 3 threatens to be detached from the supply pipe side connection tubular portion 572 or to be damaged. Hence, the supply pipe 3 is preferably configured to be able to be connected relatively rotatably to the supply pipe side connection tubular portion 572. In this case, it is preferable that cross sections of the supply pipe 3 and the second connection tubular portion 571 on an occasion of having cut an overlapping region 300 where the supply pipe 3 and the second connection tubular portion 571 are overlapped with each other in a direction of being perpendicular to the communicating side axial direction are shaped in a genuinely circular form.

The first connection tubular portion 570 is held by being connected to the middle connection tubular portion 573 and is configured to be able to rotate relatively with respect to the communicating tubular side hold portion 511. That is, in a connection region between the first connection tubular portion 570 and the middle connection tubular portion 573, a relative rotation mechanism which allows a relative rotation between the first connection tubular portion 570 and the middle connection tubular portion 573 is provided. That connection region refers to a region (an overlapping region 310) where the first connection tubular portion 570 and the middle connection tubular portion 573 can be connected and overlapped with each other. On the other hand, the middle connection tubular portion 573 is held by the communicating tubular side hold portion 511 so as to be restricted from rotating relatively with respect to the communicating tubular side hold portion 511. That is, in a connection region between the middle connection tubular portion 573 and the communicating tubular side hold portion 511, a third relative rotation restriction mechanism which restricts a relative rotation of the middle connection tubular portion 573 with respect to the communicating tubular side hold portion 511 is provided. That connection region refers to a region where the middle connection tubular portion 573 and the communicating tubular side hold portion 511 can be connected and overlapped with each other. Specifically, the middle connection tubular portion 573 has, as shown in Fig.22 (A), a protrusion portion 573A which protrudes from its own outer circumferential surface outward in its own radial direction. Then, the communicating tubular side hold portion 511 has a relative rotation regulation surface (positioned on a paper surface deep side of the protrusion portion 573A of Fig.22 (A)) which is engaged with the protrusion portion 573A in a circumferential direction of the middle connection tubular portion 573 and regulates a relative rotation of the middle connection tubular portion 573 with respect to the communicating tubular side hold portion 511. Since the protrusion portion 573A is engaged with a relative rotation regulation surface in a circumferential direction of the middle connection tubular portion 573, the middle connection tubular portion 573 cannot rotate relatively with respect to the communicating tubular side hold portion 511. Hence, it can be considered that by the protrusion portion 573A, and a relative rotation regulation surface of the communicating tubular side hold portion 511, a third relative rotation restriction mechanism is constituted.

Additionally, although in the present embodiment, a mode wherein the second section Q2 of the first connection tubular portion 570 is inserted into an internal passageway 573B of the middle connection tubular portion 573 and wherein the first connection tubular portion 570 is disposed in the internal passageway 573B comes out as shown in Fig.22 (A), limitation thereto should not be made, and on the contrary, a mode wherein the middle connection tubular portion 573 is inserted into the internal passageway 570C of the second section Q2 of the first connection tubular portion 570 and wherein the middle connection tubular portion 573 is disposed in the internal passageway 570C also suffices. As a result, the overlapping region 310 is formed between the second section Q2 of the first connection tubular portion 570 and the middle connection tubular portion 573. When an tubular portion of a side making insertion is defined as an insertion portion and when a tubular portion of a side accepting the insertion portion is defined as an acceptance portion, it follows that either of the first connection tubular portion 570 and the middle connection tubular portion 573 is an insertion portion whereas a rest other thereof is an acceptance portion.

The second connection tubular portion 571 may be constituted by a single tubular portion. In this case, a single tubular portion which constitutes the second connection tubular portion 571 is, on one end side, connected to the supply pipe 3 and is, on another end side, connected to the first connection tubular portion 570. Additionally, the second connection tubular portion 571 may be constituted by three or more tubular portions which are connected with each other.

### <Swing mechanism>

A swing mechanism 10 is provided between the first connection tubular portion 570 and the middle connection tubular portion 573. A swing mechanism 10 has, as shown in Fig.22 (B), the first swing engagement region YK1 and the second swing engagement region YK2. The first swing engagement region YK1 is provided on the first connection tubular portion 570, as shown in Fig.22 (C). Hence, the first swing engagement region YK1 may be alternatively read as a first connection tubular side engagement region YK1. The second swing engagement region YK2 is provided on the second connection tubular portion 571 (the middle connection tubular portion 573). Hence, the second swing engagement region YK2 may be alternatively read as a second connection tubular side engagement region YK2. In the present embodiment, the first connection tubular portion 570 is an inserting side, and the first swing engagement region YK1 is provided on an outer circumferential surface of the first connection tubular portion 570. Additionally, in the present embodiment, the second connection tubular portion 571 (the middle connection tubular portion 573) is an accepting side, and the second swing engagement region YK2 is provided on an inner circumferential surface of the second connection tubular portion 571 (the middle connection tubular portion 573). The present invention is not limited to the above configuration. It is also allowable that the first connection tubular portion 570 is an accepting side, and in this case, the first swing engagement region YK1 is provided on an inner circumferential surface of the first connection tubular portion 570. Additionally, it is also allowable that the second connection tubular portion 571 (the middle connection tubular portion 573) is an inserting side, and in this case, the second swing engagement region YK2 is provided on an outer circumferential surface of the second connection tubular portion 571 (the middle connection tubular portion 573).

As shown in Fig.22 (B), the first swing engagement region YK1 and the second swing engagement region YK2 are overlapped with each other in a radial direction of the first connection tubular portion 570 (an insertion portion) or the second connection tubular portion 571 (an acceptance portion), and engaged with each other in a circumferential direction of the first connection tubular portion 570 (an insertion portion) or the second connection tubular portion 571 (an acceptance portion). A region where the first swing engagement region YK1 and the second swing engagement region YK2 are overlapped and engaged with each other is an engagement overlapping region 320. Although the first swing engagement region YK1 and the second swing engagement region YK2 are preferably engaged with each other fully around a circle in a circumferential direction of an outer circumference of an insertion portion or an acceptance portion, a mode wherein they are engaged partially around a circle also suffices. By the first swing engagement region YK1 and the second swing engagement region YK2 being engaged with each other in the above circumferential direction, a relative angle of the second tubular portion 53 is maintained.

The first swing engagement region YK1 has, for example, as shown in Fig.22 (B) and Fig.23 (A), protrusion rings 575 A, 575B which protrude in a radial direction of the first connection tubular portion 570 on an outer circumferential side of the first connection tubular portion 570 and simultaneously, extend annularly in a circumferential direction of the first connection tubular portion 570. Protrusion rings 575A, 575B are provided with spacing in the communicating side axial direction. Additionally, the first swing engagement region YK1 has a restriction protrusion portion 575C which protrudes from a protrusion ring 575A in a radial direction of the first connection tubular portion 570.

The second swing engagement region YK2 has, for example, as shown in Fig.22 (B) and Fig.23 (A), engagement portions 577A, 577B and a partial circumference groove portion 577C. Engagement portions 577A, 577B are provided in a manner of extending annularly in a circumferential direction of an inner circumferential side of the second connection tubular portion 571, and are, on an occasion where the first connection tubular portion 570 and the middle connection tubular portion 573 (the second connection tubular portion 571) are connected with each other, engaged, while orientating themselves toward protrusion rings 575 A, 575B, respectively, with the protrusion rings 575 A, 575B. Engagement portions 577A, 577B are provided with spacing in the communicating side axial direction.

The partial circumference groove portion 577C is, while recessed outward in a radial direction of the second connection tubular portion 571 at an end portion of the second connection tubular portion 571, opened to an external portion in the communicating side axial direction and extends, in a circumferential direction of an inner circumferential side of the second connection tubular portion 571, only by a partial circumference being part of a full circumference. Then, on an occasion where the first connection tubular portion 570 and the middle connection tubular portion 573 are connected with each other, the restriction protrusion portion 575C is, as shown in Fig.23 (B), disposed so as to be able to revolve within the partial circumference groove portion 577C .

When the first connection tubular portion 570 and the middle connection tubular portion 573 rotate relatively with each other, the restriction protrusion portion 575C revolves within a partial circumference groove portion 577 along the partial circumference groove portion 577. A range in which the first connection tubular portion 570 and the middle connection tubular portion 573 can rotate relatively is a range until which the restriction protrusion portion 575C revolves to get into contact with a circumferential both ends 577D, 577E of the partial circumference groove portion 577C . That is, a range in which the first connection tubular portion 570 and the middle connection tubular portion 573 can rotate relatively is a range in which the restriction protrusion portion 575C can revolve within the partial circumference groove portion 577C . As above, it can be considered that by the restriction protrusion portion 575C and the partial circumference groove portion 577C , a second relative rotation restriction mechanism which restricts, to a given relative rotation angle, a range in which the first connection tubular portion 570 and the second connection tubular portion 571 can rotate relatively with each other is constituted. A revolution angle at which the restriction protrusion portion 575C can revolve within the partial circumference groove portion 577C and a given relative rotation angle turn out to be identical. Incidentally, the above partial circumference corresponds to a length of the second connection tubular portion 571 in a circumferential direction of an inner circumferential side of the second connection tubular portion 571 corresponding to a given relative rotation angle described later. A second relative rotation restriction mechanism is not limited to the above configuration and may be configured in other ways. When the first connection tubular portion 570 is an accepting side whereas the second connection tubular portion 571 (the middle connection tubular portion 573) is an inserting side, a configuration, described above, of the first swing engagement region YK1 is a configuration of the second swing engagement region YK2, and a configuration, described above, of the second swing engagement region YK2 is a configuration of the first swing engagement region YK1.

In the present embodiment, the second swing engagement region YK2 (engagement portions 577A, 577B) presses the first swing engagement region YK1 (protrusion rings 575A, 575B), and a friction force due to such pressing is generated between the first swing engagement region YK1 and the second swing engagement region YK2. As a result, the first swing engagement region YK1 and the second swing engagement region YK2 are engaged with each other, by the friction force, in a circumferential direction of the communicating tubular portion 510. For increasing friction force, the first swing engagement region YK1 and the second swing engagement region YK2 may be constituted by a material high in coefficient of friction, and a contact surface between the first swing engagement region YK1 and the second swing engagement region YK2 may include a rough surface region which has a recessed/protruding portion.

When the circumferential direction external force is applied to the swing mechanism 10, circumferential direction engagement between the first swing engagement region YK1 and the second swing engagement region YK2 is removed, and a relative rotation between the first connection tubular portion 570 and the middle connection tubular portion 573 is allowed. As a result, a relative swing of the second tubular portion 53 is allowed. By the swing mechanism 10 of the present embodiment, the second tubular portion 53 can be, without step, made to swing relatively. The above circumferential direction external force is required to be to a magnitude of exceeding the circumferential direction engagement force. On the other hand, when the circumferential direction external force on the swing mechanism 10 is removed, a relative rotation between the first connection tubular portion 570 and the middle connection tubular portion 573 is restricted by circumferential direction engagement between the first swing engagement region YK1 and the second swing engagement region YK2. As a result, a relative swing of the second tubular portion 53 is restricted.

Additionally, in the present embodiment, a spacing modification mechanism (a connection position modification mechanism) is, in the communicating side axial direction, provided at a position farther from the second connection tubular portion 571 (the middle connection tubular portion 573) than the swing mechanism 10. Hence, the second engagement region KR2 is, in the communicating side axial direction, provided at a position farther from the second connection tubular portion 571 (the middle connection tubular portion 573) than the first swing engagement region YK1. As the present invention is not limited thereto, a spacing modification mechanism (a connection position modification mechanism) may be, in the communicating side axial direction, provided at a position nearer to the second connection tubular portion 571 (the middle connection tubular portion 573) than the swing mechanism 10.

When the first swing engagement region YK1 and the second swing engagement region YK2 can have circumferential direction engagement as above made, they may be configured (e.g. configured like the protruding portion 526E and the recessed portion 510F described in a modification example of the swing mechanism 10 of the first embodiment wherein engagement by fitting can be carried out) in other ways, and even such is included in a scope of the present invention. Then, as a matter of course, one wherein all matters regarding the first swing engagement region YK1 and the second swing engagement region YK2 of <Swing mechanism> which are described in the first embodiment, are applied to the present embodiment is also included in a scope of the present invention. When the swing mechanism 10 is configured as above, a connection position modification mechanism (a relative position modification mechanism), a first connection position modification mechanism (a first relative position modification mechanism), a second connection position modification mechanism (a second relative position modification mechanism), and an overlapping region axial length modification mechanism, and so on are constituted by the first engagement region KR1 and the second engagement region KR2, and so on, and the swing mechanism 10 is constituted by the first swing engagement region YK1 and the second swing engagement region YK2, and so on, which are regions discrete from the first engagement region KR1 and the second engagement region KR2. Then, it is preferable that a combination of the first engagement region KR1 and the second engagement region KR2, and a combination of the first swing engagement region YK1 and the second swing engagement region YK2 are arranged in order along the communicating side axial direction, and that by making a basis out of a boundary between the first section Q1 of the first connection tubular portion 570 and the second section Q2 thereof, a former one is positioned on the first section Q1 side whereas a latter one is positioned on the second section Q2 side. Since, for that reason, a connection position modification mechanism (a relative position modification mechanism) and so on have no common part mutually shared with, and turn out to be discrete mechanisms mutually independent of, the swing mechanism 10, durability of each of the mechanisms can be enhanced. Further, all configurations wherein a connection position modification mechanism (a relative position modification mechanism), a first connection position modification mechanism (a first relative position modification mechanism), a second connection position modification mechanism (a second relative position modification mechanism), and an overlapping region axial length modification mechanism, and so on are provided in separate regions wherein they are not overlapped with the swing mechanism 10 are included in a scope of the present invention.

A swing mechanism 10 in the present embodiment may, as in a case of the first embodiment, divided into the first swing mechanism piece 10A and the second swing mechanism piece 10B. Then, the swing mechanism 10, the first swing mechanism piece 10A, and the second swing mechanism piece 10B can be applied to the first tubular portion 52 in which a stretchable structure is not provided or a breathing aid tool which does not comprise a spacing modification mechanism.

### <Third embodiment>

A breathing aid device 1 in the third embodiment of the present invention is described in reference of Fig.24 to Fig.26. A breathing aid device 1 of the present embodiment has, in a midway section of the supply pipe 3, a heating portion 9 to heat a gas passing through the supply pipe 3. The heating portion 9 is, for example, a heater wire spirally wound in a midway section of the supply pipe 3. A gas which has passed through the midway section gets cool as it gets nearer to the breathing aid tool 5. On this occasion, water droplets can be formed through dew formation in an internal portion of the supply pipe 3 and the breathing aid tool 5. Then, the water droplets formed threaten to be ejected to the user 900 through the second tubular portion 53. The breathing aid device 1 in the present embodiment has a functionality of preventing the above.

A breathing aid device 1 in the present embodiment is almost identical to that in the first embodiment. What differs therebetween is a mode of the supply pipe 3 and a matter that a gas retaining portion 7, a moisture-removing portion 8, and a moisture intrusion restriction portion 12 are added. For a breathing aid device 1 in the first embodiment, the heating portion 9 is not described but may be provided.

Although in the present embodiment, the breathing aid tool 5 has the gas guiding portion 50 and the communicating portion 51, it is not limited thereto, and may have the communicating portion 51 left out and be constituted by a gas guiding portion. In this case, the supply pipe 3 is connected to one end of the first tubular portion 52 of the gas guiding portion 50 whereas the gas retaining portion 7 is connected to another end of the first tubular portion 52. Hence, in the present embodiment and thereafter, both one wherein the first tubular portion 52 and one pair of communicating tubular portions 510 are connected with each other (see, Fig.24) and one that has the one pair of communicating tubular portions 510 left out and is constituted by the first tubular portion 52 (dispensing with being illustrated) are also regarded as one tubular portion and will be referred to as a main tubular portion 54. One that has, in addition to the above mode, the gas retaining portion 7 as well included may be regarded as one tubular portion and be regarded as the main tubular portion 54. Then, the main tubular portion 54 has, in a case of any configuration, an opening on both its ends in a axial direction. Additionally, in the present embodiment, a second tubular portion will be alternatively read as a nose insertion tubular portion.

### <Supply pipe, Gas retaining portion>

A supply pipe 3 in the present embodiment does, unlike one in the first embodiment, not branch, as shown in Fig.24. Then, the supply pipe 3 in the present embodiment is connected to an opening 54A (see, Fig.25 (B)) being one end portion of the breathing aid tool 5 (the main tubular portion 54).

The gas retaining portion 7 is, as shown in Fig.24, provided on another end portion of the main tubular portion 54 and retains a gas which has passed through the main tubular portion 54. Specifically, the gas retaining portion 7 has its one end opened and another end blocked, and simultaneously, has an internal passageway 75 leading to an opening being the one end. In the present embodiment, the gas retaining portion 7 has a gas retaining pipe 71 having both ends opened and a plug portion 81A being as an exposure portion 81 described later. Then, the gas retaining portion 7 is, so as to be able to accept a gas passing through the main tubular portion 54 in an axial direction of the main tubular portion 54, connected to an opening 54B (see Fig.25 (B)) being another end of the main tubular portion 54. Additionally, the gas retaining pipe 71 has, as shown in Fig.26 (A), an opening (hereinafter, referred to as a discharge opening) 70 being a contrary side of an opening being a side of connection with the main tubular portion 54. Then, the internal passageway 75 of the gas retaining pipe 71 is opened to an external portion through a discharge opening 70.

### <Moisture-removing portion>

The moisture-removing portion 8 removes moisture in an internal portion of at least either of the supply pipe 3 and a breathing aid tool 5. The moisture-removing portion 8 can be installed in a breathing aid device 1 as a moisture-removing member. Then, the moisture-removing portion 8 in the present invention absorbs moisture in an internal portion of at least either of the supply pipe 3 and the breathing aid tool 5 to discharge it into an external portion of the supply pipe 3 and the breathing aid tool 5. The moisture-removing portion 8 in the present embodiment is constituted by a water-absorbing material having water absorbing property. The water-absorbing material may be one that lets gas pass therethrough and may be one that blocks gas. As a water-absorbing material, for example, a porous material is mentioned as one example. As a porous material, for example, a material made of woven fabrics, nonwoven fabrics, and hollow yarn, a porous water-absorbing polymer, and a porous synthetic resin such as sponges are mentioned. Although for a porous material, a plurality thereof is available as above, it is both allowable that the moisture-removing portion 8 is made of one porous material and that it is made of a plurality of porous materials. That is, it suffices that the moisture-removing portion 8 is made of at least one of the above materials. The moisture-removing portion 8 in the present embodiment has, as shown in Fig.24, an internal portion side absorption portion 80 and the exposure portion 81.

### <Internal portion side absorption portion>

The internal portion side absorption portion 80 is disposed in an internal portion of at least either of the supply pipe 3 and the breathing aid tool 5, and absorbs moisture in an internal portion of at least either of the supply pipe 3 and the breathing aid tool 5. The internal portion side absorption portion 80 is, as shown in Fig.24, constituted by a water-absorbing member 82 which extends from the supply pipe 3 through the breathing aid tool 5 to an end portion vicinity of the gas retaining portion 7. The water-absorbing member 82 is constituted by an water-absorbing material. Then, the water-absorbing member 82 leads to the exposure portion 81 and transfers absorbed moisture to the exposure portion 81. Additionally, the water-absorbing member 82 may be configured to be a belt-like sheet and may be configured to be a belt-like block having greater thickness than the belt-like sheet. Additionally, the internal portion side absorption portion 80 is, as shown in Fig.24, preferably provided on the downstream side of the heating portion 9 of the supply pipe 3.

Here, by making a basis out of a flow (see arrow marks denoted in Figs.25 (A), (B)) of a gas passing through the main tubular portion 54, as shown in Fig.25 (B), an upstream side section of the main tubular portion 54 in an axial direction as compared with a nose insertion tubular portion 53A is defined as an upstream side section T1, a downstream side section of the main tubular portion 54 in an axial direction as compared with a nose insertion tubular portion 53B as an downstream side section T3, and a section between the upstream side section T1 and the downstream side section T3 as a middle section T2. In a case of making a basis out of a flow of a gas passing through the main tubular portion 54 and an axial direction of the main tubular portion 54, a boundary between the upstream side section T1 and the middle section T2 may be a most upstream side end portion 53C of the nose insertion tubular portion 53A and may be a most upstream side end portion 529A of the communicating orifice 529. Additionally, in a case of making a basis out of a flow of a gas passing through the main tubular portion 54 and an axial direction of the main tubular portion 54, a boundary between the middle section T2 and the downstream side section T3 may be a most downstream side end portion 53D of the nose insertion tubular portion 53B and may be a most upstream side end portion 529B of the communicating orifice 529. By making a basis out of a flow of a gas passing through the main tubular portion 54, the nose insertion tubular portion 53A is positioned on a downstream side as compared with the nose insertion tubular portion 53B.

The internal portion side absorption portion 80 is preferably disposed in at least a whole section of the upstream side section T1 or a partial section thereof, as shown in Fig.25 (B). That is because moisture can, before reaching nose insertion tubular portions 53A, 53B, be absorbed by the internal portion side absorption portion 80 whereby the moisture can be prevented from being released into an external portion through the nose insertion tubular portions 53A, 53B. From this point of view, the internal portion side absorption portion 80 is preferably disposed, particularly, in a section including a terminal end (the most upstream side end portion 53C of the nose insertion tubular portion 53A or the most upstream side end portion 529B of the communicating orifice 529) of the upstream side section T1.

Additionally, the internal portion side absorption portion 80 is preferably disposed in at least a whole section of the middle section T2 or a partial section thereof, as shown in Fig.25 (B). That is because even if moisture having passed the upstream side section T1 is present, the moisture can, before reaching nose insertion tubular portions 53A, 53B, be absorbed by the internal portion side absorption portion 80 whereby the moisture can be prevented from being released into an external portion through the nose insertion tubular portions 53A, 53B. Further, the internal portion side absorption portion 80 preferably extends in a manner of striding a boundary between the upstream side section T1 and the middle section T2, as shown in Fig.25 (B).

Additionally, the internal portion side absorption portion 80 is preferably disposed in at least a whole section of the downstream side section T3 or a partial section thereof, as shown in Fig.25 (B). It is in order to remove moisture having passed the middle section T2. Further, the internal portion side absorption portion 80 preferably extends in a manner of striding a boundary between the middle section T2 and the downstream side section T3.

The internal portion side absorption portion 80 in the present embodiment is, as shown in Fig.25 (A), configured to continuously extend in a manner of spanning a whole section of the upstream side section T1 or a partial section thereof, a whole section of the middle section T2, and a whole section of the downstream side section T3.

### <Exposure portion>

As shown in Fig.24, the exposure portion 81 exposes itself, while being linked to the internal portion side absorption portion 80, to an external portion of the supply pipe 3 and the breathing aid tool 5 to discharge moisture transferred from the internal portion side absorption portion 80, into an external portion. The exposure portion 81 in the present embodiment is, as shown in Fig.24 and Figs.26 (A), (B), constituted as the plug portion 81A which blocks the discharge opening 70 of the gas retaining pipe 71. An plug portion 81A (may be referred to as a blockage portion) is configured by a water-absorbing material to be in a form where the discharge opening 70 of the gas retaining pipe 71 can be sealed (blocked). Hence, a gas leaking through the discharge opening 70 can be made minimum in amount.

Although a water-absorbing material constituting the plug portion 81A may be even one that lets gas pass therethrough, it has preferably airflow resistance above a specific extent. When the aeration resistance is above a specific extent, a gas supply amount (a gas flow amount) per unit time for the user 900 through nose insertion tubular portions 53A, 53B can be secured sufficiently. Incidentally, a water-absorbing material becomes greater in airflow resistance by absorbing moisture. When a water-absorbing material increases in moisture content amount, a gas passing through a plug portion 81A per unit time is reduced in amount, and therefore, a gas supply amount (a gas flow amount) per unit time through nose insertion tubular portions 53A, 53B increases by that degree. For controlling a gas supply amount (a gas flow amount), for example, an internal pressure measurement means (an internal pressure measurement sensor) 90 which can measure an internal pressure, and a gas supply amount measurement means (a gas flow amount measurement means) 91 which can measure a gas flow amount in an internal portion (e.g. an internal portion of the main tubular portion 54 or an internal portion of the nose insertion tubular portion 53A (the nose insertion tubular portion 53B)) of the supply pipe 3 and the breathing aid tool 5 may be, as shown in Fig.24, provided at some position (e.g. an internal portion of the main tubular portion 54 or an internal portion of the nose insertion tubular portion 53A (the nose insertion tubular portion 53B)) of an internal portion of the supply pipe 3 and the breathing aid tool 5. In this case, the gas supply source 2 may, in order to make a gas supply amount (a gas flow amount) per unit time for the user 900 to be invariable, vary a gas supply amount (a gas flow amount) on a basis of measurement results of an internal pressure measurement means (an internal pressure measurement sensor) 90 and a gas supply amount measurement means (a gas flow amount measurement means) 91. Disposition of an internal pressure measurement means (an internal pressure measurement sensor) 90 and a gas supply amount measurement means (a gas flow amount measurement means) 91 which is shown in Fig.24 is one example, and they may be disposed in other locations where a gas supply amount (a gas flow amount) per unit time through nose insertion tubular portions 53A, 53B can be grasped.

Moisture transferred from the water-absorbing member 82 is absorbed by the plug portion 81A and is retained temporarily by the plug portion 81A. Additionally, the plug portion 81A is exposed to an external portion in the discharge opening 70 of the gas retaining pipe 71. Hence, moisture contained in the plug portion 81A is dried by outside air to be gasified. Additionally, since a flow of a gas delivered through the supply pipe 3 is present on an internal portion side of the gas retaining pipe 71, the plug portion 81A is dried even on an internal portion side by the flow of a gas. As a result, moisture contained in the plug portion 81A is advanced to be gasified.

In a case where one, including even the gas retaining pipe 71, is regarded as the main tubular portion 54, a matter that the plug portion 81A blocks the discharge opening 70 of a gas retaining pipe 71 can be alternatively said that the plug portion 81A blocks the discharge opening 70 of the main tubular portion 54. Additionally, it is also allowable that the gas retaining pipe 71 is dispensed with, and that an opening of an end portion on another side of the main tubular portion 54 is configured to be the discharge opening 70. Even in this case, the plug portion 81A blocks the discharge opening 70 of the main tubular portion 54.

### <Moisture intrusion restriction portion>

The moisture intrusion restriction portion 12 is, as shown in Fig.25 (B), (C), provided at the communicating orifice 529 in an internal portion of the gas guiding portion 50 and restricts moisture from passing through the communicating orifice 529 to intrude into the internal passageway 528 of the second tubular portions 53A and 53B. Specifically, the moisture intrusion restriction portion 12 is, while provided upright from an outer margin of the communicating orifice 529 or a vicinity region thereof toward the internal passageway 521 of the first tubular portion 52, constituted by an ring-form annular wall fully circling around the communicating orifice 529. The end of the upright direction of the annular wall is not closed, and the internal passageway 521 of the first tubular portion 52 is open. It is not closed toward a tip end in a direction where an annular wall is provided upright and opened to the internal passageway 521 of the first tubular portion 52. The annular wall may be one that circles part of (partially circles) a circumference of the communicating orifice 529 in a circumferential direction of the communicating orifice 529.

Even moisture not absorbed by the internal portion side absorption portion 80 goes, together with a gas flow running the internal passageway 521 of the first tubular portion 52, toward the communicating orifice 529 in a direction slanting to the first tubular side axial direction and an axial direction (hereinafter, referred to as a second tubular side axial direction) of the second tubular portion 53A (the second tubular portion 53B), the moisture collides into an annular wall, and is, therefore, prevented from intruding into the internal passageway 528 of each of the second tubular portions 53A and 53B.

### <Modification example of third embodiment>

A modification example of the present embodiment is described in reference of Fig.27 and Fig.28. In the modification example of the present embodiment, even the exposure portion 81 is constituted by the water-absorbing member 82 formed in unity together with the internal portion side absorption portion 80 as shown in Fig.27 and Fig.28. Additionally, the gas retaining portion 7 has the discharge opening 70 on its end portion. The discharge opening 70 has an extent of making an water-absorbing member 82 pass therethrough. Then, as shown in Fig.28, an internal portion side section (an absorption side section) Q1 of the breathing aid tool 5 and the supply pipe 3 in the water-absorbing member 82 constitutes the internal portion side absorption portion 80 whereas an external portion side section (an exposure side section) Q2 of the breathing aid tool 5 and the supply pipe 3 in the water-absorbing member 82 constitutes the exposure portion 81. Incidentally, an internal portion side section (an absorption side section) Q1 refers to a section to just before the discharge opening 70, and an external portion side section (an exposure side section) Q2 includes the discharge opening 70 and refers to a section which is after an internal portion side section (an absorption side section) Q1. The water-absorbing member 82 may be configured by linking a plurality of water-absorbing members.

As for the gas retaining portion 7, an end portion of the gas retaining portion 7 where the discharge opening 70 is formed is blocked with the blockage portion 72. A blockage portion 72 is preferably configured to be able to be attached to and detached from the gas retaining portion 7. In the present modification example, the internal portion side absorption portion 80 and the exposure portion 81 are linked to each other at the discharge opening 70, as shown in Fig.28 (A). On this occasion, it is preferable that a configuration wherein no gap is provided between the discharge opening 70 and the water-absorbing member 82 is made. That is because with a gap being provided, gas leaks from that into an external portion so that power consumption of the gas supply source 2 for supplying the user 900 with a desired amount of gas can increase as compared with a case where leakage does not occur. Additionally, an exposure side section of the water-absorbing member 82 corresponding to the exposure portion 81 protrudes from the blockage portion 72 (the discharge opening 70) to an external portion and has a given length. Since the more in amount, moisture retained temporarily in the exposure portion 81 is the more preferable, it is preferable that the length is as great as possible, and however, excessive length makes the user 900 likely to get into direct contact with the exposure portion 81. Hence, an exposure side section of the water-absorbing member 82 is determined for its length in consideration of the above.

Additionally, as shown in Fig.28 (B), the discharge opening 70 may be configured to have such an extent that between itself and part of the water-absorbing member 82 corresponding to an exposure portion 81, a gap 74 is provided. Then, when the gap 74 is provided between the discharge opening 70 and the water-absorbing member 82, a gas in an internal portion of the gas retaining portion 7 leaks from the gap 74. On an occasion of having defined as a gas passage region P, a region which a gas leaking from the gap 74 passes through, the exposure portion 81 has on its surface, an overlapping region S where it is overlapped with the gas passage region P. As a result, gas is blown into the overlapping region S of the exposure portion 81, and a surrounding region thereof. Air flow due to this leakage attracts surrounding air and causes further stronger flow of air. As a result, the exposure portion 81 is dried advancingly, and moisture contained in the exposure portion 81 is gasified in a short time. Incidentally, the overlapping region S shown in Fig.27 (B) turns out to be a whole surface of the exposure portion 81.

Additionally, as shown in Fig.28 (A), a cover portion 73 to cover surroundings of a region (hereinafter, referred to as a protrusion region) 83 which protrudes from the discharge opening 70 in an exposure portion 81 may be provided in order to prevent the user 900 from getting into direct contact with the exposure portion 81. However, since moisture cannot be discharged when exposure of the exposure portion 81 is restricted, a material which constitutes the cover portion 73 preferably includes a material which lets gas pass therethrough. As a material which lets gas pass therethrough, a mesh material which has a mesh structure is preferable. When surroundings of a protrusion region 83 of the exposure portion 81 is covered with a mesh-structured material, the user 900 can be prevented from getting into direct contact with the exposure portion 81 and simultaneously, moisture which is gasified can be efficiently discharged.

What includes the gas retaining portion 7 having the discharge opening 70 may be regarded as the main tubular portion 54. In this case, the discharge opening 70 of the blockage portion 72 is regarded as the discharge opening 70 of the main tubular portion 54. Additionally, it is allowed that the gas retaining portion 7 is omitted and that an opening of another end portion of the main tubular portion 54 is configured as the discharge opening 70.

### <Fourth embodiment>

A breathing aid device 1 in the fourth embodiment of the present invention is described in reference of Fig.29 to Fig.32. A breathing aid device 1 in the present embodiment has, as in a case of the third embodiment, particularly, a functionality of preventing water droplets formed through dew formation in an internal portion of the supply pipe 3 and the breathing aid tool 5, from being ejected to the user 900 through the second tubular portion 53. The breathing aid device 1 in the present embodiment is almost identical to that in the first embodiment. What differs therebetween is whether or not the moisture-removing portion 8 is present. As shown in Fig.29, the present embodiment does, unlike a case of the third embodiment, not have the gas retaining portion 7 and comprises, as in a case of the first embodiment, the branch pipe pieces 3A and 3B.

As for the moisture-removing portion 8 in the present embodiment, the internal portion side absorption portion 80 is configured like a case of the third embodiment whereas the exposure portion 81 is configured unlike it. The exposure portion 81 in the present embodiment is, as shown in Fig.29, provided in the main tubular portion 54. Then, the exposure portion 81 makes up part of a circumferential wall 55 of the main tubular portion 54, as shown in Figs.30 (A) to (C). An outer circumferential surface 81B of the exposure portion 81 is exposed to an external portion and is in contact with the internal portion side absorption portion 80 on an inner circumferential surface 81C of the exposure portion 81, on a side contrary to the outer circumferential surface 81B.

Here, following <Third embodiment>, by making a basis out of a flow (see arrow marks denoted in Figs.30 (A) to (C)) of a gas passing through the main tubular portion 54, as shown in Fig.30 (B), a first upstream side section T4, a middle section T5, and a second upstream side section T6 are defined. Since in the present embodiment, gas is supplied from both left-right sides in an axial direction of the main tubular portion 54, sections on both left-right sides to the middle section T5 are, with a basis being made out of a flow of a gas passing through the main tubular portion 54, upstream sides both, and therefore, they are expressed as the first upstream side section T4 and the second upstream side section T6. By making a basis out of a flow of a gas supplied by the branch pipe piece 3A, the first upstream side section T4 refers to an upstream side section in an axial direction of the main tubular portion 54 as compared with the nose insertion tubular portion 53A. By making a basis out of a flow of a gas supplied by a branch pipe piece 3B, the second upstream side section T6 refers to an upstream side section in an axial direction of the main tubular portion 54 as compared with the nose insertion tubular portion 53B. The middle section T5 refers to a section between the first upstream side section T4 and the second upstream side section T6. In a case of making a basis out of a flow of a gas supplied by the branch pipe piece 3A and passing through the main tubular portion 54 and an axial direction of the main tubular portion 54, a boundary between the first upstream side section T4 and the middle section T5 may be the most upstream side end portion 53C of the nose insertion tubular portion 53A and may be the most upstream side end portion 529A of the communicating orifice 529. Additionally, in a case of making a basis out of a flow of a gas supplied by a branch pipe piece 3B and passing through the main tubular portion 54 and an axial direction of the main tubular portion 54, a boundary between the middle section T5 and the second upstream side section T6 may be a most upstream side end portion 53E of the nose insertion tubular portion 53B and may be a most upstream side end portion 529C of the communicating orifice 529.

As shown in Figs.30 (A),(B), the exposure portion 81 in the present embodiment may be provided in plurality in a state of being separated. In this case, each exposure portion 81 may be provided in one, or two, or all of the first upstream side section T4, the middle section T5 , the second upstream side section T6 in separation for each section. Additionally, the exposure portion 81 may be provided continuously in a manner of spanning adjacent sections out of the first upstream side section T4, the middle section T5, and the second upstream side section T6. That is, the exposure portion 81 may be provided in a manner of spanning the first upstream side section T4 and the middle section T5, and may be provided in a manner of spanning the middle section T5 and the second upstream side section T6, and may be provided in a manner of spanning all of the first upstream side section T4, the middle section T5, and the second upstream side section T6. Additionally, in the present embodiment, the exposure portion 81 in the first upstream side section T4 or the second upstream side section T6 is provided on the first tubular portion 52, it is not limited thereto, and may be provided so as to make up part of a circumferential wall of either of one pair of communicating tubular portions 510. Various combinations of modes of the exposure portion 81 are included in a scope of the present invention.

When the exposure portion 81 is provided in the first upstream side section T4 and the second upstream side section T6, moisture which has been absorbed into the internal portion side absorption portion 80 is absorbed into the exposure portion 81 before it reaches nose insertion tubular portions 53A, 53B, and therefore, it can be made less likely that moisture reaches the nose insertion tubular portions 53A, 53B. Additionally, in some case, the internal portion side absorption portion 80 absorbs, in the middle section T5, moisture which cannot have been captured in the first upstream side section T4 and the second upstream side section T6. Since the moisture is , in this case, released in not the first upstream side section T4 or the second upstream side section T6 but into an external portion in the middle section T5, it is preferable that the exposure portion 81 is provided in the middle section T5.

Additionally, although as shown in Figs.30 (A), (B), the internal portion side absorption portion 80 in the present embodiment is continuous in a manner of spanning each section of the first upstream side section T4, the middle section T5, and the second upstream side section T6, limitation thereto should not be made. Although as shown in Fig.30 (C), the internal portion side absorption portion 80 spans two sections adjacent to each other, it is also allowable that they are not continuous and discontinue midway. The internal portion side absorption portion 80 may, without spanning two sections adjacent to each other, be provided in any section. Additionally, the internal portion side absorption portion 80 may be provided in at least one section out of the first upstream side section T4 and the second upstream side section T6. It is preferable that particularly in the first upstream side section T4 and the second upstream side section T6, the internal portion side absorption portion 80 is provided. That is for capturing moisture before the moisture reaches nose insertion tubular portions 53A, 53B. It is preferable to provide, in order to capture moisture which cannot have been captured in the internal portion side absorption portion 80 of the first upstream side section T4 and the second upstream side section T6, the internal portion side absorption portion 80 in the middle section T5. However, even if the internal portion side absorption portion 80 is provided in any mode described above, the internal portion side absorption portion 80 is preferably provided so as to be in contact with the exposure portion 81.

Additionally, as shown in Fig.31, the exposure portion 81 is preferably provided on a contrary side region (contrary side circumferential wall: hereinafter, same) 55B positioned on a side contrary to an opposite region (an opposite circumferential wall: herein after same) 55A of the main tubular portion 54 opposite to a skin surface 930A under a nose of the face 940 of the user 900. That is for avoid water vapor through gasification from the exposure portion 81 from being released to a skin of the user 900 constantly.

Further, the exposure portion 81 is preferable to dispose in a position that is easily dried, in a contrary side region 55B. Hence, on an occasion where as shown in Fig. 31, a region which exhalation released from the nostrils 910A and 910B of the user 900 passes through is defined as an exhalation passage region V, the exposure portion 81 is preferable to provide in an overlapping region that is overlapped with the exhalation passage region V, in the contrary side region 55B. That is because the exposure portion 81 is, since having exhalation blown thereto in an overlapping region, efficiently dried so that gasification of moisture contained in the exposure portion 81 is advanced. A exposure portion 81 provided in all or part of the overlapping region is orientated to a side contrary to the face 940 of the user 900 (the skin surface 930A under a nose of the face 940 of the user 900) and simultaneously, at least a partial region of the exposure portion 81 is opposite the nostrils 910A and 910B of the user 900.

### <Modification example of Fourth embodiment>

In a modification example of the present embodiment, as shown in Fig.32, the exposure portion 81 (81A) may be provided so as to form a part of a circumferential wall of the supply pipe 3 (the branch pipe pieces 3A and 3B). A structure of the exposure portion 81 (81A) in this case is like a structure of the exposure portion 81 in the present embodiment.

Additionally, as in a case of a modification example of the third embodiment, it is allowable that in a circumferential wall of the supply pipe 3 or a circumferential wall of the main tubular portion 54, a discharge opening which lets the water-absorbing member 82 formed in unity by the internal portion side absorption portion 80 and the exposure portion 81 (81B) pass therethrough may be provided. The internal portion side absorption portion 80 and the exposure portion 81 (81B) are linked to each other at that discharge opening. It is allowable that on the exposure portion 81 (81B), a cover portion (dispensing with being illustrated) is provided as in a modification example of the third embodiment.

Although the internal portion side absorption portion 80 in the third and fourth embodiments is continuous, it may be discontinued for each section to be provided in plurality. In this case, a plurality of internal portion side absorption portion 80 are disposed discontinuously with respect to each other in, for example, the supply pipe 3, the main tubular portion 54, and the gas retaining portion 7 (the gas retaining pipe 71). Then, in each of the internal portion side absorption portion 80, the exposure portion 81 corresponding thereto is provided in at least one for each of the supply pipe 3, the main tubular portion 54, and the gas retaining portion 7. A exposure portion 81 may make up a circumferential wall of each portion of that position, and may be provided in a manner that at a discharge opening provided in each portion, the internal portion side absorption portion 80 and the exposure portion 81 are linked with each other. A exposure portion 81 of the fourth embodiment may be added to the third embodiment.

Although in the first to fourth embodiments, the second tubular portion 53 is described by making one example out of a mode configured to be a nose insertion tubular portion to be inserted into the nostrils 910A and 910B of the user 900, it is not limited thereto. The second tubular section 53 may be extended to the concept of an ejection portion that is provided midway the main tubular portion 54 and that can be directed towards the nostrils 910A, 910B or mouth 920 of the user 900 and that can eject gas passing through the main tubular section 54 to the outside. In other words, the ejection portions include, as a matter of course, one that is inserted into the nostrils 910A and 910B of the user 900, such as the second tubular portion 53, and besides includes one that is not inserted into the nostrils 910A and 910B of the user 900 and is disposed so as to make its ejection orifices be opposite the nostrils 910A and 910B in a vicinity of the nostrils 910A and 910B, and one whose ejection orifice is opposite the mouth 920 in a vicinity of the mouth 920. Hence, the breathing aid tool 5 having an ejection portion includes not only nasal cannulas, but also, for example, nasal masks, mouth masks, and so on, which are used in CPAP. Particularly, since breathing aid tools and breathing aid devices in the third and fourth embodiments include, as one of their functionalities, removal of moisture that has formed in an internal portion of the supply pipe 3 and the breathing aid tool 5 due to dew formation, they are useful for not only nasal cannulas, but also, for example, nasal masks, mouth masks, and so on, which are used in CPAP. Additionally, both only one ejection portion and a plurality thereof is allowable. Additionally, the ejection portion may be configured to be tubular as in the present embodiment, and may be configured to be a hole (ejection orifice) which penetrates a circumferential wall of the first tubular portion 52 midway on the first tubular portion 52.

### <Fifth embodiment>

A breathing aid device 1 in the fifth embodiment of the present invention is described in reference of Fig.33 to Fig.35. In the present embodiment, a breathing aid device 1 is one that comprises, in breathing aid devices 1 in the first to fourth embodiments, further an under-nose region protection portion 11. In a case where by a breathing aid device 1 in the present embodiment being, on the user 900, mounted, the breathing aid tool side hold portion 60 has been in contact with a skin of the under-nose region 930 of the user 900 over a long time, unpleasantness due to sweat can be, on the user 900, generated in a skin of the under-nose region 930. The under-nose region protection portion 11 alleviates the above unpleasantness for the user 900.

After a breathing aid device 1 in the present embodiment is mounted on the user 900, the under-nose region protection portion 11 is, as shown in Fig. 33(B), interposed between the breathing aid tool side hold portion 60 and the under-nose region 930 of the user 900 to protect a skin surface of the under-nose region 930 of the user 900 from the breathing aid tool side hold portion 60. In the present embodiment, the under-nose region protection portion 11 has, for example, a protection sheet portion 110 and an attachment mechanism 111, as shown in Figs.33(A) and (B).

When a breathing aid device 1 in the present embodiment is mounted on the user 900, the protection sheet portion 110 is, while interposed between the under-nose region 930 of the user 900 and the breathing aid tool side hold portion 60, abutting with the under-nose region 930 of the user 900 to protect the under-nose region 930. Although the protection sheet portion 110 is, for example, constituted by a belt-like sheet as shown in Figs.34 (A) and (B), it is not limited thereto and may be a sheet in another form. The protection sheet portion 110 is preferably constituted by a water-absorbing material having water absorbing property ,which can absorb sweat of the user 900 or excess moisture contained in inhalation or exhalation, and/or a breathable material through which gas can pass. Additionally, the protection sheet portion 110 is preferably constituted by a material which is deformable, flexible and/or stretchable. As a water-absorbing material and/or a breathable material, for example, a porous material is mentioned as one example. As a water-absorbing material, for example, a water-absorbing polymer is mentioned as one example. As a water-absorbing material and/or a breathable material, for example, a material made of woven fabrics, nonwoven fabrics, and hollow yarn, a porous water-absorbing polymer, and a porous synthetic resin such as sponges are mentioned. Although a material of the protection sheet portion 110 can be selected from a plurality of types as described above, the protection sheet portion 110 may be formed of one porous material or a combination of a plurality of porous materials. In other words, it suffices that the protection sheet portion 110 is formed of at least one of the above materials.

Here, a supplementary explanation is given regarding the breathing aid tool side hold portion 60. Here, a supplementary explanation is given regarding a breathing aid tool side hold portion 60. When a case where the protection sheet portion 110 is not present is assumed in Fig. 33(A), all or part of the breathing aid tool side hold portion 60 gets into direct contact with the under-nose region 930 of a user. Hereinafter, a region of direct contact between the two can be defined as a skin surface contact region 930B. The protection sheet portion 110 keeps, by being interposed in at least part of the skin surface contact region 930B, the breathing aid tool side hold portion 60 and the under-nose region 930 away from each other. As a result, direct contact between the breathing aid tool side hold portion 60 and the under-nose region 930 can be reduced in area.

The attachment mechanism 111 mounts the protection sheet portion 110 to make one flat surface portion 110A of the protection sheet portion 110 be orientated to a reverse side surface 60B of the breathing aid tool side hold portion 60 as shown in Fig. 33 (A). The reverse side surface 60B of the breathing aid tool side hold portion 60 is a surface orientated toward the user 900. The attachment mechanism 111 has, for example, one pair of engaged portions 112 (see, Fig.34 (A)) provided on the protection sheet portion 110 and one pair of engagement portions 114 (see, Fig.34 (C)) provided on the breathing aid tool side hold portion 60 and to be respectively engaged with one pair of engaged portions 112. One pair of engaged portions 112 is provided on both ends in a belt longitudinal direction of the protection sheet portion 110 or in a vicinity thereof.

As shown in Fig.34, the engaged portion 112 is, here, a member shaped in a string form or a belt-like form and has both of its own ends to be combined to the protection sheet portion 110 with spacing provided in a band width direction of the protection sheet portion 110. As a result, the engaged portion 112 has, by being configured to be in a ring form in collaboration with the protection sheet portion 110, an open hole formed. The engaged portion 112 is allowed both to be constituted by a material identical to that of the protection sheet portion 110 and to be constituted by a material different therefrom. Additionally, although a case where the engaged portion 112 forms an open hole by being in a band form (a ring form or a cylindrical form) is exemplified here, the present invention is not limited to that, and the open hole is allowed to be shaped in a bottomed cap form, and further, the open hole is allowed to be formed with a slit or a through hole, being formed on a sheet material of the protection sheet portion 110.

The engagement portion 114 is, as shown in Fig.34 (C), constituted, for example, by the front side surface 60A of an end portion vicinity of the breathing aid tool side hold portion 60 and a step surface 61A of the supply pipe hold portion 61 (the foundation portion 66). The step surface 61A has an attachment surface piece 61B, a restriction surface piece 61C and an opposite surface piece 61D. The attachment surface piece 61B is a surface that abuts on the front side surface 60A of the breathing aid tool side hold portion 60 on an occasion where the supply pipe hold portion 61 (the foundation portion 66) is attached to the breathing aid tool side hold portion 60. The restriction surface piece 61C is a surface continuous to an end portion of the attachment surface piece 61B and standing in a direction away from the front side surface 60A. the opposite surface piece 61D is a surface continuous to an end portion of the restriction surface piece 61C and extending toward an end portion of the breathing aid tool side hold portion 60 while providing a gap space between itself and the front side surface 60A of the breathing aid tool side hold portion 60. The opposite surface piece 61D preferably extends along a length direction of the breathing aid tool side hold portion 60.

More specifically, the engagement portion 114 has a recessed region 113 surrounded by the restriction surface piece 61C, the opposite surface piece 61D, and the front side surface 60A of the breathing aid tool side hold portion 60. The engaged portion 112 can be inserted into the recessed region 113 through an insertion opening 113A formed between a distal end of the opposite surface piece 61D and a distal end of the front side surface 60A which make a basis out of the restriction surface piece 61C. As a result, an end portion vicinity of the breathing aid tool side hold portion 60 is inserted into an open hole of the engaged portion 112, and the engagement portion 114 and the engaged portion 112 are caught by each other. As a result, the protection sheet portion 110 is attached to the breathing aid tool side hold portion 60.

A mode wherein the engagement portion 114 and the engaged portion 112 are engaged with each other is not limited to the above-mentioned mode. For example, as a mode of engagement between the engagement portion 114 and the engaged portion 112, a structure in which the breathing aid tool side hold portion 60 and the protection sheet portion 110 are engaged with each other in a belt longitudinal direction is preferable. For the engagement portion 114, various structures having, for example, a first restriction surface (corresponding to the restriction surface piece 61C in the present embodiment) that restricts a movement of the engaged portion 112 from both end sides or vicinity sides of the breathing aid tool side hold portion 60 toward a middle side can also be adopted. Additionally, as a mode of engagement between the engagement portion 114 and the engaged portion 112, a structure in which the breathing aid tool side hold portion 60 and the protection sheet portion 110 are engaged with each other in the user 900 direction is preferable. For the engagement portion 114, various structures having, for example, a second restriction surface (corresponding to the front side surface 60A of the breathing aid tool side hold portion 60 in the present embodiment) that prevents the protection sheet portion 110 from breaking away toward the user 900. Further, as a mode of engagement between the engagement portion 114 and the engaged portion 112, a structure in which the breathing aid tool side hold portion 60 and the protection sheet portion 110 are engaged with each other in a belt-width direction is preferable. That can be realized by a catch between an open hole of the engaged portion 112 and an end portion of the breathing aid tool side hold portion 60 to be inserted thereinto. As above, by engaging (catching) each of one pair of engaged portions 112 with each of one pair of engagement portions 114 corresponding thereto, the protection sheet portion 110 is attached to the breathing aid tool side hold portion 60.

At least either of the protection sheet portion 110 and the engaged portion 112 is preferably constituted by a material which is stretchable (elastic). On an occasion of attaching the protection sheet portion 110 in the present embodiment by the attachment mechanism 111, both ends of the protection sheet portion 110 or a vicinity thereof gets into contact with the breathing aid tool side hold portion 60, as shown in Fig.33 (A). On the other hand, at least a middle region of the flat surface portion 110A of the protection sheet portion 110 goes away from the reverse side surface 60B of the breathing aid tool side hold portion 60. That is, a distance S1 between one pair of attachment mechanisms 111 in the protection sheet portion 110 and the engaged portion 112 is, in a state of attachment, set to be smaller than a creepage distance (a distance along the reverse side surface 60B) S2 between one pair of attachment mechanisms 111 in the reverse side surface 60B. In Fig.33 (A), the distance S1 is a distance along the protection sheet portion 110 and corresponds to a shortest distance between one pair of attachment mechanisms 111, in the protection sheet portion 110 and the engaged portion 112. On this occasion, the protection sheet portion 110 is preferably in a state of being straightly stretched without being curved. In that state, the protection sheet portion 110 gets into a state where tension works thereon, and is hard to detach from the breathing aid tool side hold portion 60. In other words, a distance (dispensing with being illustrated) between one pair of engaged portions 112 in the protection sheet portion 110 in a state (a non-stretch state) where they are detached is set to be smaller than the distance S1 in a state of attachment or be equal to or smaller than the distance S1 in a state of attachment.

When a breathing aid device 1 is mounted on the user 900 as shown in Fig.33 (B), the protection sheet portion 110 and/or the engaged portion 112 is, by being pressed on the under-nose region 930 of the user 900, further stretched and is deformed so as to protrude toward the reverse side surface 60B of the breathing aid tool side hold portion 60. As a result, the protection sheet portion 110 gets into contact with the reverse side surface 60B of the breathing aid tool side hold portion 60. That is, a configuration wherein by its own stretch, deformation can be made from the distance S1 in a belt longitudinal direction of the protection sheet portion 110 and the engaged portion 112 to a creepage distance S2 of the reverse side surface 60B is preferably made.

In the midst of being used by the user 900, resilience (elastic force) of at least either of the protection sheet portion 110 and the engaged portion 112 acts. This resilience presses the protection sheet portion 110 onto the user 900. As a result, dislocation between the protection sheet portion 110 and the under-nose region 930 of the user 900 can be restrained. The protection sheet portion 110 is preferably set to be in band width equal to or more than the breathing aid tool side hold portion 60, as shown in Fig.34 (B).

A band length and band width of the protection sheet portion 110 is not limited to this if the user 900 can, on an occasion of having mounted a breathing aid device 1 in the present embodiment, diminish area at which the under-nose region 930 and the breathing aid tool side hold portion 60 are in direct contact with each other. That is, it suffices that the protection sheet portion 110 can be at least interposed in part of an area between the breathing aid tool side hold portion 60 and the under-nose region 930.

For example, on an occasion where the protection sheet portion 110 has been attached by the attachment mechanism 111, a state where as shown by a first modification example of the fifth embodiment in Fig.35 (A), tension does not work on the protection sheet portion 110 also suffices. Even in this case, it is preferable that the protection sheet portion 110 and/or the engaged portion 112 is, when a breathing aid device 1 is mounted on the user 900, stretched as shown in Fig.33 (B), and that a state where tension is applied to the protection sheet portion 110 comes out. That is, as for the protection sheet portion 110, even in a case where tension does not work in an initial attachment state, it suffices that at any timing from a time when the protection sheet portion 110 gets into contact with the reverse side surface 60B of the breathing aid tool side hold portion 60 to a time when it is completely mounted, tension is applied.

Additionally, although in the fifth embodiment, a case of fixing the protection sheet portion 110 only by the attachment mechanism 111 disposed on both ends of the breathing aid tool side hold portion 60 is exemplified, the present invention is not limited thereto. It is preferable to additionally provide, on a middle side in a longitudinal direction of the reverse side surface 60B of the breathing aid tool side hold portion 60, a fixation portion 111A which fixes the protection sheet portion 110 to the reverse side surface 60B of the breathing aid tool side hold portion 60, as shown by a second modification example of the fifth embodiment in Fig.35 (B). As a result, since an initial time where the protection sheet portion 110 is attached to the breathing aid tool side hold portion 60 by the attachment mechanism 111 and the fixation portion 111A, the protection sheet portion 110 curves along the reverse side surface 60B of the breathing aid tool side hold portion 60.

The protection sheet portion 110 can be applied to another medical tool which can abut on any region of the face 940 of the user 900. Although in the embodiments 1 to 4, the mounting portion 6 (the breathing aid tool side hold portion 60) corresponds to the present medical tool, the present medical tool is not limited thereto, and all medical tools which can abut on any region of the face 940 of the user 900 are included in the present medical tool. Even in this case, the protection sheet portion 110 is, when the present medical tool is mounted on the user 900, interposed between the present medical tool and a region of the face 940 of the user 900, corresponding to the present medical tool, and simultaneously, abuts on the region of the face 940 of the user 900, corresponding to the present medical tool. Then, on an occasion where an explanation in the present embodiment is applied to the present medical tool, it suffices that the under-nose region 930 of the user 900 is replaced by a region of the face 940 of the user 900 which the present medical tool can abut on and that a breathing aid tool side hold portion 60 is replaced by the present medical tool. Additionally, the present invention can be, by its scope being further extended, applied to tools other than medical ones.

A breathing aid tool, a breathing aid device, and a moisture-removing member, according to the present invention are not limited to embodiments described above, and can, as a matter of course, have various modifications added within a scope not deviating from purposes of the present invention. Then, all combinations by combining, as appropriate, respective configurational elements of first to fifth embodiments can be included naturally in a breathing aid tool, a breathing aid device, and a moisture-removing member, according to the present invention.

### [Reference Signs List]

1 Breathing aid device
2 Gas supply source
3 Supply pipe
3A, 3B Branch pipe piece
4 Humidifier
5 Breathing aid tool
6 Mounting portion
7 Gas retaining portion
8 Moisture-removing portion
9 Heating portion
10 Swing mechanism
10A First swing mechanism piece
10B Second swing mechanism piece
11 Under-nose region protecting portion
16 Connection portion
50 Gas guiding portion
51 Communicating portion
52 First tubular portion
53, 53A, 53B Second tubular portion (Nose insertion tubular portion)
54 Main tubular portion
55 Circumferential wall
55A Opposite region
55B Contrary side region
60 Breathing aid tool side hold portion
61 Supply pipe hold portion
62 Band portion
63 Recessed portion
64 Constraint piece
64A Protruding surface
64B Recessed surface
65 Region
70 Discharge opening
71 Gas retaining pipe
72 Blockage portion
73 Cover portion
74 Gap
75 Internal passageway
80 Internal portion side absorption portion
82 Water absorption member
81 Exposure portion
81A Plug portion
81B Outer wall surface
81C Inner wall surface
110 Protection sheet portion
111 Attachment mechanism
170 Trough region
510 Communicating tubular portion
511 Communicating tubular side hold portion
512 Main body holding portion
512A Protruding portion
513 Connection portion
514 Gas guiding portion side tubular piece
515 Supply pipe side tubular piece
516 Connection structure
517, 517A, 517B, 517C, 517D Engagement portion
518A, 518B Stopper
520 Opening
521 Internal passageway
522 First tubular piece
523 Stretchable structure piece
524 Second tubular piece
525 Pipe main body portion
526 Diameter-reduction portion
526A Innermost surface
526B Internal portion side vicinity region
526C External portion side vicinity region
527 Outlet opening
528 Internal passageway
529 Communicating orifice
900 User
910 Nose
910A, 910B Nostril
920 Mouth
930 Under-nose region
940 Face
V Exhalation passage region
KR1 First engagement region (First axial engagement region)
KR2 Second engagement region (Second axial engagement region)
YK1 First swing engagement region
YK2 Second swing engagement region
T1 Middle section
T2 End portion section

## Claims

1. A breathing aid tool to guide a gas supplied by a supply pipe to nostrils of a user, the breathing aid tool comprising:
a first tubular portion which conducts a gas supplied by the supply pipe, which is disposed in a posture of extending in a width direction of a face of the user in an under-nose region between a nose of the user and a mouth thereof, and which has a stretchable structure of being able to extend and retract in its own axial direction (hereinafter referred to as a first tubular side axial direction); and
one pair of second tubular portions which is configured to be, while branching from the first tubular portion, able to be inserted into the nostril of the user and is configured to be able to eject, from its own outlet opening, a gas passing through the first tubular portion,wherein
spacing of one pair of the second tubular portions can be varied by extending or retracting the first tubular portion in the first tubular side axial direction.

2. A breathing aid tool according to claim 1, comprising:
a spacing modification mechanism which is, so as to be able to guide a gas supplied by the supply pipe to the first tubular portion, connected to the first tubular portion, and which allows or restricts, by allowing or restricting extension and retraction of the first tubular portion in accordance with a magnitude of external force applied, a modification of spacing of one pair of the second tubular portions in the first tubular side axial direction.

3. A breathing aid tool according to claim 2, wherein the spacing modification mechanism includes
one pair of communicating tubular portions which is, so as to be able to guide a gas supplied by the supply pipe to the first tubular portion, connected to each of both ends of the first tubular portion; and
one pair of connection position modification mechanisms which is configured to be between the first tubular portion and one pair of the communicating tubular portions, and which allows or restricts a modification of a relative connection position (hereinafter, referred to as a relative connection position) between the first tubular portion and the communicating tubular portion in accordance with a magnitude of external force applied,wherein
when the relative connection position between the first tubular portion and the communicating tubular portion is modified by one pair of the connection position modification mechanisms, the stretchable structure extends and retracts in accordance with a modification of this relative connection position while spacing of one pair of the second tubular portions is modified.

4. A breathing aid tool according to claim 3, wherein the first tubular portion and the communicating tubular portion are, when the relative connection position between the first tubular portion and the communicating tubular portion is modified by the connection position modification mechanism, connected with each other so as to modify a length (hereinafter, referred to as an axial length) in an axial direction (hereinafter, referred to as a communicating side axial direction) of the communicating tubular portion of an overlapping region where the first tubular portion is overlapped with the communicating tubular portion.

5. A breathing aid tool according to claim 3, wherein the connection position modification mechanism includes
one pair of first axial engagement regions which is provided, by making a basis out of a middle of the first tubular portion in the first tubular side axial direction, on both sides of the first tubular portion; and
a second axial engagement region which is provided on each of one pair of the communicating tubular portions and is, when each of one pair of the communicating tubular portions is connected to the first tubular portion, overlapped with the first axial engagement regions to be engaged with the first axial engagement regions in an axial direction (hereinafter, referred to as a communicating side axial direction) of the communicating tubular portion, wherein
the first axial engagement regions and the second axial engagement region are configured in a manner that with the relative connection position being modified, an engagement overlapping region where the first axial engagement regions and the second axial engagement region are overlapped with each other to be engaged varies in a given range in the communicating side axial direction,
by applying external force, a modification of a range of the engagement overlapping region is allowed by engagement between the first axial engagement regions and the second axial engagement region being removed so that a relative movement between the first axial engagement regions and the second axial engagement region is allowed ;
on removing external force, a relative movement between the first axial engagement regions and the second axial engagement region is restricted by engagement between the first axial engagement regions and the second axial engagement region so that a modification of a range of the engagement overlapping region is restricted.

6. A breathing aid tool according to claim 5, wherein at least either of the first axial engagement regions and the second axial engagement region is constituted by an elastic deformation material, which can be elastically deformed,
by applying external force, at least either of the first axial engagement regions and the second axial engagement region which are constituted by an elastic deformation material is elastically deformed so that engagement between the first axial engagement regions and the second axial engagement region is removed.

7. A breathing aid tool according to claim 5 or 6, wherein either of the first axial engagement regions and the second axial engagement region has at least one protrusion portion which protrudes, in a state of being mutually engaged, toward a rest other of the first axial engagement regions and the second axial engagement region,
the rest other of the first axial engagement regions and the second axial engagement region has a plurality of engagement portions which is provided in plurality with intervals in the first tubular side axial direction or in the communicating side axial direction and is configured to be, while respectively facing the protrusion portion in a state of being engaged with each other, able to be engaged with the protrusion portion; and
the protrusion portion and the engagement portion are configured to be engaged in the communicating side axial direction and restrict relative movement between the first axial engagement regions and the second axial engagement region.

8. A breathing aid tool according to claim 2, which comprises a swing mechanism which allows or restricts a swing of the second tubular portion around a central axis of the first tubular portion in accordance with a magnitude of external force applied.

9. A breathing aid tool according to claim 8, wherein the spacing modification mechanism has a communicating tubular portion which is, so as to be able to guide a gas supplied by the supply pipe to the first tubular portion, connected to an end portion of the first tubular portion,
the swing mechanism is configured to allow or restrict, in accordance with a magnitude of external force applied, a relative swing of the second tubular portion with respect to at least part of the communicating tubular portion in a circumferential direction of the communicating tubular portion together with a relative rotation of the first tubular portion with at least part of the communicating tubular portion being an axis with respect to at least part of the communicating tubular portion.

10. A breathing aid tool according to claim 9, wherein the swing mechanism includes
a first swing engagement region which is provided on the first tubular portion; and
a second swing engagement region which is provided on the communicating tubular portion and is, when the communicating tubular portion is connected to the first tubular portion, overlapped with the first swing engagement region to be engaged with the first swing engagement region in a circumferential direction of the communicating tubular portion, wherein
by applying external force, engagement between the first swing engagement region and the second swing engagement region is removed so that the relative swing of the second tubular portion together with the relative rotation of the first tubular portion is allowed,
on removing external force, the relative swing of the second tubular portion together with the relative rotation of the first tubular portion is restricted by engagement between the first swing engagement region and the second swing engagement region.

11. A breathing aid tool according to claim 10, wherein the spacing modification mechanism has a connection position modification mechanism which is formed between the first tubular portion and one pair of the communicating tubular portions, and which allows or restricts a modification of a relative connection position (hereinafter, referred to as a relative connection position) between the first tubular portion and the communicating tubular portion in accordance with a magnitude of external force applied,
when the relative connection position between the first tubular portion and the communicating tubular portion is modified by the connection position modification mechanism, the stretchable structure extends and retracts in accordance with a modification of this relative connection position while spacing of one pair of the second tubular portions is modified,
the connection position modification mechanism includes
one pair of first axial engagement regions which is provided, by making a basis out of a middle of the first tubular portion in the first tubular side axial direction, on both sides of the first tubular portion; and
a second axial engagement region which is provided on each of one pair of the communicating tubular portions and is, when each of one pair of the communicating tubular portions is connected to the first tubular portion, overlapped with the first axial engagement regions to be engaged with the first axial engagement regions in an axial direction (hereinafter, referred to as a communicating side axial direction) of the communicating tubular portion, wherein
the first axial engagement regions and the second axial engagement region are configured in a manner that with the relative connection position being modified, an engagement overlapping region where the first axial engagement regions and the second axial engagement region are overlapped with each other to be engaged varies in a given range in the communicating side axial direction,
the first axial engagement regions and the first swing engagement region are at least partially in common with each other while the second axial engagement region and the second swing engagement region are at least partially in common with each other.

12. A breathing aid tool according to claim 8, wherein the spacing modification mechanism has one pair of communicating tubular portions which is, so as to be able to guide a gas supplied by the supply pipe to the first tubular portion, connected to each of both ends of the first tubular portion,
on an occasion of dividing the first tubular portion into two regions by making a basis out of a middle in the first tubular side axial direction to define as a first region, a region being one side of the first tubular portion and being connected to the communicating tubular portion (hereinafter, referred to as a first communicating tubular portion) on one side while to define as a second region, a region being another side of the first tubular portion and being connected to the communicating tubular portion (hereinafter, referred to as a second communicating tubular portion) on another side, the swing mechanism includes
a first swing mechanism piece which allows or restricts, in accordance with a magnitude of external force applied, a relative swing of the second tubular portion of one side in a circumferential direction of the first communicating tubular portion together with a relative rotation of the first region with at least part of the first communicating tubular portion being an axis with respect to at least part of the first communicating tubular portion; and
a second swing mechanism piece which allows or restricts, in accordance with a magnitude of external force applied, a relative swing of the second tubular portion of another side in a circumferential direction of the second communicating tubular portion together with a relative rotation of the second region with at least part of the second communicating tubular portion being an axis with respect to at least part of the second communicating tubular portion.

13. A breathing aid tool according to claim 12, wherein the first swing mechanism piece and the second swing mechanism piece operate independently of each other.

14. A breathing aid tool according to claim 12, wherein the first swing mechanism piece and the second swing mechanism piece operate independently of each other when a relative angle of the second tubular portion of another side by making a basis out of the second tubular portion of one side is less than a threshold value, and when this relative angle is a threshold value or greater, a relative swing is restricted by dint of a resilience due to a twist of the first tubular portion.

15. A breathing aid tool according to claim 1, comprising:
a communicating tubular portion which is, so as to be able to guide a gas supplied by the supply pipe to the first tubular portion, connected to an end portion of the first tubular portion; and
a swing mechanism which allows or restricts, in accordance with a magnitude of external force applied, a swing of the second tubular portion in a circumferential direction of the communicating tubular portion,
wherein the communicating tubular portion includes
a first connection tubular portion which is connected to the first tubular portion;and
a second connection tubular portion whose own one end side is connected to the first connection tubular portion and whose own one end side is connected to the supply pipe,
wherein the swing mechanism includes
a relative rotation restriction mechanism which restricts a relative rotation between the first tubular portion and the first connection tubular portion; and
a relative rotation mechanism which allows or restricts, in accordance with a magnitude of external force applied, a relative rotation between the first connection tubular portion and the second connection tubular portion, wherein
with respect to the second connection tubular portion, the first tubular portion together with the first connection tubular portion makes a relative rotation, whereby the second tubular portion swings in a circumferential direction of the communicating tubular portion.

16. A breathing aid tool according to claim 15, wherein the relative rotation restriction mechanism is configured to be in a connection region between the first tubular portion and the first connection tubular portion,
in the connection region, the first tubular portion and the first connection tubular portion are in contact while overlapped with each other, while in a cross section of the first connection tubular portion and the first tubular portion being cut in a direction perpendicular to an axial direction (hereinafter, referred to as a communicating side axial direction) of the communicating tubular portion, a region where the first tubular portion and the first connection tubular portion are in contact with each other is shaped in a non-genuinely circular form.

17. A breathing aid tool according to claim 15 or 16, wherein the relative rotation mechanism includes
a first connection tubular side engagement region which is provided on an inner circumferential surface or outer circumferential surface of the first connection tubular portion; and
a second connection tubular side engagement region which is provided in the second connection tubular portion and is, on an occasion of the first connection tubular portion and the second connection tubular portion being connected, engaged with the first connection tubular side engagement region while facing the first connection tubular side engagement region, wherein
by applying external force, engagement between the first connection tubular side engagement region and the second connection tubular side engagement region is removed so that a relative rotation between the first connection tubular portion and the second connection tubular portion is allowed,
on removing external force, a relative rotation between the first connection tubular portion and the second connection tubular portion is restricted by engagement between the first connection tubular side engagement region and the second connection tubular side engagement region.

18. A breathing aid tool according to claim 15, wherein the second connection tubular portion includes
a middle connection tubular portion whose own one end side is connected to the first connection tubular portion; and
a supply pipe side connection tubular portion whose own one end side is connected to another end side of the middle connection tubular portion and whose own another end side is connected relatively rotatably to the supply pipe.

19. A breathing aid tool according to claim 15, comprising:
a second relative rotation restriction mechanism which restricts a range of a relative rotation between the first connection tubular portion and the second connection tubular portion to a given relative rotation angle,
wherein the second relative rotation restriction mechanism includes
a partial circumference groove portion which is provided on either of the first connection tubular portion and the second connection tubular portion, and which extends, while recessed in a radial direction of the communicating tubular portion, only by a partial circumference corresponding to the given relative rotation angle in a circumferential direction of the communicating tubular portion, and
a restriction protrusion portion which is provided on a rest other of the first connection tubular portion and the second connection tubular portion, which protrudes in a radial direction of the communicating tubular portion, and which is provided so as to be, when the first connection tubular portion and the second connection tubular portion make a relative rotation, able to revolve in a circumferential direction of the communicating tubular portion along the partial circumference groove portion within the partial circumference groove portions, wherein
a revolution angle at which the restriction protrusion portion can revolve within the partial circumference groove portion is identical with the given relative rotation angle at which the first connection tubular portion and the second connection tubular portion can make a relative rotation.

20. A breathing aid tool according to claim 17, comprising:
a connection position modification mechanism which is configured to be between the first tubular portion and the first connection tubular portion, and allows or restricts, in accordance with a magnitude of external force applied, a modification of a relative connection position (hereinafter, referred to as a relative connection position) between the first tubular portion and the first connection tubular portion,
wherein when the relative connection position between the first tubular portion and the first connection tubular portion is modified by one pair of the connection position modification mechanism, the stretchable structure extends and retracts in accordance with a modification of this relative connection position while spacing of one pair of the second tubular portions is modified,
the connection position modification mechanism includes
a first axial engagement region which is provided on the first tubular portion; and
a second axial engagement region which is provided on the first connection tubular portion and is, when the first connection tubular portion is connected with the first tubular portion, overlapped with the first axial engagement region to be engaged with the first axial engagement region in an axial direction (hereinafter, referred to as a communicating side axial direction) of the communicating tubular portion, wherein
the second axial engagement region is provided on a position distal from the second connection tubular portion in the communicating side axial direction as compared with the first connection tubular side engagement region.

21. A breathing aid tool according to claim 3, comprising:
a mounting portion to mount one pair of the communicating tubular portions on a face of the user in a manner that on the under-nose region, the first tubular portion is in a posture of extending in the width direction of a face of the user.

22. A breathing aid tool according to claim 21, wherein the mounting portion is extended from the communicating tubular portion toward the under-nose region, and the communicating tubular portion is, by abutting on a face of the user in the under-nose region, determined in position to a face of the user in a manner that the first tubular portion is in a posture of extending in the width direction of a face of the user.

23. A breathing aid tool according to claim 1, comprising:
a moisture-removing portion which is constituted by a water-absorbing material having water absorbing property and which absorbs moisture in an internal portion of the supply pipe and the breathing aid tool to discharge it into an external portion of the supply pipe and the breathing aid tool,
wherein the moisture-removing portion includes
an internal portion side absorption portion which is disposed in an internal portion of at least either of the supply pipe and the breathing aid tool to absorb moisture in an internal portion of at least either of the supply pipe and the breathing aid tool; and
an exposure portion which exposes itself, while being linked to the internal portion side absorption portion, to an external portion of the supply pipe and the breathing aid tool to discharge moisture transferred from the internal portion side absorption portion, into an external portion.

24. A breathing aid tool according to claim 21, comprising:
while interposed between the mounting portion and the under-nose region, a protection sheet portion to protect the under-nose region by abutting on the under-nose region.

25. A breathing aid tool according to claim 24, wherein onto the protection sheet portion, tension is applied in the width direction.

26. A breathing aid tool to guide a gas supplied by a gas-conducting supply pipe to nostrils of a user, the breathing aid tool comprising:
a first tubular portion which conducts a gas supplied by the supply pipe, and which is disposed in a posture of extending in a width direction of a face of the user in an under-nose region between a nose of the user and a mouth thereof, and
one pair of second tubular portions which is configured to be, while branching from the first tubular portion, able to be inserted into the nostril of the user and is configured to be able to eject, from its own outlet opening, a gas passing through the first tubular portion
a communicating tubular portion which is, so as to be able to guide a gas supplied by the supply pipe, connected to an end portion of the first tubular portion; and
a swing mechanism which allows or restricts, in accordance with a magnitude of external force applied, a swing of the second tubular portion in a circumferential direction of the communicating tubular portion,
wherein the communicating tubular portion includes
a first connection tubular portion which is connected to the first tubular portion, and
a second connection tubular portion whose own one end side is connected to the first connection tubular portion and whose own one end side is connected to the supply pipe,
wherein the swing mechanism includes
a relative rotation restriction mechanism which restricts a relative rotation between the first tubular portion and the first connection tubular portion, and
a relative rotation mechanism which allows or restricts, in accordance with a magnitude of external force applied, a relative rotation between the first connection tubular portion and the second connection tubular portion,
wherein with respect to the second connection tubular portion, the first tubular portion together with the first connection tubular portion makes a relative rotation, whereby the second tubular portion swings in a circumferential direction of the communicating tubular portion.

27. A breathing aid device comprising:
a supply pipe for supplying a user with gas; and
a breathing aid tool to guide, to nostrils of the user, a gas supplied through the supply pipe,
wherein the breathing aid tool includes
a first tubular portion which conducts a gas supplied by the supply pipe, which is disposed in a posture of extending in a width direction of a face of the user in an under-nose region between a nose of the user and a mouth thereof, and which has a stretchable structure of being able to extend and retract in its own axial direction (hereinafter referred to as a first tubular side axial direction); and
one pair of second tubular portions which is configured to be, while branching from the first tubular portion, able to be inserted into the nostril of the user and is configured to be able to eject, from its own outlet opening, a gas passing through the first tubular portion,
wherein it is configured in a manner that by extending and retracting the first tubular portion in the first tubular side axial direction, spacing of one pair of the second tubular portions can be varied.

28. A breathing aid device comprising:
a supply pipe for supplying a user with gas,
a breathing aid tool to guide a gas supplied through the supply pipe to nostrils of the user or a mouth thereof; and
a moisture-removing portion which is constituted by a water-absorbing material having water absorbing property and which absorbs moisture in an internal portion of the supply pipe and the breathing aid tool to discharge it into an external portion of the supply pipe and the breathing aid tool,
wherein the moisture-removing portion includes
an internal portion side absorption portion which is disposed in an internal portion of at least either of the supply pipe and the breathing aid tool to absorb moisture in an internal portion of at least either of the supply pipe and the breathing aid tool; and
an exposure portion which exposes itself, while being linked to the internal portion side absorption portion, to an external portion of the supply pipe and the breathing aid tool to discharge moisture transferred from the internal portion side absorption portion, into an external portion.

29. A breathing aid device according to claim 28, wherein a discharge opening for discharging moisture is formed on at least either of the supply pipe and the breathing aid tool,
the exposure portion exposes itself to an external portion via the discharge opening.

30. A breathing aid device according to claim 29, wherein the breathing aid tool includes
a main tubular portion which is disposed in a manner of extending in a width direction of a face of the user and to which the supply pipe is connected on one end portion of this width direction; and
an ejection portion which has an ejection orifice able to be opposite the nostrils or mouth of the user while able to eject into an external portion, a gas passing through the main tubular portion, and is provided midway on the main tubular portion, on another end portion side of the main tubular portion, the discharge opening is formed,
wherein the internal portion side absorption portion extends from an internal portion of at least either of the supply pipe and the main tubular portion to the discharge opening.

31. A breathing aid device according to claim 30, wherein the exposure portion is constituted by a plug portion which blocks the discharge opening,
the plug portion temporarily holds moisture transferred from the internal portion side absorption portion and discharges it into an external portion after vaporizing, by drying, it.

32. A breathing aid device according to claim 30, wherein the breathing aid tool is provided on another end portion of the main tubular portion and has a gas retaining portion which retains a gas having passed the main tubular portion.

33. A breathing aid device according to claim 32, wherein the gas retaining portion has a blockage portion which blocks the end portion of the gas retaining portion and has the discharge opening,
the blockage portion can be freely attached and detached.

34. A breathing aid device according to claim 29, wherein a gap is provided between the discharge opening and the exposure portion,
on an occasion of having defined as a gas passage region, a region through which a gas leaking from the gap passes, the exposure portion has on its surface, an overlapping region which overlaps with the gas passage region.

35. A breathing aid device according to claim 29, wherein the exposure portion is made so as to protrude into an external portion from the discharge opening,
comprises a cover portion which is constituted by a material through which gas can pass, and which covers surroundings of a region protruding from the discharge opening in the exposure region.

36. A breathing aid device according to claim 30, wherein the internal portion side absorption portion is, by making a basis out of a flow of a gas passing through the main tubular portion, disposed in an upstream side section of the main tubular portion as compared with the ejection portion.

37. A breathing aid device according to claim 30, wherein the ejection portion branches into two from the main tubular portion,
the internal portion side absorption portion is, by making a basis out of a flow of a gas passing through the main tubular portion, disposed in a manner of spanning an upstream side section of the main tubular portion as compared with the ejection portion and a section between two of the ejection portions.

38. A breathing aid device according to claim 28, wherein the breathing aid tool includes
a main tubular portion which is disposed in a manner of extending in a width direction of a face of the user and to which the supply pipe is connected on one end portion of this width direction; and
an ejection portion which has an ejection orifice able to be opposite the nostrils or mouth of the user while able to eject into an external portion, a gas passing through the main tubular portion, and is provided midway on the main tubular portion,
wherein the exposure portion constitutes part of a circumferential wall of the main tubular portion.

39. A breathing aid device according to claim 38,
wherein the exposure portion constitutes, by making a basis out of a flow of a gas passing through the main tubular portion, part of a circumferential wall of the main tubular portion in an upstream side section as compared with the ejection portion.

40. A breathing aid device according to claim 38, wherein the ejection portion branches into two from the main tubular portion,
the exposure portion constitutes, by making a basis out of a flow of a gas passing through the main tubular portion, part of a circumferential wall of the main tubular portion in a manner of spanning an upstream side section as compared with the ejection portion and a section between two of the ejection portions.

41. A breathing aid device according to claim 38, wherein the exposure portion is, on an occasion where the ejection orifices are disposed in a manner of being opposite the nostrils of the user, provided in a contrary side region of the main tubular portion, which is located on a side contrary to an opposite region of the main tubular portion, which is opposite a skin surface under a nose of the user.

42. A breathing aid device according to claim 41, wherein the exposure portion is, on an occasion of having defined as an exhalation passage region, a region through which an exhalation emitted from the nostrils of the user passes, provided in an overlapping region which overlaps with the exhalation passage region, out of the contrary side region.

43. A moisture-removing member which, in a breathing aid device comprising a supply pipe for supplying a user with gas and a breathing aid tool to guide, to the user, a gas supplied through this supply pipe, absorbs moisture in an internal portion of the supply pipe and the breathing aid tool to discharge it into an external portion of the supply pipe and the breathing aid tool, moisture-removing member comprising ;
an internal portion side absorption portion which is constituted by a water-absorbing material having water absorbing property and which is disposed in an internal portion of at least either of the supply pipe and the breathing aid tool to absorb moisture in an internal portion of at least either of the supply pipe and the breathing aid tool, and
an exposure portion which is constituted by the water-absorbing material and which exposes itself, while being linked to the internal portion side absorption portion, to an external portion of the supply pipe and the breathing aid tool to discharge moisture transferred from the internal portion side absorption portion, into an external portion.
